(19) Europäisches Patentamt
European Patent Office
Office européen des brevets

(11) EP 4 786 485 A1

(12) **EUROPEAN PATENT APPLICATION**
published in accordance with Art. 153(4) EPC

(43) Date of publication:
**05.08.2026 Bulletin 2026/32**

(21) Application number: **24870955.2**

(22) Date of filing: **27.09.2024**

(51) International Patent Classification (IPC):
***C07K 7/06*** *(2006.01)* ***A61K 38/08*** *(2019.01)*
***A61Q 19/08*** *(2006.01)* ***A61P 17/00*** *(2006.01)*

(52) Cooperative Patent Classification (CPC):
**A61K 38/08; A61P 17/00; A61Q 19/08; C07K 7/06**

(86) International application number:
**PCT/CN2024/121723**

(87) International publication number:
**WO 2025/067418 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 CN 202311272706**
**20.09.2024 CN 202411324581**

(71) Applicant: **Shenzhen Zhongge Biological Technology Co., Ltd.**
**Futian District**
**Shenzhen, Guangdong 518017 (CN)**

(72) Inventors:
- **LI, Hongmei**
  **Shenzhen, Guangdong 518017 (CN)**
- **SUN, Jixue**
  **Shenzhen, Guangdong 518017 (CN)**
- **LIN, Sheng**
  **Shenzhen, Guangdong 518017 (CN)**
- **GUI, Linna**
  **Shenzhen, Guangdong 518017 (CN)**
- **MA, Songling**
  **Shenzhen, Guangdong 518017 (CN)**
- **XIE, Dewei**
  **Shenzhen, Guangdong 518017 (CN)**
- **MA, Jin**
  **Shenzhen, Guangdong 518017 (CN)**
- **ZHAO, Liyu**
  **Shenzhen, Guangdong 518017 (CN)**
- **LUO, Jie**
  **Shenzhen, Guangdong 518017 (CN)**
- **LI, Wenting**
  **Shenzhen, Guangdong 518017 (CN)**
- **CHEN, Bin**
  **Shenzhen, Guangdong 518017 (CN)**
- **WANG, Shaohui**
  **Shenzhen, Guangdong 518017 (CN)**
- **ZHANG, Peiyu**
  **Shenzhen, Guangdong 518017 (CN)**
- **WEN, Shuhao**
  **Shenzhen, Guangdong 518017 (CN)**

(74) Representative: **Meissner Bolte Partnerschaft mbB**
**Patentanwälte Rechtsanwälte**
**Postfach 86 06 24**
**81633 München (DE)**

Remarks:
The complete document including Reference Table(s) and the Sequence Listing(s) can be downloaded from the EPO website

# (54) STRAIGHT CHAIN PEPTIDE COMPOUND AND USE THEREOF

(57) A straight chain peptide compound and the use thereof. Specifically provided is a straight chain peptide compound as represented by formula O. The compound has a novel structure and efficacy that is equivalent to or even better than that of GHK tripeptides. Also provided is the use of the straight-chain peptide compound in dermatological or cosmetic compositions for skin anti-aging or hair growth stimulation.
$R^1\text{-}(AA^1)_{n1}\text{-}(AA^2)_{n2}\text{-}(L^1)_{n3}\text{-}(AA^3)_{n4}\text{-}AA^a\text{-}AA^b\text{-}AA^c\text{-}(L^2)\text{-}_{n5}\text{-}(AA^4)_{n6}\text{-}(AA^5)_{n7}\text{-}(AA^a\text{-}AA^b\text{-}AA^c)_m\text{-}R^2$ (Formula O)

EP 4 786 485 A1

## Description

### CROSS REFERENCE TO RELATED APPLICATIONS

The present application claims priority to Chinese patent application CN 202311272706.9 filed on September 27, 2023 and Chinese patent application CN 202411324581.4 filed on September 20, 2024, the contents of which are incorporated herein by reference in their entirety for all purposes.

### TECHNICAL FIELD

The present invention relates to the field of biomedicine or cosmetic products, specifically to linear peptide compounds and uses thereof.

### BACKGROUND ART

GHK is a tripeptide molecule, also known as tripeptide or tripeptide-1, with a sequence of glycyl-histidyl-lysine, which is a hydrolysate of collagen. It is naturally found in human blood, saliva, and urine. GHK is known for its ability to promote production of type I collagen fragments. Its mechanism of action can be simply understood as that when collagen is naturally degraded in the skin, the resulting peptide fragments signal to the skin that it should start working and produce some new healthy collagen.

GHK has a high affinity for divalent copper ions, and can spontaneously bind thereto to form a complex GHK-Cu, which is also known as the blue copper peptide because of the blue color of the aqueous solution and is the main form of GHK that exerts efficacy *in vivo*. Its functions mainly include: (1) producing chemotactic effects on repair-related cells, such as macrophages, mast cells, and capillary cells; (2) producing anti-inflammatory effects (inhibiting free radicals, thromboxane formation, transforming growth factor beta-1, tumor necrosis factor alpha, and protein glycation, simultaneously increasing superoxide dismutase, dilating blood vessels, blocking UV damage to cutaneous keratinocytes, and improving fibroblast recovery after X-ray therapy, etc.); (3) increasing the synthesis of collagen, elastin, hyaluronic acid, matrix metalloproteinases, antiproteases, vascular endothelial growth factor, fibroblast growth factor 2, nerve growth factor, neutrophil elastases 3 and 4, and erythropoietin; (4) increasing the proliferation of fibroblasts and keratinocytes, nerve growth, angiogenesis, and hair follicle size. Control studies of aged skin have shown that GHK-Cu can tighten the skin, improve elasticity and firmness, and reduce fine lines, wrinkles, photodamage, and hyperpigmentation, and can also promote hair growth and improve the success rate of hair transplantation.

### SUMMARY OF THE INVENTION

The present invention provides a linear peptide compound having a novel structure and an efficacy comparable to or even better than that of GHK tripeptide, and use of said linear peptide compound in a dermatological or cosmetic composition for stimulating hair growth or anti-aging of the skin.

To this end, in a first aspect of the present invention, the present invention provides a linear peptide compound represented by formula O, or a stereoisomer thereof,

$$R^1\text{-}(AA^1)_{n1}\text{-}(AA^2)_{n2}\text{-}(L^1)_{n3}\text{-}(AA^3)_{n4}\text{-}AA^a\text{-}AA^b\text{-}AA^c\text{-}(L^2)_{n5}\text{-}(AA^4)_{n6}\text{-}(AA^5)_{n7}\text{-}(AA^a\text{-}AA^b\text{-}AA^c)_m\text{-}R^2$$

Formula O

wherein:

n1, n2, n3, n4, n5, n6, and n7 are each independently selected from 0 and 1; m is selected from 0 and 1; and the following two conditions are met:

1) when n1 and n7 are both 0, n3 and n5 are not both 0;
2) when n3 and n5 are both 0, n1 and n7 are not both 0;

$AA^a$ is selected from the following amino acid residues: GLY, LYS, DLYS, LYO, LHY, LHI, and LPY;
preferably, $AA^a$ is selected from the following amino acid residues: GLY, LYS, DLYS, LYO, and LHY;
more preferably, $AA^a$ is the following amino acid residue: GLY or LYS;
$AA^b$ is selected from the following amino acid residues: HIS and DHIS;
preferably, $AA^b$ is the following amino acid residue: HIS;
$AA^c$ is selected from the following amino acid residues: LYS, DLYS, LYO, LHY, LHI, LPY, and GLY;
preferably, $AA^c$ is selected from the following amino acid residues: LYS, DLYS, LYO, LHY, and GLY;

more preferably, $AA^c$ is selected from the following amino acid residues: LYS, LYO, LHY, and GLY;
most preferably, $AA^c$ is the following amino acid residue: LYS or GLY;
wherein when $AA^a$ is the following amino acid residue: GLY, $AA^c$ is selected from the following amino acid residues: LYS, DLYS, LYO, LHY, LHI, and LPY, and when $AA^a$ is selected from the following amino acid residues: LYS, DLYS, LYO, LHY, LHI, and LPY, $AA^c$ is the following amino acid residue: GLY;
preferably, $AA^a$-$AA^b$-$AA^c$, as a whole, is selected from the following peptide residues: GLY HIS LYS, LYS HIS GLY, GLY DHIS DLYS, GLY HIS LYO, GLY HIS LHY, GLY HIS LHI, and GLY HIS LPY;
$AA^1$ is selected from the following amino acid residues: AGM, NMM, 2MR, MLZ, MLY, M3L, 4PH, ZCL, A30, and D-M3L;
preferably, $AA^1$ is selected from the following amino acid residues: M3L, 4PH, ZCL, and A30;
more preferably, $AA^1$ is selected from the following amino acid residues: 4PH, ZCL, and A30;
most preferably, $AA^1$ is the following amino acid residue: M3L;
$AA^2$ is selected from M3L, 2MR, ALA, and GLY;
preferably, $AA^2$ is selected from the following amino acid residues: M3L and 2MR;
more preferably, $AA^2$ is the following amino acid residue: M3L;
or preferably, $AA^2$ is selected from ALA and GLY;
$AA^3$ is the following amino acid residue: M3L;
$AA^4$ is selected from the following amino acid residues: ALA, GLY, and PRO;
preferably, $AA^4$ is selected from the following amino acid residues: ALA and GLY;
$AA^5$ is selected from the following amino acid residues: M3L, 4PH, ZCL, A30, D0Q, EXL, 0UO, D-4PH, D-ZCL, and D-A30;
preferably, $AA^5$ is selected from the following amino acid residues: M3L, 4PH, ZCL, and A30;
more preferably, $AA^5$ is selected from the following amino acid residues: 4PH and M3L;
$R^1$ is selected from ACE, PAL, BIO, PEG, and VIC;
preferably, $R^1$ is selected from ACE, PAL, and BIO;
more preferably, $R^1$ is ACE;
$R^2$ is selected from NME, -OH, and -NH2;
preferably, $R^2$ is NME;
$L^1$ is selected from P01, P02, P03, P04, P05, P06, P07, P08, P09, P10, P11, P12, P13, P14, P15, P16, P17, P18, P19, P20, P21, P22, P23, P24, P25, P26, P27, P28, P29, and P30;
preferably, $L^1$ is selected from P15, P10, P13, P23, P11, P21, P06, P12, P14, P20, P05, P07, P08, P19, P22, P24, P25, and P29;
more preferably, $L^1$ is selected from P23, P21, P06, P20, P05, P07, P08, P19, P22, P24, P25, and P29;
most preferably, $L^1$ is selected from P23, P21, P06, P20, and P25;
$L^2$ is selected from P01, P02, P03, P04, P05, P06, P07, P08, P09, P10, P11, P12, P13, P14, P15, P16, P17, P18, P19, P20, P21, P22, P23, P24, P25, P26, P27, P28, P29, and P30;
preferably, $L^2$ is selected from P05, P06, P07, P08, P19, P20, P21, P22, P23, P24, P25, and P29;
more preferably, $L^2$ is selected from P05, P06, P07, P08, P19, P20, P21, P22, P23, P24, and P25;
wherein in the linear peptide compound represented by formula O, the sequence from left to right is from the N-terminus to the C-terminus;
wherein the meanings of P01, P02, P03, P04, P05, P06, P07, P08, P09, P10, P11, P12, P13, P14, P15, P16, P17, P18, P19, P20, P21, P22, P23, P24, P25, P26, P27, P28, P29, P30, ACE, PAL, BIO, PEG, VIC, NME, GLY, HIS, DHIS, LYS, DLYS, LYO, LHY, LHI, LPY, AGM, NMM, 2MR, MLZ, MLY, M3L, 4PH, ZCL, A30, D0Q, EXL, 0UO, D-4PH, D-ZCL, D-A30, D-M3L, ALA, and PRO are as shown in Table E.

In some embodiments, the linear peptide compound has a structure represented by formula I,

$$R^1\text{-}AA^a\text{-}AA^b\text{-}AA^c\text{-}L^2\text{-}AA^a\text{-}AA^b\text{-}AA^c\text{-}R^2 \qquad \text{Formula I}$$

wherein:

$AA^a$ is the following amino acid residue: GLY;
$AA^b$ is selected from the following amino acid residues: HIS and DHIS;
preferably, $AA^b$ is the following amino acid residue: HIS;
$AA^c$ is selected from the following amino acid residues: LYS, DLYS, LYO, and LHY;
preferably, $AA^c$ is the following amino acid residue: LYS;
preferably, $AA^a$-$AA^b$-$AA^c$, as a whole, is the following peptide residue: GLY HIS LYS;
$R^1$ is selected from ACE, PAL, and BIO;

preferably, $R^1$ is ACE;
$R^2$ is selected from NME, -OH, and -NH2;
preferably, $R^2$ is NME;
$L^2$ is selected from P01, P02, P03, P04, P05, P06, P07, P08, P09, P10, P11, P12, P13, P14, P15, P16, P17, P18, P19, P20, P21, P22, P23, P24, P25, P26, P27, P28, P29, and P30;
preferably, $L^2$ is selected from P05, P06, P07, P08, P19, P20, P21, P22, P23, P24, P25, and P29;
more preferably, $L^2$ is selected from P05, P06, P07, P08, P19, P20, P21, P22, P23, P24, and P25.

In some embodiments, the linear peptide compound has a structure represented by formula II,

$$R^1\text{-}(AA^1)_{n1}\text{-}(AA^2)_{n2}\text{-}L^1\text{-}(AA^3)_{n4}\text{-}AA^a\text{-}AA^b\text{-}AA^c\text{-}(AA^4)_{n6}\text{-}(AA^5)_{n7}\text{-}R^2 \qquad \text{Formula II}$$

wherein:

n1, n2, n4, n6, and n7 are each independently selected from 0 and 1;
preferably, n1 is selected from 0 and 1, and n2, n4, n6, and n7 are all 0;
or preferably, n1, n2, n4, n6, and n7 are all 0;
$AA^a$ is selected from the following amino acid residues: GLY, LYS, DLYS, LYO, and LHY;
preferably, $AA^a$ is selected from the following amino acid residues: GLY and LYS;
$AA^b$ is selected from the following amino acid residues: HIS and DHIS;
preferably, $AA^b$ is the following amino acid residue: HIS;
$AA^c$ is selected from the following amino acid residues: LYS, DLYS, LYO, LHY, and GLY;
preferably, $AA^c$ is selected from the following amino acid residues: LYS, LYO, LHY, and GLY;
preferably, $AA^c$ is selected from the following amino acid residues: LYS and GLY;
wherein when $AA^a$ is the following amino acid residue: GLY, $AA^c$ is selected from the following amino acid residues: LYS, DLYS, LYO, and LHY, and when $AA^a$ is selected from the following amino acid residues: LYS, DLYS, LYO, and LHY, $AA^c$ is the following amino acid residue: GLY;
preferably, $AA^a$-$AA^b$-$AA^c$, as a whole, is selected from the following peptide residues: GLY HIS LYS, LYS HIS GLY, GLY DHIS DLYS, GLY HIS LYO, and GLY HIS LHY;
$AA^1$ is selected from the following amino acid residues: M3L, 4PH, ZCL, and A30;
preferably, $AA^1$ is the following amino acid residue: M3L;
$AA^2$ is the following amino acid residue: M3L;
$AA^3$ is the following amino acid residue: M3L;
$AA^4$ is selected from the following amino acid residues: GLY and ALA;
$AA^5$ is the following amino acid residue: 4PH;
$R^1$ is selected from ACE, PAL, and BIO;
preferably, $R^1$ is ACE;
$R^2$ is selected from NME, -OH, and -NH2;
preferably, $R^2$ is NME;
$L^1$ is selected from P15, P10, P13, P23, P11, P21, P06, P12, P14, P20, P05, P07, P08, P19, P22, P24, P25, and P29;
preferably, $L^1$ is selected from P23, P21, P06, P20, P05, P07, P08, P19, P22, P24, P25, and P29;
more preferably, $L^1$ is selected from P23, P21, P06, P20, and P25.

In some embodiments, the linear peptide compound has a structure represented by formula II-1,

$$R^1\text{-}(AA^1)_{n1}\text{-}(AA^2)_{n2}\text{-}L^1\text{-}(AA^3)_{n4}\text{-}AA^{a1}\text{-}AA^{b1}\text{-}AA^{c1}\text{-}(AA^4)_{n6}\text{-}(AA^5)_{n7}\text{-}R^2 \qquad \text{Formula II-1}$$

wherein:

n1, n2, n4, n6, and n7 are each independently selected from 0 and 1;
preferably, n1 is selected from 0 and 1, and n2, n4, n6, and n7 are all 0;
$AA^{a1}$ is the following amino acid residue: GLY;
$AA^{b1}$ is selected from the following amino acid residues: HIS and DHIS;
preferably, $AA^{b1}$ is the following amino acid residue: HIS;
$AA^{c1}$ is selected from the following amino acid residues: LYS, DLYS, LYO, and LHY;
preferably, $AA^{c1}$ is the following amino acid residue: LYS, LYO, or LHY;
preferably, $AA^{c1}$ is the following amino acid residue: LYS;
preferably, $AA^{a1}$-$AA^{b1}$-$AA^{c1}$, as a whole, is selected from the following peptide residues: GLY HIS LYS, GLY DHIS

DLYS, GLY HIS LYO, and GLY HIS LHY;
$AA^1$ is selected from the following amino acid residues: M3L, 4PH, ZCL, and A30;
preferably, $AA^1$ is the following amino acid residue: M3L;
$AA^2$ is the following amino acid residue: M3L;
$AA^3$ is the following amino acid residue: M3L;
$AA^4$ is selected from the following amino acid residues: GLY and ALA;
$AA^5$ is the following amino acid residue: 4PH;
$R^1$ is selected from ACE, PAL, and BIO;
preferably, $R^1$ is ACE;
$R^2$ is selected from NME, -OH, and -NH2;
preferably, $R^2$ is NME;
$L^1$ is selected from P15, P10, P13, P23, P11, P21, P06, P12, P14, P20, P05, P07, P08, P19, P22, P24, P25, and P29;
preferably, $L^1$ is selected from P23, P21, P06, P20, P05, P07, P08, P19, P22, P24, P25, and P29;
more preferably, $L^1$ is selected from P23, P21, P06, P20, and P25.

In some embodiments, the linear peptide compound has a structure represented by formula II-1-1,

$$R^1\text{-}L^1\text{-}AA^{a1}\text{-}AA^{b1}\text{-}AA^{c1}\text{-}R^2 \qquad \text{Formula II-1-1}$$

wherein:

$AA^{a1}$ is the following amino acid residue: GLY;
$AA^{b1}$ is selected from the following amino acid residues: HIS and DHIS;
$AA^{c1}$ is selected from the following amino acid residues: LYS, DLYS, LYO, and LHY;
preferably, $AA^{a1}$-$AA^{b1}$-$AA^{c1}$, as a whole, is selected from the following peptide residues: GLY HIS LYS, GLY DHIS DLYS, GLY HIS LYO, and GLY HIS LHY;
$R^1$ is selected from ACE, PAL, and BIO;
$R^2$ is selected from NME, -OH, and -NH2;
$L^1$ is selected from P15, P10, P13, P23, P11, P21, P06, P12, P14, P20, P05, P07, P08, P19, P22, P24, P25, and P29;
preferably, $L^1$ is selected from P23, P21, P06, P20, P05, P07, P08, P19, P22, P24, P25, and P29;
more preferably, $L^1$ is selected from P23, P21, P06, P20, P25, and P29;
most preferably, $L^1$ is selected from P21, P06, and P25.

In some embodiments, the linear peptide compound has a structure represented by formula II-1-2,

$$R^1\text{-}AA^1\text{-}L^1\text{-}AA^{a1}\text{-}AA^{b1}\text{-}AA^{c1}\text{-}R^2 \qquad \text{Formula II-1-2}$$

wherein:

$AA^{a1}$ is the following amino acid residue: GLY;
$AA^{b1}$ is selected from the following amino acid residues: HIS and DHIS;
$AA^{c1}$ is selected from the following amino acid residues: LYS, DLYS, LYO, and LHY;
preferably, $AA^{a1}$-$AA^{b1}$-$AA^{c1}$, as a whole, is selected from the following peptide residues: GLY HIS LYS, GLY DHIS DLYS, GLY HIS LYO, and GLY HIS LHY;
$AA^1$ is selected from the following amino acid residues: M3L and 4PH;
$R^1$ is selected from ACE, PAL, and BIO;
$R^2$ is selected from NME, -OH, and -NH2;
$L^1$ is selected from P15, P10, P13, P23, P11, P21, P06, P12, P14, P20, P05, P07, P08, P19, P22, P24, P25, and P29;
preferably, $L^1$ is selected from P23, P21, P06, P20, P05, P07, P08, P19, P22, P24, P25, and P29;
more preferably, $L^1$ is selected from P23, P21, and P06.

In some embodiments, the linear peptide compound has a structure represented by formula II-1-3,

$$R^1\text{-}L^1\text{-}AA^3\text{-}AA^{a1}\text{-}AA^{b1}\text{-}AA^{c1}\text{-}R^2 \qquad \text{Formula II-1-3}$$

wherein:

$AA^{a1}$ is the following amino acid residue: GLY;

$AA^{b1}$ is selected from the following amino acid residues: HIS and DHIS;
preferably, $AA^{b1}$ is the following amino acid residue: HIS;
$AA^{c1}$ is selected from the following amino acid residues: LYS, DLYS, LYO, and LHY;
preferably, $AA^{c1}$ is the following amino acid residue: LYS;
preferably, $AA^{a1}$-$AA^{b1}$-$AA^{c1}$, as a whole, is the following peptide residue: GLY HIS LYS;
$AA^3$ is the following amino acid residue: M3L;
$R^1$ is selected from ACE, PAL, and BIO;
$R^2$ is selected from NME, -OH, and -NH2;
$L^1$ is selected from P15, P10, P13, P23, P11, P21, P06, P12, P14, P20, P25, and P29;
preferably, $L^1$ is selected from P23, P21, P06, P20, P25, and P29.

In some embodiments, the linear peptide compound has a structure represented by formula II-1-4,

$R^1$-$AA^1$-$AA^2$-$L^1$-$AA^{a1}$-$AA^{b1}$-$AA^{c1}$-$R^2$ Formula II-1-4

wherein:

$AA^{a1}$ is the following amino acid residue: GLY;
$AA^{b1}$ is selected from the following amino acid residues: HIS and DHIS;
preferably, $AA^{b1}$ is the following amino acid residue: HIS;
$AA^{c1}$ is selected from the following amino acid residues: LYS, DLYS, LYO, and LHY;
preferably, $AA^{c1}$ is the following amino acid residue: LYS;
preferably, $AA^{a1}$-$AA^{b1}$-$AA^{c1}$, as a whole, is the following peptide residue: GLY HIS LYS;
$AA^1$ is selected from the following amino acid residues: 4PH, ZCL, and A30;
$AA^2$ is the following amino acid residue: M3L;
$R^1$ is selected from ACE, PAL, and BIO;
preferably, $R^1$ is ACE;
$R^2$ is selected from NME, -OH, and -NH2;
preferably, $R^2$ is NME;
$L^1$ is selected from P15, P10, P13, P23, P11, P21, P06, P12, P14, and P20;
preferably, $L^1$ is selected from P23, P21, P06, and P20.

In some embodiments, the linear peptide compound has a structure represented by formula II-1-5,

$R^1$-$L^1$-$AA^{a1}$-$AA^{b1}$-$AA^{c1}$-$AA^5$-$R^2$ Formula II-1-5

wherein:

$AA^{a1}$ is the following amino acid residue: GLY;
$AA^{b1}$ is selected from the following amino acid residues: HIS and DHIS;
preferably, $AA^{b1}$ is the following amino acid residue: HIS;
$AA^{c1}$ is selected from the following amino acid residues: LYS, DLYS, LYO, and LHY;
preferably, $AA^{c1}$ is the following amino acid residue: LYS;
preferably, $AA^{a1}$-$AA^{b1}$-$AA^{c1}$, as a whole, is the following peptide residue: GLY HIS LYS;
$AA^5$ is the following amino acid residue: 4PH;
$R^1$ is selected from ACE, PAL, and BIO;
preferably, $R^1$ is ACE;
$R^2$ is selected from NME, -OH, and -NH2;
preferably, $R^2$ is NME;
$L^1$ is selected from P15, P10, P13, P23, P11, P21, P06, P12, P14, and P20;
preferably, $L^1$ is selected from P23, P21, P06, and P20.

In some embodiments, the linear peptide compound has a structure represented by formula II-1-6,

$R^1$-$L^1$-$AA^{a1}$-$AA^{b1}$-$AA^{c1}$-$AA^4$-$AA^5$-$R^2$ Formula II-1-6

wherein:

$AA^{a1}$ is the following amino acid residue: GLY;
$AA^{b1}$ is selected from the following amino acid residues: HIS and DHIS;
preferably, $AA^{b1}$ is the following amino acid residue: HIS;
$AA^{c1}$ is selected from the following amino acid residues: LYS, DLYS, LYO, and LHY;
preferably, $AA^{c1}$ is the following amino acid residue: LYS;
preferably, $AA^{a1}$-$AA^{b1}$-$AA^{c1}$, as a whole, is the following peptide residue: GLY HIS LYS;
$AA^4$ is selected from the following amino acid residues: GLY and ALA;
$AA^5$ is the following amino acid residue: 4PH;
$R^1$ is selected from ACE, PAL, and BIO;
preferably, $R^1$ is ACE;
$R^2$ is selected from NME, -OH, and -NH2;
preferably, $R^2$ is NME;
$L^1$ is selected from P15, P10, P13, P23, P11, P21, P06, P12, P14, and P20;
preferably, $L^1$ is selected from P23, P21, P06, and P20.

In some embodiments, the linear peptide compound has a structure represented by formula II-1-7,

$$R^1\text{-}AA^1\text{-}L^1\text{-}AA^{a1}\text{-}AA^{b1}\text{-}AA^{c1}\text{-}AA^4\text{-}AA^5\text{-}R^2 \qquad \text{Formula II-1-7}$$

wherein:

$AA^{a1}$ is the following amino acid residue: GLY;
$AA^{b1}$ is selected from the following amino acid residues: HIS and DHIS;
preferably, $AA^{b1}$ is the following amino acid residue: HIS;
$AA^{c1}$ is selected from the following amino acid residues: LYS, DLYS, LYO, and LHY;
preferably, $AA^{c1}$ is the following amino acid residue: LYS;
preferably, $AA^{a1}$-$AA^{b1}$-$AA^{c1}$, as a whole, is the following peptide residue: GLY HIS LYS;
$AA^1$ is the following amino acid residue: M3L;
$AA^4$ is selected from the following amino acid residues: GLY and ALA;
preferably, $AA^4$ is the following amino acid residue: ALA;
$AA^5$ is the following amino acid residue: 4PH;
$R^1$ is selected from ACE, PAL, and BIO;
preferably, $R^1$ is ACE;
$R^2$ is selected from NME, -OH, and -NH2;
preferably, $R^2$ is NME;
$L^1$ is selected from P15, P10, P13, P23, P11, P21, P06, P12, P14, and P20;
preferably, $L^1$ is selected from P23, P21, P06, and P20.

In some embodiments, the linear peptide compound has a structure represented by formula II-2,

$$R^1\text{-}(AA^1)_{n1}\text{-}L^1\text{-}AA^{a2}\text{-}AA^{b2}\text{-}AA^{c2}\text{-}R^2 \qquad \text{Formula II-2}$$

wherein:

n1 is selected from 0 and 1;
preferably, n1 is 0;
$AA^{a2}$ is selected from the following amino acid residues: LYS, DLYS, LYO, and LHY;
preferably, $AA^{a2}$ is the following amino acid residue: LYS;
$AA^{b2}$ is selected from the following amino acid residues: HIS and DHIS;
preferably, $AA^{b2}$ is the following amino acid residue: HIS;
$AA^{c2}$ is the following amino acid residue: GLY;
preferably, $AA^{a2}$-$AA^{b2}$-$AA^{c2}$, as a whole, is the following peptide residue: LYS HIS GLY;
$AA^1$ is the following amino acid residue: M3L;
$R^1$ is selected from ACE, PAL, and BIO;
$R^2$ is selected from NME, -OH, and -NH2;
$L^1$ is selected from P15, P10, P13, P23, P11, P21, P06, P12, P14, P20, P25, and P29;
preferably, $L^1$ is selected from P23, P21, P06, P20, P25, and P29;
more preferably, $L^1$ is selected from P23, P21, P06, P20, and P25.

In some embodiments, the linear peptide compound has a structure represented by formula II-2-1,

$$R^1\text{-}L^1\text{-}AA^{a2}\text{-}AA^{b2}\text{-}AA^{c2}\text{-}R^2 \quad \text{Formula II-2-1}$$

wherein:

$AA^{a2}$ is selected from the following amino acid residues: LYS, DLYS, LYO, and LHY;
preferably, $AA^{a2}$ is the following amino acid residue: LYS;
$AA^{b2}$ is selected from the following amino acid residues: HIS and DHIS;
preferably, $AA^{b2}$ is the following amino acid residue: HIS;
$AA^{c2}$ is the following amino acid residue: GLY;
preferably, $AA^{a2}$-$AA^{b2}$-$AA^{c2}$, as a whole, is the following peptide residue: LYS HIS GLY;
$R^1$ is selected from ACE, PAL, and BIO;
$R^2$ is selected from NME, -OH, and -NH2;
$L^1$ is selected from P15, P10, P13, P23, P11, P21, P06, P12, P14, P20, P25, and P29;
preferably, $L^1$ is selected from P23, P21, P06, P20, P25, and P29;
more preferably, $L^1$ is selected from P23, P21, P06, P20, and P25.

In some embodiments, the linear peptide compound has a structure represented by formula II-2-2,

$$R^1\text{-}AA^1\text{-}L^1\text{-}AA^{a2}\text{-}AA^{b2}\text{-}AA^{c2}\text{-}R^2 \quad \text{Formula II-2-2}$$

wherein:

$AA^{a2}$ is selected from the following amino acid residues: LYS, DLYS, LYO, and LHY;
preferably, $AA^{a2}$ is the following amino acid residue: LYS;
$AA^{b2}$ is selected from the following amino acid residues: HIS and DHIS;
preferably, $AA^{b2}$ is the following amino acid residue: HIS;
$AA^{c2}$ is the following amino acid residue: GLY;
preferably, $AA^{a2}$-$AA^{b2}$-$AA^{c2}$, as a whole, is the following peptide residue: LYS HIS GLY;
$AA^1$ is the following amino acid residue: M3L;
$R^1$ is selected from ACE, PAL, and BIO;
preferably, $R^1$ is selected from ACE and PAL;
$R^2$ is selected from NME, -OH, and -NH2;
preferably, $R^2$ is selected from NME and -OH;
$L^1$ is selected from P15, P10, P13, P23, P11, P21, P06, P12, P14, P20, P25, and P29;
preferably, $L^1$ is selected from P23, P21, P06, P20, P25, and P29;
more preferably, $L^1$ is selected from P25 and P29.

In some embodiments, the linear peptide compound has a structure represented by formula III,

$$R^1\text{-}AA^1\text{-}(AA^2)_{n2}\text{-}AA^a\text{-}AA^b\text{-}AA^c\text{-}(AA^4)_{n6}\text{-}AA^5\text{-}R^2 \quad \text{Formula III}$$

wherein:

n2 and n6 are each independently selected from 0 and 1;
preferably, n2 is 0, and n6 is selected from 0 and 1;
or preferably, n2 is selected from 0 and 1, and n6 is 0;
$AA^a$ is selected from the following amino acid residues: GLY, LYS, DLYS, LYO, and LHY;
preferably, $AA^a$ is selected from the following amino acid residues: GLY and LYS;
$AA^b$ is selected from the following amino acid residues: HIS and DHIS;
preferably, $AA^b$ is the following amino acid residue: HIS;
$AA^c$ is selected from the following amino acid residues: LYS, DLYS, LYO, LHY, LHI, LPY, and GLY;
preferably, $AA^c$ is selected from the following amino acid residues: LYS, LYO, LHY, and GLY;
more preferably, $AA^c$ is selected from the following amino acid residues: LYS and GLY;
wherein when $AA^a$ is the following amino acid residue: GLY, $AA^c$ is selected from the following amino acid residues: LYS, DLYS, LYO, LHY, LHI, and LPY, and when $AA^a$ is selected from the following amino acid residues: LYS, DLYS, LYO, and LHY, $AA^c$ is the following amino acid residue: GLY;

preferably, $AA^a$-$AA^b$-$AA^c$, as a whole, is selected from the following peptide residues: GLY HIS LYS, LYS HIS GLY, GLY DHIS DLYS, GLY HIS LYO, GLY HIS LHI, GLY HIS LHY, and GLY HIS LPY;

$R^1$ is selected from ACE, PAL, BIO, PEG, and VIC;

preferably, $R^1$ is selected from ACE, PAL, and BIO;

$R^2$ is selected from NME, -OH, and -NH2;

$AA^1$ is selected from the following amino acid residues: AGM, NMM, 2MR, MLZ, MLY, M3L, 4PH, ZCL, A30, and D-M3L;

preferably, $AA^1$ is selected from the following amino acid residues: M3L, 4PH, ZCL, and A30;

more preferably, $AA^1$ is selected from the following amino acid residues: 4PH, ZCL, and A30;

more preferably, $AA^1$ is the following amino acid residue: M3L;

$AA^2$ is selected from the following amino acid residues: M3L, 2MR, ALA, and GLY;

preferably, $AA^2$ is selected from the following amino acid residues: M3L and 2MR;

preferably, $AA^2$ is selected from the following amino acid residues: ALA and GLY;

$AA^4$ is selected from the following amino acid residues: ALA, GLY, and PRO;

preferably, $AA^4$ is selected from the following amino acid residues: ALA and GLY;

$AA^5$ is selected from the following amino acid residues: M3L, 4PH, ZCL, A30, D0Q, EXL, 0UO, D-4PH, D-ZCL, and D-A30;

preferably, $AA^5$ is selected from the following amino acid residues: M3L, 4PH, ZCL, and A30;

more preferably, $AA^5$ is selected from the following amino acid residues: 4PH, ZCL, and A30;

more preferably, $AA^5$ is the following amino acid residue: M3L.

In some embodiments, the linear peptide compound has a structure represented by formula III-1,

$$R^1\text{-}AA^1\text{-}(AA^2)_{n2}\text{-}AA^{a1}\text{-}AA^{b1}\text{-}AA^{c1}\text{-}(AA^4)_{n6}\text{-}AA^5\text{-}R^2 \qquad \text{Formula III-1}$$

wherein:

n2 and n6 are each independently selected from 0 and 1;

preferably, n2 is 0, and n6 is selected from 0 and 1;

$AA^{a1}$ is the following amino acid residue: GLY;

$AA^{b1}$ is selected from the following amino acid residues: HIS and DHIS;

preferably, $AA^{b1}$ is the following amino acid residue: HIS;

$AA^{c1}$ is selected from the following amino acid residues: LYS, DLYS, LYO, LHY, LHI, and LPY;

preferably, $AA^{c1}$ is selected from the following amino acid residues: LYS, LYO, and LHY;

more preferably, $AA^{c1}$ is the following amino acid residue: LYS;

preferably, $AA^{a1}$-$AA^{b1}$-$AA^{c1}$, as a whole, is selected from the following peptide residues: GLY HIS LYS, GLY DHIS DLYS, GLY HIS LYO, GLY HIS LHI, GLY HIS LHY, and GLY HIS LPY;

$R^1$ is selected from ACE, PAL, BIO, PEG, and VIC;

preferably, $R^1$ is selected from ACE, PAL, and BIO;

$R^2$ is selected from NME, -OH, and -NH2;

$AA^1$ is selected from the following amino acid residues: AGM, NMM, 2MR, MLZ, MLY, M3L, 4PH, ZCL, A30, and D-M3L;

preferably, $AA^1$ is selected from the following amino acid residues: M3L, 4PH, ZCL, and A30;

more preferably, $AA^1$ is the following amino acid residue: M3L;

$AA^2$ is selected from the following amino acid residues: M3L and 2MR;

$AA^4$ is selected from the following amino acid residues: ALA, GLY, and PRO;

preferably, $AA^4$ is selected from the following amino acid residues: ALA and GLY;

$AA^5$ is selected from the following amino acid residues: 4PH, ZCL, A30, D0Q, EXL, 0UO, D-4PH, D-ZCL, and D-A30;

preferably, $AA^5$ is selected from the following amino acid residues: 4PH, ZCL, and A30.

In some embodiments, the linear peptide compound has a structure represented by formula III-1-1,

$$R^1\text{-}AA^1\text{-}AA^{a1}\text{-}AA^{b1}\text{-}AA^{c1}\text{-}AA^5\text{-}R^2 \qquad \text{Formula III-1-1}$$

wherein:

$AA^{a1}$ is the following amino acid residue: GLY;

$AA^{b1}$ is selected from the following amino acid residues: HIS and DHIS;

$AA^{c1}$ is selected from the following amino acid residues: LYS, DLYS, LYO, LHY, LHI, and LPY;
preferably, $AA^{c1}$ is selected from the following amino acid residues: LYS, DLYS, LYO, and LHY;
preferably, $AA^{a1}$-$AA^{b1}$-$AA^{c1}$, as a whole, is selected from the following peptide residues: GLY HIS LYS, GLY DHIS DLYS, GLY HIS LYO, GLY HIS LHI, GLY HIS LHY, and GLY HIS LPY;
$R^1$ is selected from ACE, PAL, BIO, PEG, and VIC;
preferably, $R^1$ is selected from ACE, PAL, and BIO;
$R^2$ is selected from NME, -OH, and -NH2;
$AA^1$ is selected from the following amino acid residues: AGM, NMM, 2MR, MLZ, MLY, M3L, 4PH, ZCL, A30, and D-M3L;
preferably, $AA^1$ is selected from the following amino acid residues: M3L, 4PH, ZCL, and A30;
more preferably, $AA^1$ is the following amino acid residue: M3L;
$AA^5$ is selected from the following amino acid residues: 4PH, ZCL, A30, D0Q, EXL, 0UO, D-4PH, D-ZCL, and D-A30;
preferably, $AA^5$ is selected from the following amino acid residues: 4PH, ZCL, and A30.

In some embodiments, the linear peptide compound has a structure represented by formula III-1-2,

$R^1$-$AA^1$-$AA^{a1}$-$AA^{b1}$-$AA^{c1}$-$AA^4$-$AA^5$-$R^2$ Formula III-1-2

wherein:

$AA^{a1}$ is the following amino acid residue: GLY;
$AA^{b1}$ is selected from the following amino acid residues: HIS and DHIS;
preferably, $AA^{b1}$ is the following amino acid residue: HIS;
$AA^{c1}$ is selected from the following amino acid residues: LYS, DLYS, LYO, LHY, LHI, and LPY;
preferably, $AA^{c1}$ is the following amino acid residue: LYS;
preferably, $AA^{a1}$-$AA^{b1}$-$AA^{c1}$, as a whole, is the following peptide residue: GLY HIS LYS;
$R^1$ is selected from ACE, PAL, BIO, PEG, and VIC;
preferably, $R^1$ is selected from ACE and PAL;
$R^2$ is selected from NME, -OH, and -NH2;
preferably, $R^2$ is selected from NME and -OH;
$AA^1$ is selected from the following amino acid residues: M3L, MLZ, and MLY;
preferably, $AA^1$ is the following amino acid residue: M3L;
$AA^4$ is selected from the following amino acid residues: ALA, GLY, and PRO;
preferably, $AA^4$ is selected from the following amino acid residues: ALA and GLY;
$AA^5$ is selected from the following amino acid residues: 4PH, ZCL, and A30.

In some embodiments, the linear peptide compound has a structure represented by formula III-1-3,

$R^1$-$AA^1$-$AA^2$-$AA^{a1}$-$AA^{b1}$-$AA^{c1}$-$AA^4$-$AA^5$-$R^2$ Formula III-1-3

wherein:

$AA^{a1}$ is the following amino acid residue: GLY;
$AA^{b1}$ is selected from the following amino acid residues: HIS and DHIS;
preferably, $AA^{b1}$ is the following amino acid residue: HIS;
$AA^{c1}$ is selected from the following amino acid residues: LYS, DLYS, LYO, LHY, LHI, and LPY;
preferably, $AA^{c1}$ is the following amino acid residue: LYS;
preferably, $AA^{a1}$-$AA^{b1}$-$AA^{c1}$, as a whole, is the following peptide residue: GLY HIS LYS;
$R^1$ is selected from ACE, PAL, BIO, PEG, and VIC;
preferably, $R^1$ is ACE;
$R^2$ is selected from NME, -OH, and -NH2;
preferably, $R^2$ is NME;
$AA^1$ is selected from the following amino acid residues: M3L and 2MR;
$AA^2$ is selected from the following amino acid residues: M3L and 2MR;
$AA^4$ is the following amino acid residue: ALA;
$AA^5$ is selected from 4PH, ZCL, and A30.

In some embodiments, the linear peptide compound has a structure represented by formula III-2,

$R^1$-$AA^1$-$(AA^2)_{n2}$-$AA^{a2}$-$AA^{b2}$-$AA^{c2}$-$AA^5$-$R^2$ Formula III-2

wherein:

n2 is selected from 0 and 1;
$AA^{a2}$ is selected from the following amino acid residues: LYS, DLYS, LYO, and LHY;
preferably, $AA^{a2}$ is the following amino acid residue: LYS;
$AA^{b2}$ is selected from the following amino acid residues: HIS and DHIS;
preferably, $AA^{b2}$ is the following amino acid residue: HIS;
$AA^{c2}$ is the following amino acid residue: GLY;
preferably, $AA^{a2}$-$AA^{b2}$-$AA^{c2}$, as a whole, is the following peptide residue: LYS HIS GLY;
$R^1$ is selected from ACE, PAL, BIO, PEG, and VIC;
preferably, $R^1$ is selected from ACE, PAL, and BIO;
preferably, $R^1$ is ACE;
$R^2$ is selected from NME, -OH, and -NH2;
preferably, $R^2$ is NME;
$AA^1$ is selected from the following amino acid residues: M3L, 4PH, ZCL, and A30;
preferably, $AA^1$ is selected from the following amino acid residues: 4PH, ZCL, and A30;
$AA^2$ is selected from the following amino acid residues: ALA and GLY;
$AA^5$ is selected from the following amino acid residues: M3L, 4PH, ZCL, and A30;
preferably, $AA^5$ is the following amino acid residue: M3L.

In some embodiments, the linear peptide compound has a structure represented by formula III-2-1,

$R^1$-$AA^1$-$AA^{a2}$-$AA^{b2}$-$AA^{c2}$-$AA^5$-$R^2$ Formula III-2-1

wherein:

$AA^{a2}$ is selected from the following amino acid residues: LYS, DLYS, LYO, and LHY;
preferably, $AA^{a2}$ is the following amino acid residue: LYS;
$AA^{b2}$ is selected from the following amino acid residues: HIS and DHIS;
preferably, $AA^{b2}$ is the following amino acid residue: HIS;
$AA^{c2}$ is the following amino acid residue: GLY;
preferably, $AA^{a2}$-$AA^{b2}$-$AA^{c2}$, as a whole, is the following peptide residue: LYS HIS GLY;
$R^1$ is selected from ACE, PAL, BIO, PEG, and VIC;
preferably, $R^1$ is ACE;
$R^2$ is selected from NME, -OH, and -NH2;
preferably, $R^2$ is NME;
$AA^1$ is selected from the following amino acid residues: M3L, 4PH, ZCL, and A30;
preferably, $AA^1$ is selected from the following amino acid residues: 4PH, ZCL, and A30;
$AA^5$ is selected from the following amino acid residues: M3L, 4PH, ZCL, and A30;
preferably, $AA^5$ is the following amino acid residue: M3L.

In some embodiments, the linear peptide compound has a structure represented by formula III-2-2,

$R^1$-$AA^1$-$AA^2$-$AA^{a2}$-$AA^{b2}$-$AA^{c2}$-$AA^5$-$R^2$ Formula III-2-2

$AA^{a2}$ is selected from the following amino acid residues: LYS, DLYS, LYO, and LHY;
preferably, $AA^{a2}$ is the following amino acid residue: LYS;
$AA^{b2}$ is selected from the following amino acid residues: HIS and DHIS;
preferably, $AA^{b2}$ is the following amino acid residue: HIS;
$AA^{c2}$ is the following amino acid residue: GLY;
preferably, $AA^{a2}$-$AA^{b2}$-$AA^{c2}$, as a whole, is the following peptide residue: LYS HIS GLY;
$R^1$ is selected from ACE, PAL, and BIO;
$R^2$ is selected from NME, -OH, and -NH2;
$AA^1$ is selected from the following amino acid residues: 4PH, ZCL, and A30;
$AA^2$ is selected from the following amino acid residues: ALA and GLY;
$AA^5$ is the following amino acid residue: M3L.

In some embodiments, the linear peptide compound has a structure represented by formula IV,

$R^1$-$(AA^1)_{n1}$-$(L^1)_{n3}$-$AA^a$-$AA^b$-$AA^c$-$(AA^5)_{n7}$-$R^2$ Formula IV

wherein:

n1, n3, and n7 are each independently selected from 0 and 1, and are not all 0;
preferably, n1 and n7 are 0, and n3 is 1; or n1 and n7 are 1; n3 is 0; or n1 and n3 are 1, and n7 is 0;
$AA^a$-$AA^b$-$AA^c$, as a whole, is selected from the following peptide residues: LYS HIS GLY, GLY HIS LYS, GLY HIS LYO, and GLY HIS LHY;
preferably, $AA^a$-$AA^b$-$AA^c$, as a whole, is selected from the following peptide residues: LYS HIS GLY, GLY HIS LYS, and GLY HIS LYO;
$AA^1$ is selected from the following amino acid residues: M3L, AGM, NMM, 2MR, MLZ, MLY, 4PH, ZCL, A30, and D-M3L;
preferably, $AA^1$ is selected from the following amino acid residues: M3L, 4PH, ZCL, and A30;
preferably, $AA^1$ is selected from the following amino acid residues: M3L and 4PH;
preferably, $AA^1$ is the following amino acid residue: M3L;
$AA^5$ is selected from the following amino acid residues: 4PH, ZCL, A30, D0Q, EXL, 0UO, M3L, D-4PH, D-ZCL, and D-A30;
preferably, $AA^5$ is selected from the following amino acid residues: 4PH, ZCL, A30, and M3L;
preferably, $AA^5$ is selected from the following amino acid residues: 4PH, A30, and M3L;
preferably, $AA^5$ is the following amino acid residue: 4PH;
preferably, $AA^5$ is the following amino acid residue: A30;
$L^1$ is selected from P15, P10, P13, P23, P11, P21, P06, P12, P14, P20, P05, P07, P08, P19, P22, P24, P25, and P29;
preferably, $L^1$ is selected from P23, P21, P06, P20, P05, P07, P08, P19, P22, P24, P25, and P29;
preferably, $L^1$ is selected from P21, P06, P23, P25, and P20;
preferably, $L^1$ is selected from P21, P06, P25, and P23;
$R^1$ is selected from ACE, PAL, BIO, PEG, and VIC;
preferably, $R^1$ is selected from ACE, PAL, and BIO;
preferably, $R^1$ is selected from ACE and PAL;
more preferably, $R^1$ is ACE;
$R^2$ is selected from NME, -OH, and -NH2;
preferably, $R^2$ is selected from NME and -OH;
more preferably, $R^2$ is NME.

In some embodiments, the linear peptide compound has a structure represented by formula I-a,

$R^1$-GLY-HIS-LYS-$L^2$-GLY-HIS-LYS-$R^2$ (SEQ ID NO: 433) Formula I-a

wherein:

$R^1$, $L^2$, and $R^2$ are as defined in any of the aforementioned embodiments;
preferably, $L^2$ is selected from P01, P02, P03, P04, P05, P06, P07, P08, P19, P20, P21, P22, and P23;
further preferably, $L^2$ is selected from P05, P06, P07, P08, P19, P20, P21, P22, and P23;
more preferably, $L^2$ is selected from P06, P21, and P23.

In some embodiments, the linear peptide compound has any one of the structures represented by formulae II-a to II-o:

$R^1$-$L^1$-GLY-HIS-LYS-$R^2$ Formula II-a

$R^1$-$AA^1$-$L^1$-GLY-HIS-LYS-$R^2$ Formula II-b

$R^1$-$L^1$-$AA^3$-GLY-HIS-LYS-$R^2$ Formula II-c

$R^1$-$AA^1$-$AA^2$-$L^1$-GLY-HIS-LYS-$R^2$ Formula II-d

$R^1$-$L^1$-GLY-HIS-LYS-$AA^5$-$R^2$ Formula II-e

$R^1$-$L^1$-GLY-HIS-LYS-$AA^4$-$AA^5$-$R^2$ Formula II-f

$R^1$-$AA^1$-$L^1$-GLY-HIS-LYS-$AA^4$-$AA^5$-$R^2$ Formula II-g

$R^1$-$L^1$-GLY-DHIS-DLYS-$R^2$ Formula II-h

$R^1$-$AA^1$-$L^1$-GLY-DHIS-DLYS-$R^2$ Formula II-i

$R^1$-$L^1$-GLY-HIS-LYO-$R^2$ Formula II-j

$R^1$-$AA^1$-$L^1$-GLY-HIS-LYO-$R^2$ Formula II-k

$R^1$-$L^1$-GLY-HIS-LHY-$R^2$ Formula II-l

$R^1$-$AA^1$-$L^1$-GLY-HIS-LHY-$R^2$ Formula II-m

$R^1$-$L^1$-LYS-HIS-GLY-$R^2$ Formula II-n

$R^1$-$AA^1$-$L^1$-LYS-HIS-GLY-$R^2$ Formula II-o

wherein $R^1$, $L^1$, $R^2$, $AA^1$, $AA^2$, $AA^3$, $AA^4$, and $AA^5$ are as defined in any of the embodiments above.

In some embodiments, the linear peptide compound has a structure represented by formula II-n.

In some embodiments, the linear peptide compound has a structure represented by formula II-a.

In some embodiments, the linear peptide compound has a structure represented by formula II-b.

In some embodiments, the linear peptide compound has a structure represented by formula II-l.

In some embodiments, the linear peptide compound has a structure represented by formula II-k.

In some embodiments, in formula II-a, preferably, $R^1$ is selected from ACE, PAL, and BIO; $L^1$ is selected from P15, P10, P13, P23, P11, P21, P06, P12, P14, P20, P05, P07, P08, P19, P22, P24, P25, and P29; $R^2$ is selected from NME, -OH, and $-NH_2$; more preferably, $R^1$ is selected from PAL and BIO; $L^1$ is P25; $R^2$ is selected from -OH and $-NH_2$.

In some embodiments, in formula II-b, preferably, $R^1$ is selected from ACE and PAL; $AA^1$ is selected from M3L and 4PH; $L^1$ is selected from P15, P10, P13, P23, P11, P21, P06, P12, P14, P20, P05, P07, P08, P19, P22, P24, P25, and P29; $R^2$ is selected from NME and -OH; more preferably, $R^1$ is PAL; $AA^1$ is M3L; $L^1$ is P23; $R^2$ is -OH.

In some embodiments, in formula II-c, preferably, $R^1$ is selected from ACE, PAL, and BIO; $L^1$ is selected from P15, P10, P13, P23, P11, P21, P06, P12, P14, P20, P25, and P29; $AA^3$ is M3L; $R^2$ is selected from NME, -OH, and $-NH_2$.

In some embodiments, in formula II-d, preferably, $R^1$ is ACE; $AA^1$ is selected from 4PH, ZCL, and A30; $AA^2$ is M3L; $L^1$ is selected from P15, P10, P13, P23, P11, P21, P06, P12, P14, and P20; $R^2$ is NME.

In some embodiments, in formula II-e, preferably, $R^1$ is ACE; $L^1$ is selected from P15, P10, P13, P23, P11, P21, P06, P12, P14, and P20; $AA^5$ is 4PH; $R^2$ is NME.

In some embodiments, in formula II-f, preferably, $R^1$ is ACE; $L^1$ is selected from P15, P10, P13, P23, P11, P21, P06, P12, P14, and P20; $AA^4$ is selected from GLY and ALA; $AA^5$ is 4PH; $R^2$ is NME.

In some embodiments, in formula II-g, preferably, $R^1$ is ACE; $AA^1$ is M3L; $L^1$ is selected from P15, P10, P13, P23, P11, P21, P06, P12, P14, and P20; $AA^4$ is ALA; $AA^5$ is 4PH; $R^2$ is NME.

In some embodiments, in formula II-h, preferably, $R^1$ is selected from ACE, PAL, and BIO; $L^1$ is selected from P15, P10, P13, P23, P11, P21, P06, P12, P14, P20, P25, and P29; $R^2$ is selected from NME, -OH, and $-NH_2$.

In some embodiments, in formula II-i, preferably, $R^1$ is selected from ACE and PAL; $AA^1$ is M3L; $L^1$ is selected from P15, P10, P13, P23, P11, P21, P06, P12, P14, P20, P25, and P29; $R^2$ is selected from NME and -OH.

In some embodiments, in formula II-j, preferably, $R^1$ is ACE; $L^1$ is selected from P15, P10, P13, P23, P11, P21, P06, P12, P14, and P20; $R^2$ is NME.

In some embodiments, in formula II-k, preferably, $R^1$ is selected from ACE and BIO; $AA^1$ is M3L; $L^1$ is selected from P15, P10, P13, P23, P11, P21, P06, P12, P14, and P20; $R^2$ is selected from NME and $-NH_2$; more preferably, $R^1$ is BIO; $AA^1$ is M3L; $L^1$ is P06; $R^2$ is $-NH_2$.

In some embodiments, in formula II-l, preferably, $R^1$ is selected from ACE, PAL, and BIO; $L^1$ is selected from P15, P10, P13, P23, P11, P21, P06, P12, P14, and P20; $R^2$ is selected from NME, -OH, and $-NH_2$; more preferably, $R^1$ is PAL; $L^1$ is P21; $R^2$ is -OH.

In some embodiments, in formula II-m, preferably, $R^1$ is ACE; $AA^1$ is M3L; $L^1$ is selected from P15, P10, P13, P23, P11, P21, P06, P12, P14, and P20; $R^2$ is NME.

In some embodiments, in formula II-n, preferably, $R^1$ is selected from ACE, PAL, and BIO; $L^1$ is selected from P15,

P10, P13, P23, P11, P21, P06, P12, P14, P20, P25, and P29; $R^2$ is selected from NME, -OH, and $-NH_2$; more preferably, $R^1$ is selected from ACE, PAL, and BIO; $L^1$ is selected from P06, P21, and P25; $R^2$ is selected from NME, -OH, and $-NH_2$.

In some embodiments, in formula II-o, preferably, $R^1$ is selected from ACE and PAL; $AA^1$ is M3L; $L^1$ is selected from P15, P10, P13, P23, P11, P21, P06, P12, P14, P20, P25, and P29; $R^2$ is selected from NME and -OH.

In some embodiments, the linear peptide compound has any one of the structures represented by formulae III-a to III-j:

$R^1$-$AA^1$-GLY-HIS-LYS-$AA^5$-$R^2$ Formula III-a

$R^1$-$AA^1$-GLY-HIS-LYO-$AA^5$-$R^2$ Formula III-b

$R^1$-$AA^1$-GLY-HIS-LYS-$AA^4$-$AA^5$-$R^2$ Formula III-c

$R^1$-$AA^1$-$AA^2$-GLY-HIS-LYS-$AA^4$-$AA^5$-$R^2$ Formula III-d

$R^1$-$AA^1$-LYS-HIS-GLY-$AA^5$-$R^2$ Formula III-e

$R^1$-$AA^1$-GLY-DHIS-DLYS-$AA^5$-$R^2$ Formula III-f

$R^1$-$AA^1$-GLY-HIS-LHI-$AA^5$-$R^2$ Formula III-g

$R^1$-$AA^1$-GLY-HIS-LHY-$AA^5$-$R^2$ Formula III-h

$R^1$-$AA^1$-GLY-HIS-LPY-$AA^5$-$R^2$ Formula III-i

$R^1$-$AA^1$-$AA^2$-LYS-HIS-GLY-$AA^5$-$R^2$ Formula III-j

wherein $R^1$, $AA^1$, $AA^2$, $AA^4$, $AA^5$, and $R^2$ are as defined in any of the aforementioned embodiments.

In some embodiments, the linear peptide compound has a structure represented by formula III-b.

In some embodiments, the linear peptide compound has a structure represented by formula III-c.

In some embodiments, the linear peptide compound has a structure represented by formula III-e.

In some embodiments, the linear peptide compound has a structure represented by formula III-h.

In some embodiments, the linear peptide compound has a structure represented by formula III-j.

In some embodiments, in formula III-a, preferably, $R^1$ is selected from ACE, PAL, BIO, PEG, and VIC; $AA^1$ is selected from AGM, NMM, 2MR, MLZ, MLY, M3L, 4PH, ZCL, A30, and D-M3L; $AA^5$ is selected from 4PH, ZCL, A30, D0Q, EXL, 0UO, D-4PH, D-ZCL, and D-A30; $R^2$ is selected from NME, -OH, and $-NH_2$.

In some embodiments, in formula III-b, preferably, $R^1$ is selected from ACE, PAL, and BIO; $AA^1$ is M3L; $AA^5$ is selected from 4PH, ZCL, and A30; $R^2$ is selected from NME, -OH, and $-NH_2$; more preferably, $R^1$ is selected from ACE and PAL; $AA^1$ is M3L; $AA^5$ is selected from 4PH and A30; $R^2$ is selected from NME and -OH.

In some embodiments, in formula III-c, preferably, $R^1$ is selected from ACE and PAL; $AA^1$ is selected from M3L, MLZ, and MLY; $AA^4$ is selected from ALA, GLY, and PRO; $AA^5$ is selected from 4PH, ZCL, and A30; $R^2$ is selected from NME and -OH; more preferably, $R^1$ is selected from ACE and PAL; $AA^1$ is M3L; $AA^4$ is selected from ALA and GLY; $AA^5$ is selected from 4PH and ZCL; $R^2$ is selected from NME and -OH.

In some embodiments, in formula III-d, preferably, $R^1$ is ACE; $AA^1$ is selected from M3L and 2MR; $AA^2$ is selected from 2MR and M3L; $AA^4$ is ALA; $AA^5$ is selected from 4PH, ZCL, and A30; $R^2$ is NME.

In some embodiments, in formula III-e, preferably, $R^1$ is ACE; $AA^1$ is selected from M3L, 4PH, ZCL, and A30; $AA^5$ is selected from 4PH, ZCL, A30, and M3L; $R^2$ is NME; more preferably, $R^1$ is ACE; $AA^1$ is 4PH; $AA^5$ is M3L; $R^2$ is NME.

In some embodiments, in formula III-f, preferably, $R^1$ is ACE; $AA^1$ is selected from M3L and D-M3L; $AA^5$ is selected from 4PH, ZCL, A30, D-4PH, D-ZCL, and D-A30; $R^2$ is NME.

In some embodiments, in formula III-g, preferably, $R^1$ is ACE; $AA^1$ is M3L; $AA^5$ is selected from 4PH, ZCL, and A30; $R^2$ is NME.

In some embodiments, in formula III-h, preferably, $R^1$ is selected from ACE, PAL, and BIO; $AA^1$ is M3L; $AA^5$ is selected from 4PH, ZCL, and A30; $R^2$ is selected from NME, -OH, and $-NH_2$; more preferably, $R^1$ is selected from ACE, PAL, and BIO; $AA^1$ is M3L; $AA^5$ is 4PH; $R^2$ is selected from NME, -OH, and $-NH_2$.

In some embodiments, in formula III-i, preferably, $R^1$ is ACE; $AA^1$ is M3L; $AA^5$ is selected from 4PH, ZCL, and A30; $R^2$ is NME.

In some embodiments, in formula III-j, preferably, $R^1$ is selected from ACE, PAL, and BIO; $AA^1$ is selected from 4PH, ZCL, and A30; $AA^2$ is selected from ALA and GLY; $AA^5$ is M3L; $R^2$ is selected from NME, -OH, and $-NH_2$; more

preferably, $R^1$ is selected from PAL and BIO; $AA^1$ is selected from 4PH, ZCL, and A30; $AA^2$ is ALA; $AA^5$ is M3L; $R^2$ is selected from -OH and $-NH_2$.

In some embodiments, the structure of the linear peptide compound is as shown in Table A.

Table A: The structural formula of the linear peptide compound

| SEQ ID NO: | The structural formula of the linear peptide compound (N-terminus → C-terminus) |
|---|---|
| 1 | ACE GLY HIS LYS P01 GLY HIS LYS NME |
| 2 | ACE GLY HIS LYS P02 GLY HIS LYS NME |
| 3 | ACE GLY HIS LYS P03 GLY HIS LYS NME |
| 4 | ACE GLY HIS LYS P04 GLY HIS LYS NME |
| 5 | ACE GLY HIS LYS P05 GLY HIS LYS NME |
| 6 | ACE GLY HIS LYS P06 GLY HIS LYS NME |
| 7 | ACE GLY HIS LYS P07 GLY HIS LYS NME |
| 8 | ACE GLY HIS LYS P08 GLY HIS LYS NME |
| 9 | ACE GLY HIS LYS P09 GLY HIS LYS NME |
| 10 | ACE GLY HIS LYS P10 GLY HIS LYS NME |
| 11 | ACE GLY HIS LYS P11 GLY HIS LYS NME |
| 12 | ACE GLY HIS LYS P12 GLY HIS LYS NME |
| 13 | ACE GLY HIS LYS P13 GLY HIS LYS NME |
| 14 | ACE GLY HIS LYS P14 GLY HIS LYS NME |
| 15 | ACE GLY HIS LYS P15 GLY HIS LYS NME |
| 16 | ACE GLY HIS LYS P16 GLY HIS LYS NME |
| 17 | ACE GLY HIS LYS P17 GLY HIS LYS NME |
| 18 | ACE GLY HIS LYS P18 GLY HIS LYS NME |
| 19 | ACE GLY HIS LYS GLY GLY GLY HIS LYS NME |
| 20 | ACE GLY HIS LYS GLY GLY GLY GLY HIS LYS NME |
| 21 | ACE GLY HIS LYS P21 GLY HIS LYS NME |
| 22 | ACE GLY HIS LYS ALA ALA GLY HIS LYS NME |
| 23 | ACE GLY HIS LYS ALA ALA ALA GLY HIS LYS NME |
| 24 | ACE GLY HIS LYS PRO PRO GLY HIS LYS NME |
| 25 | ACE GLY HIS LYS PRO PRO PRO GLY HIS LYS NME |
| 26 | ACE GLY HIS LYS P26 GLY HIS LYS NME |
| 27 | ACE GLY HIS LYS P27 GLY HIS LYS NME |
| 28 | ACE GLY HIS LYS P28 GLY HIS LYS NME |
| 29 | ACE GLY HIS LYS P29 GLY HIS LYS NME |
| 30 | ACE GLY HIS LYS P30 GLY HIS LYS NME |
| 31 | ACE P15 GLY HIS LYS NME |
| 32 | ACE P10 GLY HIS LYS NME |
| 33 | ACE P13 GLY HIS LYS NME |
| 34 | ACE ALA ALA ALA GLY HIS LYS NME |
| 35 | ACE P11 GLY HIS LYS NME |
| 36 | ACE P21 GLY HIS LYS NME |

(continued)

| SEQ ID NO: | The structural formula of the linear peptide compound (N-terminus → C-terminus) |
|---|---|
| 37 | ACE P06 GLY HIS LYS NME |
| 38 | ACE P12 GLY HIS LYS NME |
| 39 | ACE P14 GLY HIS LYS NME |
| 40 | ACE GLY GLY GLY GLY HIS LYS NME |
| 41 | ACE M3L P15 GLY HIS LYS NME |
| 42 | ACE M3L P10 GLY HIS LYS NME |
| 43 | ACE M3L P13 GLY HIS LYS NME |
| 44 | ACE M3L ALA ALA ALA GLY HIS LYS NME |
| 45 | ACE M3L P11 GLY HIS LYS NME |
| 46 | ACE M3L P21 GLY HIS LYS NME |
| 47 | ACE M3L P06 GLY HIS LYS NME |
| 48 | ACE M3L P12 GLY HIS LYS NME |
| 49 | ACE M3L P14 GLY HIS LYS NME |
| 50 | ACE M3L GLY GLY GLY GLY HIS LYS NME |
| 51 | ACE 4PH P15 GLY HIS LYS NME |
| 52 | ACE 4PH P10 GLY HIS LYS NME |
| 53 | ACE 4PH P13 GLY HIS LYS NME |
| 54 | ACE 4PH ALA ALA ALA GLY HIS LYS NME |
| 55 | ACE 4PH P11 GLY HIS LYS NME |
| 56 | ACE 4PH P21 GLY HIS LYS NME |
| 57 | ACE 4PH P06 GLY HIS LYS NME |
| 58 | ACE 4PH P12 GLY HIS LYS NME |
| 59 | ACE 4PH P14 GLY HIS LYS NME |
| 60 | ACE 4PH GLY GLY GLY GLY HIS LYS NME |
| 61 | ACE P15 M3L GLY HIS LYS NME |
| 62 | ACE P10 M3L GLY HIS LYS NME |
| 63 | ACE P13 M3L GLY HIS LYS NME |
| 64 | ACE ALA ALA ALA M3L GLY HIS LYS NME |
| 65 | ACE P11 M3L GLY HIS LYS NME |
| 66 | ACE P21 M3L GLY HIS LYS NME |
| 67 | ACE P06 M3L GLY HIS LYS NME |
| 68 | ACE P12 M3L GLY HIS LYS NME |
| 69 | ACE P14 M3L GLY HIS LYS NME |
| 70 | ACE GLY GLY GLY M3L GLY HIS LYS NME |
| 71 | ACE P15 LYS HIS GLY NME |
| 72 | ACE P10 LYS HIS GLY NME |
| 73 | ACE P13 LYS HIS GLY NME |
| 74 | ACE ALA ALA ALA LYS HIS GLY NME |
| 75 | ACE P11 LYS HIS GLY NME |

(continued)

| SEQ ID NO: | The structural formula of the linear peptide compound (N-terminus → C-terminus) |
|---|---|
| 76 | ACE P21 LYS HIS GLY NME |
| 77 | ACE P06 LYS HIS GLY NME |
| 78 | ACE P12 LYS HIS GLY NME |
| 79 | ACE P14 LYS HIS GLY NME |
| 80 | ACE GLY GLY GLY LYS HIS GLY NME |
| 81 | ACE M3L P15 LYS HIS GLY NME |
| 82 | ACE M3L P10 LYS HIS GLY NME |
| 83 | ACE M3L P13 LYS HIS GLY NME |
| 84 | ACE M3L ALA ALA ALA LYS HIS GLY NME |
| 85 | ACE M3L P11 LYS HIS GLY NME |
| 86 | ACE M3L P21 LYS HIS GLY NME |
| 87 | ACE M3L P06 LYS HIS GLY NME |
| 88 | ACE M3L P12 LYS HIS GLY NME |
| 89 | ACE M3L P14 LYS HIS GLY NME |
| 90 | ACE M3L GLY GLY GLY LYS HIS GLY NME |
| 91 | ACE 4PH M3L P15 GLY HIS LYS NME |
| 92 | ACE 4PH M3L P10 GLY HIS LYS NME |
| 93 | ACE 4PH M3L P13 GLY HIS LYS NME |
| 94 | ACE 4PH M3L ALA ALA ALA GLY HIS LYS NME |
| 95 | ACE 4PH M3L P11 GLY HIS LYS NME |
| 96 | ACE 4PH M3L P21 GLY HIS LYS NME |
| 97 | ACE 4PH M3L P06 GLY HIS LYS NME |
| 98 | ACE 4PH M3L P12 GLY HIS LYS NME |
| 99 | ACE 4PH M3L P14 GLY HIS LYS NME |
| 100 | ACE 4PH M3L GLY GLY GLY GLY HIS LYS NME |
| 101 | ACE ZCL M3L P15 GLY HIS LYS NME |
| 102 | ACE ZCL M3L P10 GLY HIS LYS NME |
| 103 | ACE ZCL M3L P13 GLY HIS LYS NME |
| 104 | ACE ZCL M3L ALA ALA ALA GLY HIS LYS NME |
| 105 | ACE ZCL M3L P11 GLY HIS LYS NME |
| 106 | ACE ZCL M3L P21 GLY HIS LYS NME |
| 107 | ACE ZCL M3L P06 GLY HIS LYS NME |
| 108 | ACE ZCL M3L P12 GLY HIS LYS NME |
| 109 | ACE ZCL M3L P14 GLY HIS LYS NME |
| 110 | ACE ZCL M3L GLY GLY GLY GLY HIS LYS NME |
| 111 | ACE A30 M3L P15 GLY HIS LYS NME |
| 112 | ACE A30 M3L P10 GLY HIS LYS NME |
| 113 | ACE A30 M3L P13 GLY HIS LYS NME |
| 114 | ACE A30 M3L ALA ALA ALA GLY HIS LYS NME |

(continued)

| SEQ ID NO: | The structural formula of the linear peptide compound (N-terminus → C-terminus) |
|---|---|
| 115 | ACE A30 M3L P11 GLY HIS LYS NME |
| 116 | ACE A30 M3L P21 GLY HIS LYS NME |
| 117 | ACE A30 M3L P06 GLY HIS LYS NME |
| 118 | ACE A30 M3L P12 GLY HIS LYS NME |
| 119 | ACE A30 M3L P14 GLY HIS LYS NME |
| 120 | ACE A30 M3L GLY GLY GLY GLY HIS LYS NME |
| 121 | ACE P15 GLY HIS LYS 4PH NME |
| 122 | ACE P10 GLY HIS LYS 4PH NME |
| 123 | ACE P13 GLY HIS LYS 4PH NME |
| 124 | ACE ALA ALA ALA GLY HIS LYS 4PH NME |
| 125 | ACE P11 GLY HIS LYS 4PH NME |
| 126 | ACE P21 GLY HIS LYS 4PH NME |
| 127 | ACE P06 GLY HIS LYS 4PH NME |
| 128 | ACE P12 GLY HIS LYS 4PH NME |
| 129 | ACE P14 GLY HIS LYS 4PH NME |
| 130 | ACE GLY GLY GLY GLY HIS LYS 4PH NME |
| 131 | ACE P15 GLY HIS LYS GLY 4PH NME |
| 132 | ACE P10 GLY HIS LYS GLY 4PH NME |
| 133 | ACE P13 GLY HIS LYS GLY 4PH NME |
| 134 | ACE ALA ALA ALA GLY HIS LYS GLY 4PH NME |
| 135 | ACE P11 GLY HIS LYS GLY 4PH NME |
| 136 | ACE P21 GLY HIS LYS GLY 4PH NME |
| 137 | ACE P06 GLY HIS LYS GLY 4PH NME |
| 138 | ACE P12 GLY HIS LYS GLY 4PH NME |
| 139 | ACE P14 GLY HIS LYS GLY 4PH NME |
| 140 | ACE GLY GLY GLY GLY HIS LYS GLY 4PH NME |
| 141 | ACE P15 GLY HIS LYS ALA 4PH NME |
| 142 | ACE P10 GLY HIS LYS ALA 4PH NME |
| 143 | ACE P13 GLY HIS LYS ALA 4PH NME |
| 144 | ACE ALA ALA ALA GLY HIS LYS ALA 4PH NME |
| 145 | ACE P11 GLY HIS LYS ALA 4PH NME |
| 146 | ACE P21 GLY HIS LYS ALA 4PH NME |
| 147 | ACE P06 GLY HIS LYS ALA 4PH NME |
| 148 | ACE P12 GLY HIS LYS ALA 4PH NME |
| 149 | ACE P14 GLY HIS LYS ALA 4PH NME |
| 150 | ACE GLY GLY GLY GLY HIS LYS ALA 4PH NME |
| 151 | ACE M3L P15 GLY HIS LYS ALA 4PH NME |
| 152 | ACE M3L P10 GLY HIS LYS ALA 4PH NME |
| 153 | ACE M3L P13 GLY HIS LYS ALA 4PH NME |

(continued)

| SEQ ID NO: | The structural formula of the linear peptide compound (N-terminus → C-terminus) |
|---|---|
| 154 | ACE M3L ALA ALA ALA GLY HIS LYS ALA 4PH NME |
| 155 | ACE M3L P11 GLY HIS LYS ALA 4PH NME |
| 156 | ACE M3L P21 GLY HIS LYS ALA 4PH NME |
| 157 | ACE M3L P06 GLY HIS LYS ALA 4PH NME |
| 158 | ACE M3L P12 GLY HIS LYS ALA 4PH NME |
| 159 | ACE M3L P14 GLY HIS LYS ALA 4PH NME |
| 160 | ACE M3L GLY GLY GLY GLY HIS LYS ALA 4PH NME |
| 161 | ACE P15 GLY DHIS DLYS NME |
| 162 | ACE P10 GLY DHIS DLYS NME |
| 163 | ACE P13 GLY DHIS DLYS NME |
| 164 | ACE ALA ALA ALA GLY DHIS DLYS NME |
| 165 | ACE P11 GLY DHIS DLYS NME |
| 166 | ACE P21 GLY DHIS DLYS NME |
| 167 | ACE P06 GLY DHIS DLYS NME |
| 168 | ACE P12 GLY DHIS DLYS NME |
| 169 | ACE P14 GLY DHIS DLYS NME |
| 170 | ACE GLY GLY GLY GLY DHIS DLYS NME |
| 171 | ACE M3L P15 GLY DHIS DLYS NME |
| 172 | ACE M3L P10 GLY DHIS DLYS NME |
| 173 | ACE M3L P13 GLY DHIS DLYS NME |
| 174 | ACE M3L ALA ALA ALA GLY DHIS DLYS NME |
| 175 | ACE M3L P11 GLY DHIS DLYS NME |
| 176 | ACE M3L P21 GLY DHIS DLYS NME |
| 177 | ACE M3L P06 GLY DHIS DLYS NME |
| 178 | ACE M3L P12 GLY DHIS DLYS NME |
| 179 | ACE M3L P14 GLY DHIS DLYS NME |
| 180 | ACE M3L GLY GLY GLY GLY DHIS DLYS NME |
| 181 | ACE P15 GLY HIS LYO NME |
| 182 | ACE P10 GLY HIS LYO NME |
| 183 | ACE P13 GLY HIS LYO NME |
| 184 | ACE ALA ALA ALA GLY HIS LYO NME |
| 185 | ACE P11 GLY HIS LYO NME |
| 186 | ACE P21 GLY HIS LYO NME |
| 187 | ACE P06 GLY HIS LYO NME |
| 188 | ACE P12 GLY HIS LYO NME |
| 189 | ACE P14 GLY HIS LYO NME |
| 190 | ACE GLY GLY GLY GLY HIS LYO NME |
| 191 | ACE M3L P15 GLY HIS LYO NME |
| 192 | ACE M3L P10 GLY HIS LYO NME |

(continued)

| SEQ ID NO: | The structural formula of the linear peptide compound (N-terminus → C-terminus) |
|---|---|
| 193 | ACE M3L P13 GLY HIS LYO NME |
| 194 | ACE M3L ALA ALA ALA GLY HIS LYO NME |
| 195 | ACE M3L P11 GLY HIS LYO NME |
| 196 | ACE M3L P21 GLY HIS LYO NME |
| 197 | ACE M3L P06 GLY HIS LYO NME |
| 198 | ACE M3L P12 GLY HIS LYO NME |
| 199 | ACE M3L P14 GLY HIS LYO NME |
| 200 | ACE M3L GLY GLY GLY GLY HIS LYO NME |
| 201 | ACE P15 GLY HIS LHY NME |
| 202 | ACE P10 GLY HIS LHY NME |
| 203 | ACE P13 GLY HIS LHY NME |
| 204 | ACE ALA ALA ALA GLY HIS LHY NME |
| 205 | ACE P11 GLY HIS LHY NME |
| 206 | ACE P21 GLY HIS LHY NME |
| 207 | ACE P06 GLY HIS LHY NME |
| 208 | ACE P12 GLY HIS LHY NME |
| 209 | ACE P14 GLY HIS LHY NME |
| 210 | ACE GLY GLY GLY GLY HIS LHY NME |
| 211 | ACE M3L P15 GLY HIS LHY NME |
| 212 | ACE M3L P10 GLY HIS LHY NME |
| 213 | ACE M3L P13 GLY HIS LHY NME |
| 214 | ACE M3L ALA ALA ALA GLY HIS LHY NME |
| 215 | ACE M3L P11 GLY HIS LHY NME |
| 216 | ACE M3L P21 GLY HIS LHY NME |
| 217 | ACE M3L P06 GLY HIS LHY NME |
| 218 | ACE M3L P12 GLY HIS LHY NME |
| 219 | ACE M3L P14 GLY HIS LHY NME |
| 220 | ACE M3L GLY GLY GLY GLY HIS LHY NME |
| 221 | PAL P13 GLY DHIS DLYS OH |
| 222 | PAL P10 GLY HIS LYS OH |
| 223 | PAL M3L P10 GLY DHIS DLYS OH |
| 224 | PAL P06 LYS HIS GLY OH |
| 225 | PAL M3L P13 GLY DHIS DLYS OH |
| 226 | PAL P11 GLY DHIS DLYS OH |
| 227 | PAL M3L P15 GLY DHIS DLYS OH |
| 228 | PAL P15 M3L GLY HIS LYS OH |
| 229 | PAL M3L P15 GLY HIS LYS OH |
| 230 | PAL M3L P11 GLY HIS LYS OH |
| 231 | PAL 4PH P15 GLY HIS LYS OH |

(continued)

| SEQ ID NO: | The structural formula of the linear peptide compound (N-terminus → C-terminus) |
|---|---|
| 232 | PAL P15 LYS HIS GLY OH |
| 233 | PAL M3L P13 GLY HIS LYS OH |
| 234 | PAL M3L ALA ALA ALA GLY HIS LYS OH |
| 235 | PAL 4PH P10 GLY HIS LYS OH |
| 236 | PAL P13 GLY HIS LYS OH |
| 237 | PAL M3L ALA ALA ALA GLY DHIS DLYS OH |
| 238 | PAL M3L P10 GLY HIS LYS OH |
| 239 | PAL M3L P10 LYS HIS GLY OH |
| 240 | PAL P12 LYS HIS GLY OH |
| 241 | BIO P13 GLY DHIS DLYS NH2 |
| 242 | BIO P06 LYS HIS GLY NH2 |
| 243 | BIO P11 GLY DHIS DLYS NH2 |
| 244 | BIO P15 LYS HIS GLY NH2 |
| 245 | BIO P13 GLY HIS LYS NH2 |
| 246 | BIO P12 LYS HIS GLY NH2 |
| 247 | BIO P14 M3L GLY HIS LYS NH2 |
| 248 | BIO P15 GLY HIS LYS NH2 |
| 249 | BIO P11 GLY HIS LYS NH2 |
| 250 | BIO P14 LYS HIS GLY NH2 |
| 251 | PAL P15 GLY HIS LHY OH |
| 252 | PAL P13 GLY HIS LHY OH |
| 253 | PAL P10 GLY HIS LHY OH |
| 254 | PAL P11 GLY HIS LHY OH |
| 255 | PAL P06 GLY HIS LHY OH |
| 256 | PAL P21 GLY HIS LHY OH |
| 257 | PAL P12 GLY HIS LHY OH |
| 258 | PAL ALA ALA ALA GLY HIS LHY OH |
| 259 | PAL GLY GLY GLY GLY HIS LHY OH |
| 260 | PAL P14 GLY HIS LHY OH |
| 261 | BIO P06 GLY HIS LHY NH2 |
| 262 | BIO P14 GLY HIS LHY NH2 |
| 263 | BIO M3L P13 GLY HIS LYO NH2 |
| 264 | BIO M3L P12 GLY HIS LYO NH2 |
| 265 | BIO M3L P06 GLY HIS LYO NH2 |
| 266 | ACE P05 GLY HIS LYS NME |
| 267 | ACE P07 GLY HIS LYS NME |
| 268 | ACE P08 GLY HIS LYS NME |
| 269 | ACE GLY GLY GLY HIS LYS NME |
| 270 | ACE ALA ALA GLY HIS LYS NME |

(continued)

| SEQ ID NO: | The structural formula of the linear peptide compound (N-terminus → C-terminus) |
|---|---|
| 271 | ACE PRO PRO GLY HIS LYS NME |
| 272 | ACE PRO PRO PRO GLY HIS LYS NME |
| 273 | ACE P29 GLY HIS LYS NME |
| 274 | ACE M3L P05 GLY HIS LYS NME |
| 275 | ACE M3L P07 GLY HIS LYS NME |
| 276 | ACE M3L P08 GLY HIS LYS NME |
| 277 | ACE M3L GLY GLY GLY HIS LYS NME |
| 278 | ACE M3L ALA ALA GLY HIS LYS NME |
| 279 | ACE M3L PRO PRO GLY HIS LYS NME |
| 280 | ACE M3L PRO PRO PRO GLY HIS LYS NME |
| 281 | ACE M3L P29 GLY HIS LYS NME |
| 282 | ACE 4PH PRO PRO PRO GLY HIS LYS NME |
| 283 | ACE 4PH P29 GLY HIS LYS NME |
| 284 | ACE PRO PRO PRO M3L GLY HIS LYS NME |
| 285 | ACE P29 M3L GLY HIS LYS NME |
| 286 | ACE PRO PRO PRO LYS HIS GLY NME |
| 287 | ACE P29 LYS HIS GLY NME |
| 288 | ACE M3L PRO PRO PRO LYS HIS GLY NME |
| 289 | ACE M3L P29 LYS HIS GLY NME |
| 290 | ACE PRO PRO PRO GLY DHIS DLYS NME |
| 291 | ACE P29 GLY DHIS DLYS NME |
| 292 | ACE M3L PRO PRO PRO GLY DHIS DLYS NME |
| 293 | ACE M3L P29 GLY DHIS DLYS NME |
| 294 | PAL PRO PRO PRO GLY HIS LYS OH |
| 295 | PAL P29 GLY HIS LYS OH |
| 296 | BIO PRO PRO PRO GLY HIS LYS NH2 |
| 297 | BIO P29 GLY HIS LYS NH2 |
| 298 | ACE AGM GLY HIS LYS 4PH NME |
| 299 | ACE AGM GLY HIS LYS ZCL NME |
| 300 | ACE AGM GLY HIS LYS A30 NME |
| 301 | ACE AGM GLY HIS LYS D0Q NME |
| 302 | ACE AGM GLY HIS LYS EXL NME |
| 303 | ACE AGM GLY HIS LYS 0UO NME |
| 304 | ACE NMM GLY HIS LYS 4PH NME |
| 305 | ACE NMM GLY HIS LYS ZCL NME |
| 306 | ACE NMM GLY HIS LYS A30 NME |
| 307 | ACE NMM GLY HIS LYS D0Q NME |
| 308 | ACE NMM GLY HIS LYS EXL NME |
| 309 | ACE NMM GLY HIS LYS 0UO NME |

(continued)

| SEQ ID NO: | The structural formula of the linear peptide compound (N-terminus → C-terminus) |
|---|---|
| 310 | ACE 2MR GLY HIS LYS 4PH NME |
| 311 | ACE 2MR GLY HIS LYS ZCL NME |
| 312 | ACE 2MR GLY HIS LYS A30 NME |
| 313 | ACE 2MR GLY HIS LYS D0Q NME |
| 314 | ACE 2MR GLY HIS LYS EXL NME |
| 315 | ACE 2MR GLY HIS LYS 0UO NME |
| 316 | ACE MLZ GLY HIS LYS 4PH NME |
| 317 | ACE MLZ GLY HIS LYS ZCL NME |
| 318 | ACE MLZ GLY HIS LYS A30 NME |
| 319 | ACE MLZ GLY HIS LYS D0Q NME |
| 320 | ACE MLZ GLY HIS LYS EXL NME |
| 321 | ACE MLZ GLY HIS LYS 0UO NME |
| 322 | ACE MLY GLY HIS LYS 4PH NME |
| 323 | ACE MLY GLY HIS LYS ZCL NME |
| 324 | ACE MLY GLY HIS LYS A30 NME |
| 325 | ACE MLY GLY HIS LYS D0Q NME |
| 326 | ACE MLY GLY HIS LYS EXL NME |
| 327 | ACE MLY GLY HIS LYS 0UO NME |
| 328 | ACE M3L GLY HIS LYS 4PH NME |
| 329 | ACE M3L GLY HIS LYS ZCL NME |
| 330 | ACE M3L GLY HIS LYS A30 NME |
| 331 | ACE M3L GLY HIS LYS D0Q NME |
| 332 | ACE M3L GLY HIS LYS EXL NME |
| 333 | ACE M3L GLY HIS LYS 0UO NME |
| 334 | ACE M3L GLY HIS LYO 4PH NME |
| 335 | ACE M3L GLY HIS LYO ZCL NME |
| 336 | ACE M3L GLY HIS LYO A30 NME |
| 337 | ACE M3L GLY HIS LYS ALA 4PH NME |
| 338 | ACE M3L GLY HIS LYS ALA ZCL NME |
| 339 | ACE M3L GLY HIS LYS ALA A30 NME |
| 340 | ACE M3L 2MR GLY HIS LYS ALA 4PH NME |
| 341 | ACE M3L 2MR GLY HIS LYS ALA ZCL NME |
| 342 | ACE M3L 2MR GLY HIS LYS ALA A30 NME |
| 343 | ACE 2MR M3L GLY HIS LYS ALA 4PH NME |
| 344 | ACE 2MR M3L GLY HIS LYS ALA ZCL NME |
| 345 | ACE 2MR M3L GLY HIS LYS ALA A30 NME |
| 346 | ACE 4PH GLY HIS LYS 4PH NME |
| 347 | ACE ZCL GLY HIS LYS ZCL NME |
| 348 | ACE A30 GLY HIS LYS A30 NME |

(continued)

| SEQ ID NO: | The structural formula of the linear peptide compound (N-terminus → C-terminus) |
|---|---|
| 349 | ACE M3L GLY HIS LYS GLY 4PH NME |
| 350 | ACE M3L GLY HIS LYS GLY ZCL NME |
| 351 | ACE M3L GLY HIS LYS GLY A30 NME |
| 352 | ACE M3L GLY HIS LYS PRO 4PH NME |
| 353 | ACE M3L GLY HIS LYS PRO ZCL NME |
| 354 | ACE M3L GLY HIS LYS PRO A30 NME |
| 355 | ACE M3L LYS HIS GLY 4PH NME |
| 356 | ACE M3L LYS HIS GLY ZCL NME |
| 357 | ACE M3L LYS HIS GLY A30 NME |
| 358 | ACE 4PH LYS HIS GLY M3L NME |
| 359 | ACE ZCL LYS HIS GLY M3L NME |
| 360 | ACE A30 LYS HIS GLY M3L NME |
| 361 | ACE D-M3L GLY HIS LYS D-4PH NME |
| 362 | ACE D-M3L GLY HIS LYS D-ZCL NME |
| 363 | ACE D-M3L GLY HIS LYS D-A30 NME |
| 364 | ACE M3L GLY D-HIS D-LYS 4PH NME |
| 365 | ACE M3L GLY D-HIS D-LYS ZCL NME |
| 366 | ACE M3L GLY D-HIS D-LYS A30 NME |
| 367 | ACE D-M3L GLY D-HIS D-LYS D-4PH NME |
| 368 | ACE D-M3L GLY D-HIS D-LYS D-ZCL NME |
| 369 | ACE D-M3L GLY D-HIS D-LYS D-A30 NME |
| 370 | ACE M3L GLY HIS LHI 4PH NME |
| 371 | ACE M3L GLY HIS LHI ZCL NME |
| 372 | ACE M3L GLY HIS LHI A30 NME |
| 373 | ACE M3L GLY HIS LHY 4PH NME |
| 374 | ACE M3L GLY HIS LHY ZCL NME |
| 375 | ACE M3L GLY HIS LHY A30 NME |
| 376 | ACE M3L GLY HIS LPY 4PH NME |
| 377 | ACE M3L GLY HIS LPY ZCL NME |
| 378 | ACE M3L GLY HIS LPY A30 NME |
| 379 | PAL M3L GLY HIS LYS 4PH NME |
| 380 | PAL M3L GLY HIS LYS ZCL NME |
| 381 | PAL M3L GLY HIS LYS A30 NME |
| 382 | BIO M3L GLY HIS LYS 4PH NME |
| 383 | BIO M3L GLY HIS LYS ZCL NME |
| 384 | BIO M3L GLY HIS LYS A30 NME |
| 385 | PEG M3L GLY HIS LYS 4PH NME |
| 386 | PEG M3L GLY HIS LYS ZCL NME |
| 387 | PEG M3L GLY HIS LYS A30 NME |

(continued)

| SEQ ID NO: | The structural formula of the linear peptide compound (N-terminus → C-terminus) |
|---|---|
| 388 | VIC M3L GLY HIS LYS 4PH NME |
| 389 | VIC M3L GLY HIS LYS ZCL NME |
| 390 | VIC M3L GLY HIS LYS A30 NME |
| 391 | ACE M3L GLY HIS LYS 4PH OH |
| 392 | ACE M3L GLY HIS LYS ZCL OH |
| 393 | ACE M3L GLY HIS LYS A30 OH |
| 394 | ACE M3L GLY HIS LYS 4PH NH2 |
| 395 | ACE M3L GLY HIS LYS ZCL NH2 |
| 396 | ACE M3L GLY HIS LYS A30 NH2 |
| 397 | PAL M3L GLY HIS LYO 4PH OH |
| 398 | PAL M3L GLY HIS LYO ZCL OH |
| 399 | PAL M3L GLY HIS LYO A30 OH |
| 400 | BIO M3L GLY HIS LYO 4PH NH2 |
| 401 | BIO M3L GLY HIS LYO ZCL NH2 |
| 402 | BIO M3L GLY HIS LYO A30 NH2 |
| 403 | PAL M3L GLY HIS LHY 4PH OH |
| 404 | PAL M3L GLY HIS LHY ZCL OH |
| 405 | PAL M3L GLY HIS LHY A30 OH |
| 406 | BIO M3L GLY HIS LHY 4PH NH2 |
| 407 | BIO M3L GLY HIS LHY ZCL NH2 |
| 408 | BIO M3L GLY HIS LHY A30 NH2 |
| 409 | ACE 4PH ALA LYS HIS GLY M3L NME |
| 410 | ACE ZCL ALA LYS HIS GLY M3L NME |
| 411 | ACE A30 ALA LYS HIS GLY M3L NME |
| 412 | ACE 4PH GLY LYS HIS GLY M3L NME |
| 413 | ACE ZCL GLY LYS HIS GLY M3L NME |
| 414 | ACE A30 GLY LYS HIS GLY M3L NME |
| 415 | PAL 4PH ALA LYS HIS GLY M3L OH |
| 416 | PAL ZCL ALA LYS HIS GLY M3L OH |
| 417 | PAL A30 ALA LYS HIS GLY M3L OH |
| 418 | BIO 4PH ALA LYS HIS GLY M3L NH2 |
| 419 | BIO ZCL ALA LYS HIS GLY M3L NH2 |
| 420 | BIO A30 ALA LYS HIS GLY M3L NH2 |
| 421 | PAL M3L GLY HIS LYS 4PH OH |
| 422 | PAL M3L GLY HIS LYS ZCL OH |
| 423 | PAL M3L GLY HIS LYS A30 OH |
| 424 | PAL M3L GLY HIS LYS ALA 4PH OH |
| 425 | PAL M3L GLY HIS LYS ALA ZCL OH |
| 426 | PAL M3L GLY HIS LYS ALA A30 OH |

(continued)

| SEQ ID NO: | The structural formula of the linear peptide compound (N-terminus → C-terminus) |
|---|---|
| 427 | PAL MLZ GLY HIS LYS ALA 4PH OH |
| 428 | PAL MLZ GLY HIS LYS ALA ZCL OH |
| 429 | PAL MLZ GLY HIS LYS ALA A30 OH |
| 430 | PAL MLY GLY HIS LYS ALA 4PH OH |
| 431 | PAL MLY GLY HIS LYS ALA ZCL OH |
| 432 | PAL MLY GLY HIS LYS ALA A30 OH |

In some embodiments, the structure of the linear peptide compound is as shown in Table A-1.

Table A-1: The structural formula of the linear peptide compound

| SEQ ID NO: | The structural formula of the linear peptide compound (N-terminus → C-terminus) |
|---|---|
| 1 | ACE GLY HIS LYS P01 GLY HIS LYS NME |
| 2 | ACE GLY HIS LYS P02 GLY HIS LYS NME |
| 3 | ACE GLY HIS LYS P03 GLY HIS LYS NME |
| 4 | ACE GLY HIS LYS P04 GLY HIS LYS NME |
| 5 | ACE GLY HIS LYS P05 GLY HIS LYS NME |
| 6 | ACE GLY HIS LYS P06 GLY HIS LYS NME |
| 7 | ACE GLY HIS LYS P07 GLY HIS LYS NME |
| 8 | ACE GLY HIS LYS P08 GLY HIS LYS NME |
| 9 | ACE GLY HIS LYS P09 GLY HIS LYS NME |
| 10 | ACE GLY HIS LYS P10 GLY HIS LYS NME |
| 11 | ACE GLY HIS LYS P11 GLY HIS LYS NME |
| 12 | ACE GLY HIS LYS P12 GLY HIS LYS NME |
| 13 | ACE GLY HIS LYS P13 GLY HIS LYS NME |
| 14 | ACE GLY HIS LYS P14 GLY HIS LYS NME |
| 15 | ACE GLY HIS LYS P15 GLY HIS LYS NME |
| 16 | ACE GLY HIS LYS P16 GLY HIS LYS NME |
| 17 | ACE GLY HIS LYS P17 GLY HIS LYS NME |
| 18 | ACE GLY HIS LYS P18 GLY HIS LYS NME |
| 19 | ACE GLY HIS LYS GLY GLY GLY HIS LYS NME |
| 20 | ACE GLY HIS LYS GLY GLY GLY GLY HIS LYS NME |
| 21 | ACE GLY HIS LYS P21 GLY HIS LYS NME |
| 22 | ACE GLY HIS LYS ALA ALA GLY HIS LYS NME |
| 23 | ACE GLY HIS LYS ALA ALA ALA GLY HIS LYS NME |
| 24 | ACE GLY HIS LYS PRO PRO GLY HIS LYS NME |
| 25 | ACE GLY HIS LYS PRO PRO PRO GLY HIS LYS NME |
| 26 | ACE GLY HIS LYS P26 GLY HIS LYS NME |
| 27 | ACE GLY HIS LYS P27 GLY HIS LYS NME |
| 28 | ACE GLY HIS LYS P28 GLY HIS LYS NME |
| 29 | ACE GLY HIS LYS P29 GLY HIS LYS NME |

(continued)

| SEQ ID NO: | The structural formula of the linear peptide compound (N-terminus → C-terminus) |
|---|---|
| 30 | ACE GLY HIS LYS P30 GLY HIS LYS NME |
| 31 | ACE P15 GLY HIS LYS NME |
| 32 | ACE P10 GLY HIS LYS NME |
| 33 | ACE P13 GLY HIS LYS NME |
| 34 | ACE ALA ALA ALA GLY HIS LYS NME |
| 35 | ACE P11 GLY HIS LYS NME |
| 36 | ACE P21 GLY HIS LYS NME |
| 37 | ACE P06 GLY HIS LYS NME |
| 38 | ACE P12 GLY HIS LYS NME |
| 39 | ACE P14 GLY HIS LYS NME |
| 40 | ACE GLY GLY GLY GLY HIS LYS NME |
| 41 | ACE M3L P15 GLY HIS LYS NME |
| 42 | ACE M3L P10 GLY HIS LYS NME |
| 43 | ACE M3L P13 GLY HIS LYS NME |
| 44 | ACE M3L ALA ALA ALA GLY HIS LYS NME |
| 45 | ACE M3L P11 GLY HIS LYS NME |
| 46 | ACE M3L P21 GLY HIS LYS NME |
| 47 | ACE M3L P06 GLY HIS LYS NME |
| 48 | ACE M3L P12 GLY HIS LYS NME |
| 49 | ACE M3L P14 GLY HIS LYS NME |
| 50 | ACE M3L GLY GLY GLY GLY HIS LYS NME |
| 51 | ACE 4PH P15 GLY HIS LYS NME |
| 52 | ACE 4PH P10 GLY HIS LYS NME |
| 53 | ACE 4PH P13 GLY HIS LYS NME |
| 54 | ACE 4PH ALA ALA ALA GLY HIS LYS NME |
| 55 | ACE 4PH P11 GLY HIS LYS NME |
| 56 | ACE 4PH P21 GLY HIS LYS NME |
| 57 | ACE 4PH P06 GLY HIS LYS NME |
| 58 | ACE 4PH P12 GLY HIS LYS NME |
| 59 | ACE 4PH P14 GLY HIS LYS NME |
| 60 | ACE 4PH GLY GLY GLY GLY HIS LYS NME |
| 61 | ACE P15 M3L GLY HIS LYS NME |
| 62 | ACE P10 M3L GLY HIS LYS NME |
| 63 | ACE P13 M3L GLY HIS LYS NME |
| 64 | ACE ALA ALA ALA M3L GLY HIS LYS NME |
| 65 | ACE P11 M3L GLY HIS LYS NME |
| 66 | ACE P21 M3L GLY HIS LYS NME |
| 67 | ACE P06 M3L GLY HIS LYS NME |
| 68 | ACE P12 M3L GLY HIS LYS NME |

(continued)

| SEQ ID NO: | The structural formula of the linear peptide compound (N-terminus → C-terminus) |
|---|---|
| 69 | ACE P14 M3L GLY HIS LYS NME |
| 70 | ACE GLY GLY GLY M3L GLY HIS LYS NME |
| 71 | ACE P15 LYS HIS GLY NME |
| 72 | ACE P10 LYS HIS GLY NME |
| 73 | ACE P13 LYS HIS GLY NME |
| 74 | ACE ALA ALA ALA LYS HIS GLY NME |
| 75 | ACE P11 LYS HIS GLY NME |
| 76 | ACE P21 LYS HIS GLY NME |
| 77 | ACE P06 LYS HIS GLY NME |
| 78 | ACE P12 LYS HIS GLY NME |
| 79 | ACE P14 LYS HIS GLY NME |
| 80 | ACE GLY GLY GLY LYS HIS GLY NME |
| 81 | ACE M3L P15 LYS HIS GLY NME |
| 82 | ACE M3L P10 LYS HIS GLY NME |
| 83 | ACE M3L P13 LYS HIS GLY NME |
| 84 | ACE M3L ALA ALA ALA LYS HIS GLY NME |
| 85 | ACE M3L P11 LYS HIS GLY NME |
| 86 | ACE M3L P21 LYS HIS GLY NME |
| 87 | ACE M3L P06 LYS HIS GLY NME |
| 88 | ACE M3L P12 LYS HIS GLY NME |
| 89 | ACE M3L P14 LYS HIS GLY NME |
| 90 | ACE M3L GLY GLY GLY LYS HIS GLY NME |
| 91 | ACE 4PH M3L P15 GLY HIS LYS NME |
| 92 | ACE 4PH M3L P10 GLY HIS LYS NME |
| 93 | ACE 4PH M3L P13 GLY HIS LYS NME |
| 94 | ACE 4PH M3L ALA ALA ALA GLY HIS LYS NME |
| 95 | ACE 4PH M3L P11 GLY HIS LYS NME |
| 96 | ACE 4PH M3L P21 GLY HIS LYS NME |
| 97 | ACE 4PH M3L P06 GLY HIS LYS NME |
| 98 | ACE 4PH M3L P12 GLY HIS LYS NME |
| 99 | ACE 4PH M3L P14 GLY HIS LYS NME |
| 100 | ACE 4PH M3L GLY GLY GLY GLY HIS LYS NME |
| 101 | ACE ZCL M3L P15 GLY HIS LYS NME |
| 102 | ACE ZCL M3L P10 GLY HIS LYS NME |
| 103 | ACE ZCL M3L P13 GLY HIS LYS NME |
| 104 | ACE ZCL M3L ALA ALA ALA GLY HIS LYS NME |
| 105 | ACE ZCL M3L P11 GLY HIS LYS NME |
| 106 | ACE ZCL M3L P21 GLY HIS LYS NME |
| 107 | ACE ZCL M3L P06 GLY HIS LYS NME |

(continued)

| SEQ ID NO: | The structural formula of the linear peptide compound (N-terminus → C-terminus) |
|---|---|
| 108 | ACE ZCL M3L P12 GLY HIS LYS NME |
| 109 | ACE ZCL M3L P14 GLY HIS LYS NME |
| 110 | ACE ZCL M3L GLY GLY GLY GLY HIS LYS NME |
| 111 | ACE A30 M3L P15 GLY HIS LYS NME |
| 112 | ACE A30 M3L P10 GLY HIS LYS NME |
| 113 | ACE A30 M3L P13 GLY HIS LYS NME |
| 114 | ACE A30 M3L ALA ALA ALA GLY HIS LYS NME |
| 115 | ACE A30 M3L P11 GLY HIS LYS NME |
| 116 | ACE A30 M3L P21 GLY HIS LYS NME |
| 117 | ACE A30 M3L P06 GLY HIS LYS NME |
| 118 | ACE A30 M3L P12 GLY HIS LYS NME |
| 119 | ACE A30 M3L P14 GLY HIS LYS NME |
| 120 | ACE A30 M3L GLY GLY GLY GLY HIS LYS NME |
| 121 | ACE P15 GLY HIS LYS 4PH NME |
| 122 | ACE P10 GLY HIS LYS 4PH NME |
| 123 | ACE P13 GLY HIS LYS 4PH NME |
| 124 | ACE ALA ALA ALA GLY HIS LYS 4PH NME |
| 125 | ACE P11 GLY HIS LYS 4PH NME |
| 126 | ACE P21 GLY HIS LYS 4PH NME |
| 127 | ACE P06 GLY HIS LYS 4PH NME |
| 128 | ACE P12 GLY HIS LYS 4PH NME |
| 129 | ACE P14 GLY HIS LYS 4PH NME |
| 130 | ACE GLY GLY GLY GLY HIS LYS 4PH NME |
| 131 | ACE P15 GLY HIS LYS GLY 4PH NME |
| 132 | ACE P10 GLY HIS LYS GLY 4PH NME |
| 133 | ACE P13 GLY HIS LYS GLY 4PH NME |
| 134 | ACE ALA ALA ALA GLY HIS LYS GLY 4PH NME |
| 135 | ACE P11 GLY HIS LYS GLY 4PH NME |
| 136 | ACE P21 GLY HIS LYS GLY 4PH NME |
| 137 | ACE P06 GLY HIS LYS GLY 4PH NME |
| 138 | ACE P12 GLY HIS LYS GLY 4PH NME |
| 139 | ACE P14 GLY HIS LYS GLY 4PH NME |
| 140 | ACE GLY GLY GLY GLY HIS LYS GLY 4PH NME |
| 141 | ACE P15 GLY HIS LYS ALA 4PH NME |
| 142 | ACE P10 GLY HIS LYS ALA 4PH NME |
| 143 | ACE P13 GLY HIS LYS ALA 4PH NME |
| 144 | ACE ALA ALA ALA GLY HIS LYS ALA 4PH NME |
| 145 | ACE P11 GLY HIS LYS ALA 4PH NME |
| 146 | ACE P21 GLY HIS LYS ALA 4PH NME |

(continued)

| SEQ ID NO: | The structural formula of the linear peptide compound (N-terminus → C-terminus) |
|---|---|
| 147 | ACE P06 GLY HIS LYS ALA 4PH NME |
| 148 | ACE P12 GLY HIS LYS ALA 4PH NME |
| 149 | ACE P14 GLY HIS LYS ALA 4PH NME |
| 150 | ACE GLY GLY GLY GLY HIS LYS ALA 4PH NME |
| 151 | ACE M3L P15 GLY HIS LYS ALA 4PH NME |
| 152 | ACE M3L P10 GLY HIS LYS ALA 4PH NME |
| 153 | ACE M3L P13 GLY HIS LYS ALA 4PH NME |
| 154 | ACE M3L ALA ALA ALA GLY HIS LYS ALA 4PH NME |
| 155 | ACE M3L P11 GLY HIS LYS ALA 4PH NME |
| 156 | ACE M3L P21 GLY HIS LYS ALA 4PH NME |
| 157 | ACE M3L P06 GLY HIS LYS ALA 4PH NME |
| 158 | ACE M3L P12 GLY HIS LYS ALA 4PH NME |
| 159 | ACE M3L P14 GLY HIS LYS ALA 4PH NME |
| 160 | ACE M3L GLY GLY GLY GLY HIS LYS ALA 4PH NME |
| 161 | ACE P15 GLY DHIS DLYS NME |
| 162 | ACE P10 GLY DHIS DLYS NME |
| 163 | ACE P13 GLY DHIS DLYS NME |
| 164 | ACE ALA ALA ALA GLY DHIS DLYS NME |
| 165 | ACE P11 GLY DHIS DLYS NME |
| 166 | ACE P21 GLY DHIS DLYS NME |
| 167 | ACE P06 GLY DHIS DLYS NME |
| 168 | ACE P12 GLY DHIS DLYS NME |
| 169 | ACE P14 GLY DHIS DLYS NME |
| 170 | ACE GLY GLY GLY GLY DHIS DLYS NME |
| 171 | ACE M3L P15 GLY DHIS DLYS NME |
| 172 | ACE M3L P10 GLY DHIS DLYS NME |
| 173 | ACE M3L P13 GLY DHIS DLYS NME |
| 174 | ACE M3L ALA ALA ALA GLY DHIS DLYS NME |
| 175 | ACE M3L P11 GLY DHIS DLYS NME |
| 176 | ACE M3L P21 GLY DHIS DLYS NME |
| 177 | ACE M3L P06 GLY DHIS DLYS NME |
| 178 | ACE M3L P12 GLY DHIS DLYS NME |
| 179 | ACE M3L P14 GLY DHIS DLYS NME |
| 180 | ACE M3L GLY GLY GLY GLY DHIS DLYS NME |
| 181 | ACE P15 GLY HIS LYO NME |
| 182 | ACE P10 GLY HIS LYO NME |
| 183 | ACE P13 GLY HIS LYO NME |
| 184 | ACE ALA ALA ALA GLY HIS LYO NME |
| 185 | ACE P11 GLY HIS LYO NME |

(continued)

| SEQ ID NO: | The structural formula of the linear peptide compound (N-terminus → C-terminus) |
|---|---|
| 186 | ACE P21 GLY HIS LYO NME |
| 187 | ACE P06 GLY HIS LYO NME |
| 188 | ACE P12 GLY HIS LYO NME |
| 189 | ACE P14 GLY HIS LYO NME |
| 190 | ACE GLY GLY GLY GLY HIS LYO NME |
| 191 | ACE M3L P15 GLY HIS LYO NME |
| 192 | ACE M3L P10 GLY HIS LYO NME |
| 193 | ACE M3L P13 GLY HIS LYO NME |
| 194 | ACE M3L ALA ALA ALA GLY HIS LYO NME |
| 195 | ACE M3L P11 GLY HIS LYO NME |
| 196 | ACE M3L P21 GLY HIS LYO NME |
| 197 | ACE M3L P06 GLY HIS LYO NME |
| 198 | ACE M3L P12 GLY HIS LYO NME |
| 199 | ACE M3L P14 GLY HIS LYO NME |
| 200 | ACE M3L GLY GLY GLY GLY HIS LYO NME |
| 201 | ACE P15 GLY HIS LHY NME |
| 202 | ACE P10 GLY HIS LHY NME |
| 203 | ACE P13 GLY HIS LHY NME |
| 204 | ACE ALA ALA ALA GLY HIS LHY NME |
| 205 | ACE P11 GLY HIS LHY NME |
| 206 | ACE P21 GLY HIS LHY NME |
| 207 | ACE P06 GLY HIS LHY NME |
| 208 | ACE P12 GLY HIS LHY NME |
| 209 | ACE P14 GLY HIS LHY NME |
| 210 | ACE GLY GLY GLY GLY HIS LHY NME |
| 211 | ACE M3L P15 GLY HIS LHY NME |
| 212 | ACE M3L P10 GLY HIS LHY NME |
| 213 | ACE M3L P13 GLY HIS LHY NME |
| 214 | ACE M3L ALA ALA ALA GLY HIS LHY NME |
| 215 | ACE M3L P11 GLY HIS LHY NME |
| 216 | ACE M3L P21 GLY HIS LHY NME |
| 217 | ACE M3L P06 GLY HIS LHY NME |
| 218 | ACE M3L P12 GLY HIS LHY NME |
| 219 | ACE M3L P14 GLY HIS LHY NME |
| 220 | ACE M3L GLY GLY GLY GLY HIS LHY NME |
| 221 | PAL P13 GLY DHIS DLYS OH |
| 222 | PAL P10 GLY HIS LYS OH |
| 223 | PAL M3L P10 GLY DHIS DLYS OH |
| 224 | PAL P06 LYS HIS GLY OH |

(continued)

| SEQ ID NO: | The structural formula of the linear peptide compound (N-terminus → C-terminus) |
|---|---|
| 225 | PAL M3L P13 GLY DHIS DLYS OH |
| 226 | PAL P11 GLY DHIS DLYS OH |
| 227 | PAL M3L P15 GLY DHIS DLYS OH |
| 228 | PAL P15 M3L GLY HIS LYS OH |
| 229 | PAL M3L P15 GLY HIS LYS OH |
| 230 | PAL M3L P11 GLY HIS LYS OH |
| 231 | PAL 4PH P15 GLY HIS LYS OH |
| 232 | PAL P15 LYS HIS GLY OH |
| 233 | PAL M3L P13 GLY HIS LYS OH |
| 234 | PAL M3L ALA ALA ALA GLY HIS LYS OH |
| 235 | PAL 4PH P10 GLY HIS LYS OH |
| 236 | PAL P13 GLY HIS LYS OH |
| 237 | PAL M3L ALA ALA ALA GLY DHIS DLYS OH |
| 238 | PAL M3L P10 GLY HIS LYS OH |
| 239 | PAL M3L P10 LYS HIS GLY OH |
| 240 | PAL P12 LYS HIS GLY OH |
| 241 | BIO P13 GLY DHIS DLYS NH2 |
| 242 | BIO P06 LYS HIS GLY NH2 |
| 243 | BIO P11 GLY DHIS DLYS NH2 |
| 244 | BIO P15 LYS HIS GLY NH2 |
| 245 | BIO P13 GLY HIS LYS NH2 |
| 246 | BIO P12 LYS HIS GLY NH2 |
| 247 | BIO P14 M3L GLY HIS LYS NH2 |
| 248 | BIO P15 GLY HIS LYS NH2 |
| 249 | BIO P11 GLY HIS LYS NH2 |
| 250 | BIO P14 LYS HIS GLY NH2 |
| 251 | PAL P15 GLY HIS LHY OH |
| 252 | PAL P13 GLY HIS LHY OH |
| 253 | PAL P10 GLY HIS LHY OH |
| 254 | PAL P11 GLY HIS LHY OH |
| 255 | PAL P06 GLY HIS LHY OH |
| 256 | PAL P21 GLY HIS LHY OH |
| 257 | PAL P12 GLY HIS LHY OH |
| 258 | PAL ALA ALA ALA GLY HIS LHY OH |
| 259 | PAL GLY GLY GLY GLY HIS LHY OH |
| 260 | PAL P14 GLY HIS LHY OH |
| 261 | BIO P06 GLY HIS LHY NH2 |
| 262 | BIO P14 GLY HIS LHY NH2 |
| 263 | BIO M3L P13 GLY HIS LYO NH2 |

(continued)

| SEQ ID NO: | The structural formula of the linear peptide compound (N-terminus → C-terminus) |
|---|---|
| 264 | BIO M3L P12 GLY HIS LYO NH2 |
| 265 | BIO M3L P06 GLY HIS LYO NH2 |
| 266 | ACE P05 GLY HIS LYS NME |
| 267 | ACE P07 GLY HIS LYS NME |
| 268 | ACE P08 GLY HIS LYS NME |
| 269 | ACE GLY GLY GLY HIS LYS NME |
| 270 | ACE ALA ALA GLY HIS LYS NME |
| 271 | ACE PRO PRO GLY HIS LYS NME |
| 272 | ACE PRO PRO PRO GLY HIS LYS NME |
| 273 | ACE P29 GLY HIS LYS NME |
| 274 | ACE M3L P05 GLY HIS LYS NME |
| 275 | ACE M3L P07 GLY HIS LYS NME |
| 276 | ACE M3L P08 GLY HIS LYS NME |
| 277 | ACE M3L GLY GLY GLY HIS LYS NME |
| 278 | ACE M3L ALA ALA GLY HIS LYS NME |
| 279 | ACE M3L PRO PRO GLY HIS LYS NME |
| 280 | ACE M3L PRO PRO PRO GLY HIS LYS NME |
| 281 | ACE M3L P29 GLY HIS LYS NME |
| 282 | ACE 4PH PRO PRO PRO GLY HIS LYS NME |
| 283 | ACE 4PH P29 GLY HIS LYS NME |
| 284 | ACE PRO PRO PRO M3L GLY HIS LYS NME |
| 285 | ACE P29 M3L GLY HIS LYS NME |
| 286 | ACE PRO PRO PRO LYS HIS GLY NME |
| 287 | ACE P29 LYS HIS GLY NME |
| 288 | ACE M3L PRO PRO PRO LYS HIS GLY NME |
| 289 | ACE M3L P29 LYS HIS GLY NME |
| 290 | ACE PRO PRO PRO GLY DHIS DLYS NME |
| 291 | ACE P29 GLY DHIS DLYS NME |
| 292 | ACE M3L PRO PRO PRO GLY DHIS DLYS NME |
| 293 | ACE M3L P29 GLY DHIS DLYS NME |
| 294 | PAL PRO PRO PRO GLY HIS LYS OH |
| 295 | PAL P29 GLY HIS LYS OH |
| 296 | BIO PRO PRO PRO GLY HIS LYS NH2 |
| 297 | BIO P29 GLY HIS LYS NH2 |

In some embodiments, the structure of the linear peptide compound is as shown in Table A-2.

Table A-2: The structural formula of the linear peptide compound

| SEQ ID NO: | The structural formula of the linear peptide compound (N-terminus→C-terminus) |
|---|---|
| 298 | ACE AGM GLY HIS LYS 4PH NME |

(continued)

| SEQ ID NO: | The structural formula of the linear peptide compound (N-terminus→C-terminus) |
|---|---|
| 299 | ACE AGM GLY HIS LYS ZCL NME |
| 300 | ACE AGM GLY HIS LYS A30 NME |
| 301 | ACE AGM GLY HIS LYS D0Q NME |
| 302 | ACE AGM GLY HIS LYS EXL NME |
| 303 | ACE AGM GLY HIS LYS 0UO NME |
| 304 | ACE NMM GLY HIS LYS 4PH NME |
| 305 | ACE NMM GLY HIS LYS ZCL NME |
| 306 | ACE NMM GLY HIS LYS A30 NME |
| 307 | ACE NMM GLY HIS LYS D0Q NME |
| 308 | ACE NMM GLY HIS LYS EXL NME |
| 309 | ACE NMM GLY HIS LYS 0UO NME |
| 310 | ACE 2MR GLY HIS LYS 4PH NME |
| 311 | ACE 2MR GLY HIS LYS ZCL NME |
| 312 | ACE 2MR GLY HIS LYS A30 NME |
| 313 | ACE 2MR GLY HIS LYS D0Q NME |
| 314 | ACE 2MR GLY HIS LYS EXL NME |
| 315 | ACE 2MR GLY HIS LYS 0UO NME |
| 316 | ACE MLZ GLY HIS LYS 4PH NME |
| 317 | ACE MLZ GLY HIS LYS ZCL NME |
| 318 | ACE MLZ GLY HIS LYS A30 NME |
| 319 | ACE MLZ GLY HIS LYS D0Q NME |
| 320 | ACE MLZ GLY HIS LYS EXL NME |
| 321 | ACE MLZ GLY HIS LYS 0UO NME |
| 322 | ACE MLY GLY HIS LYS 4PH NME |
| 323 | ACE MLY GLY HIS LYS ZCL NME |
| 324 | ACE MLY GLY HIS LYS A30 NME |
| 325 | ACE MLY GLY HIS LYS D0Q NME |
| 326 | ACE MLY GLY HIS LYS EXL NME |
| 327 | ACE MLY GLY HIS LYS 0UO NME |
| 328 | ACE M3L GLY HIS LYS 4PH NME |
| 329 | ACE M3L GLY HIS LYS ZCL NME |
| 330 | ACE M3L GLY HIS LYS A30 NME |
| 331 | ACE M3L GLY HIS LYS D0Q NME |
| 332 | ACE M3L GLY HIS LYS EXL NME |
| 333 | ACE M3L GLY HIS LYS 0UO NME |
| 334 | ACE M3L GLY HIS LYO 4PH NME |
| 335 | ACE M3L GLY HIS LYO ZCL NME |
| 336 | ACE M3L GLY HIS LYO A30 NME |
| 337 | ACE M3L GLY HIS LYS ALA 4PH NME |

(continued)

| SEQ ID NO: | The structural formula of the linear peptide compound (N-terminus→C-terminus) |
|---|---|
| 338 | ACE M3L GLY HIS LYS ALA ZCL NME |
| 339 | ACE M3L GLY HIS LYS ALA A30 NME |
| 340 | ACE M3L 2MR GLY HIS LYS ALA 4PH NME |
| 341 | ACE M3L 2MR GLY HIS LYS ALA ZCL NME |
| 342 | ACE M3L 2MR GLY HIS LYS ALA A30 NME |
| 343 | ACE 2MR M3L GLY HIS LYS ALA 4PH NME |
| 344 | ACE 2MR M3L GLY HIS LYS ALA ZCL NME |
| 345 | ACE 2MR M3L GLY HIS LYS ALA A30 NME |
| 346 | ACE 4PH GLY HIS LYS 4PH NME |
| 347 | ACE ZCL GLY HIS LYS ZCL NME |
| 348 | ACE A30 GLY HIS LYS A30 NME |
| 349 | ACE M3L GLY HIS LYS GLY 4PH NME |
| 350 | ACE M3L GLY HIS LYS GLY ZCL NME |
| 351 | ACE M3L GLY HIS LYS GLY A30 NME |
| 352 | ACE M3L GLY HIS LYS PRO 4PH NME |
| 353 | ACE M3L GLY HIS LYS PRO ZCL NME |
| 354 | ACE M3L GLY HIS LYS PRO A30 NME |
| 355 | ACE M3L LYS HIS GLY 4PH NME |
| 356 | ACE M3L LYS HIS GLY ZCL NME |
| 357 | ACE M3L LYS HIS GLY A30 NME |
| 358 | ACE 4PH LYS HIS GLY M3L NME |
| 359 | ACE ZCL LYS HIS GLY M3L NME |
| 360 | ACE A30 LYS HIS GLY M3L NME |
| 361 | ACE D-M3L GLY HIS LYS D-4PH NME |
| 362 | ACE D-M3L GLY HIS LYS D-ZCL NME |
| 363 | ACE D-M3L GLY HIS LYS D-A30 NME |
| 364 | ACE M3L GLY D-HIS D-LYS 4PH NME |
| 365 | ACE M3L GLY D-HIS D-LYS ZCL NME |
| 366 | ACE M3L GLY D-HIS D-LYS A30 NME |
| 367 | ACE D-M3L GLY D-HIS D-LYS D-4PH NME |
| 368 | ACE D-M3L GLY D-HIS D-LYS D-ZCL NME |
| 369 | ACE D-M3L GLY D-HIS D-LYS D-A30 NME |
| 370 | ACE M3L GLY HIS LHI 4PH NME |
| 371 | ACE M3L GLY HIS LHI ZCL NME |
| 372 | ACE M3L GLY HIS LHI A30 NME |
| 373 | ACE M3L GLY HIS LHY 4PH NME |
| 374 | ACE M3L GLY HIS LHY ZCL NME |
| 375 | ACE M3L GLY HIS LHY A30 NME |
| 376 | ACE M3L GLY HIS LPY 4PH NME |

(continued)

| SEQ ID NO: | The structural formula of the linear peptide compound (N-terminus→C-terminus) |
|---|---|
| 377 | ACE M3L GLY HIS LPY ZCL NME |
| 378 | ACE M3L GLY HIS LPY A30 NME |
| 379 | PAL M3L GLY HIS LYS 4PH NME |
| 380 | PAL M3L GLY HIS LYS ZCL NME |
| 381 | PAL M3L GLY HIS LYS A30 NME |
| 382 | BIO M3L GLY HIS LYS 4PH NME |
| 383 | BIO M3L GLY HIS LYS ZCL NME |
| 384 | BIO M3L GLY HIS LYS A30 NME |
| 385 | PEG M3L GLY HIS LYS 4PH NME |
| 386 | PEG M3L GLY HIS LYS ZCL NME |
| 387 | PEG M3L GLY HIS LYS A30 NME |
| 388 | VIC M3L GLY HIS LYS 4PH NME |
| 389 | VIC M3L GLY HIS LYS ZCL NME |
| 390 | VIC M3L GLY HIS LYS A30 NME |
| 391 | ACE M3L GLY HIS LYS 4PH OH |
| 392 | ACE M3L GLY HIS LYS ZCL OH |
| 393 | ACE M3L GLY HIS LYS A30 OH |
| 394 | ACE M3L GLY HIS LYS 4PH NH2 |
| 395 | ACE M3L GLY HIS LYS ZCL NH2 |
| 396 | ACE M3L GLY HIS LYS A30 NH2 |
| 397 | PAL M3L GLY HIS LYO 4PH OH |
| 398 | PAL M3L GLY HIS LYO ZCL OH |
| 399 | PAL M3L GLY HIS LYO A30 OH |
| 400 | BIO M3L GLY HIS LYO 4PH NH2 |
| 401 | BIO M3L GLY HIS LYO ZCL NH2 |
| 402 | BIO M3L GLY HIS LYO A30 NH2 |
| 403 | PAL M3L GLY HIS LHY 4PH OH |
| 404 | PAL M3L GLY HIS LHY ZCL OH |
| 405 | PAL M3L GLY HIS LHY A30 OH |
| 406 | BIO M3L GLY HIS LHY 4PH NH2 |
| 407 | BIO M3L GLY HIS LHY ZCL NH2 |
| 408 | BIO M3L GLY HIS LHY A30 NH2 |
| 409 | ACE 4PH ALA LYS HIS GLY M3L NME |
| 410 | ACE ZCL ALA LYS HIS GLY M3L NME |
| 411 | ACE A30 ALA LYS HIS GLY M3L NME |
| 412 | ACE 4PH GLY LYS HIS GLY M3L NME |
| 413 | ACE ZCL GLY LYS HIS GLY M3L NME |
| 414 | ACE A30 GLY LYS HIS GLY M3L NME |
| 415 | PAL 4PH ALA LYS HIS GLY M3L OH |

(continued)

| SEQ ID NO: | The structural formula of the linear peptide compound (N-terminus→C-terminus) |
|---|---|
| 416 | PAL ZCL ALA LYS HIS GLY M3L OH |
| 417 | PAL A30 ALA LYS HIS GLY M3L OH |
| 418 | BIO 4PH ALA LYS HIS GLY M3L NH2 |
| 419 | BIO ZCL ALA LYS HIS GLY M3L NH2 |
| 420 | BIO A30 ALA LYS HIS GLY M3L NH2 |
| 421 | PAL M3L GLY HIS LYS 4PH OH |
| 422 | PAL M3L GLY HIS LYS ZCL OH |
| 423 | PAL M3L GLY HIS LYS A30 OH |
| 424 | PAL M3L GLY HIS LYS ALA 4PH OH |
| 425 | PAL M3L GLY HIS LYS ALA ZCL OH |
| 426 | PAL M3L GLY HIS LYS ALA A30 OH |
| 427 | PAL MLZ GLY HIS LYS ALA 4PH OH |
| 428 | PAL MLZ GLY HIS LYS ALA ZCL OH |
| 429 | PAL MLZ GLY HIS LYS ALA A30 OH |
| 430 | PAL MLY GLY HIS LYS ALA 4PH OH |
| 431 | PAL MLY GLY HIS LYS ALA ZCL OH |
| 432 | PAL MLY GLY HIS LYS ALA A30 OH |

In some embodiments, the structure of the linear peptide compound is as shown in Table B-1 and Table B-3 or in Table B-2 and Table B-3.

In some embodiments, the structure of the linear peptide compound is as shown in Table B-1.

In some embodiments, the structure of the linear peptide compound is as shown in Table B-2.

In some embodiments, the structure of the linear peptide compound is as shown in Table B-3.

Table B-1: The preferred structural formula of the linear peptide compound

| SEQ ID NO: | The structural formula of the linear peptide compound (N-terminus→C-terminus) |
|---|---|
| 5 | ACE GLY HIS LYS P05 GLY HIS LYS NME |
| 6 | ACE GLY HIS LYS P06 GLY HIS LYS NME |
| 7 | ACE GLY HIS LYS P07 GLY HIS LYS NME |
| 8 | ACE GLY HIS LYS P08 GLY HIS LYS NME |
| 19 | ACE GLY HIS LYS GLY GLY GLY HIS LYS NME |
| 20 | ACE GLY HIS LYS GLY GLY GLY GLY HIS LYS NME |
| 21 | ACE GLY HIS LYS P21 GLY HIS LYS NME |
| 22 | ACE GLY HIS LYS ALA ALA GLY HIS LYS NME |
| 23 | ACE GLY HIS LYS ALA ALA ALA GLY HIS LYS NME |
| 24 | ACE GLY HIS LYS PRO PRO GLY HIS LYS NME |
| 25 | ACE GLY HIS LYS PRO PRO PRO GLY HIS LYS NME |
| 29 | ACE GLY HIS LYS P29 GLY HIS LYS NME |
| 34 | ACE ALA ALA ALA GLY HIS LYS NME |
| 36 | ACE P21 GLY HIS LYS NME |
| 37 | ACE P06 GLY HIS LYS NME |

(continued)

| SEQ ID NO: | The structural formula of the linear peptide compound (N-terminus→C-terminus) |
|---|---|
| 40 | ACE GLY GLY GLY GLY HIS LYS NME |
| 44 | ACE M3L ALA ALA ALA GLY HIS LYS NME |
| 46 | ACE M3L P21 GLY HIS LYS NME |
| 47 | ACE M3L P06 GLY HIS LYS NME |
| 50 | ACE M3L GLY GLY GLY GLY HIS LYS NME |
| 54 | ACE 4PH ALA ALA ALA GLY HIS LYS NME |
| 56 | ACE 4PH P21 GLY HIS LYS NME |
| 57 | ACE 4PH P06 GLY HIS LYS NME |
| 60 | ACE 4PH GLY GLY GLY GLY HIS LYS NME |
| 64 | ACE ALA ALA ALA M3L GLY HIS LYS NME |
| 66 | ACE P21 M3L GLY HIS LYS NME |
| 67 | ACE P06 M3L GLY HIS LYS NME |
| 70 | ACE GLY GLY GLY M3L GLY HIS LYS NME |
| 74 | ACE ALA ALA ALA LYS HIS GLY NME |
| 76 | ACE P21 LYS HIS GLY NME |
| 77 | ACE P06 LYS HIS GLY NME |
| 80 | ACE GLY GLY GLY LYS HIS GLY NME |
| 84 | ACE M3L ALA ALA ALA LYS HIS GLY NME |
| 86 | ACE M3L P21 LYS HIS GLY NME |
| 87 | ACE M3L P06 LYS HIS GLY NME |
| 90 | ACE M3L GLY GLY GLY LYS HIS GLY NME |
| 94 | ACE 4PH M3L ALA ALA ALA GLY HIS LYS NME |
| 96 | ACE 4PH M3L P21 GLY HIS LYS NME |
| 97 | ACE 4PH M3L P06 GLY HIS LYS NME |
| 100 | ACE 4PH M3L GLY GLY GLY GLY HIS LYS NME |
| 104 | ACE ZCL M3L ALA ALA ALA GLY HIS LYS NME |
| 106 | ACE ZCL M3L P21 GLY HIS LYS NME |
| 107 | ACE ZCL M3L P06 GLY HIS LYS NME |
| 110 | ACE ZCL M3L GLY GLY GLY GLY HIS LYS NME |
| 114 | ACE A30 M3L ALA ALA ALA GLY HIS LYS NME |
| 116 | ACE A30 M3L P21 GLY HIS LYS NME |
| 117 | ACE A30 M3L P06 GLY HIS LYS NME |
| 120 | ACE A30 M3L GLY GLY GLY GLY HIS LYS NME |
| 124 | ACE ALA ALA ALA GLY HIS LYS 4PH NME |
| 126 | ACE P21 GLY HIS LYS 4PH NME |
| 127 | ACE P06 GLY HIS LYS 4PH NME |
| 130 | ACE GLY GLY GLY GLY HIS LYS 4PH NME |
| 134 | ACE ALA ALA ALA GLY HIS LYS GLY 4PH NME |
| 136 | ACE P21 GLY HIS LYS GLY 4PH NME |

(continued)

| SEQ ID NO: | The structural formula of the linear peptide compound (N-terminus→C-terminus) |
|---|---|
| 137 | ACE P06 GLY HIS LYS GLY 4PH NME |
| 140 | ACE GLY GLY GLY GLY HIS LYS GLY 4PH NME |
| 144 | ACE ALA ALA ALA GLY HIS LYS ALA 4PH NME |
| 146 | ACE P21 GLY HIS LYS ALA 4PH NME |
| 147 | ACE P06 GLY HIS LYS ALA 4PH NME |
| 150 | ACE GLY GLY GLY GLY HIS LYS ALA 4PH NME |
| 154 | ACE M3L ALA ALA ALA GLY HIS LYS ALA 4PH NME |
| 156 | ACE M3L P21 GLY HIS LYS ALA 4PH NME |
| 157 | ACE M3L P06 GLY HIS LYS ALA 4PH NME |
| 160 | ACE M3L GLY GLY GLY GLY HIS LYS ALA 4PH NME |
| 164 | ACE ALA ALA ALA GLY DHIS DLYS NME |
| 166 | ACE P21 GLY DHIS DLYS NME |
| 167 | ACE P06 GLY DHIS DLYS NME |
| 170 | ACE GLY GLY GLY GLY DHIS DLYS NME |
| 174 | ACE M3L ALA ALA ALA GLY DHIS DLYS NME |
| 176 | ACE M3L P21 GLY DHIS DLYS NME |
| 177 | ACE M3L P06 GLY DHIS DLYS NME |
| 180 | ACE M3L GLY GLY GLY GLY DHIS DLYS NME |
| 184 | ACE ALA ALA ALA GLY HIS LYO NME |
| 186 | ACE P21 GLY HIS LYO NME |
| 187 | ACE P06 GLY HIS LYO NME |
| 190 | ACE GLY GLY GLY GLY HIS LYO NME |
| 194 | ACE M3L ALA ALA ALA GLY HIS LYO NME |
| 196 | ACE M3L P21 GLY HIS LYO NME |
| 197 | ACE M3L P06 GLY HIS LYO NME |
| 200 | ACE M3L GLY GLY GLY GLY HIS LYO NME |
| 204 | ACE ALA ALA ALA GLY HIS LHY NME |
| 206 | ACE P21 GLY HIS LHY NME |
| 207 | ACE P06 GLY HIS LHY NME |
| 210 | ACE GLY GLY GLY GLY HIS LHY NME |
| 214 | ACE M3L ALA ALA ALA GLY HIS LHY NME |
| 216 | ACE M3L P21 GLY HIS LHY NME |
| 217 | ACE M3L P06 GLY HIS LHY NME |
| 220 | ACE M3L GLY GLY GLY GLY HIS LHY NME |
| 224 | PAL P06 LYS HIS GLY OH |
| 234 | PAL M3L ALA ALA ALA GLY HIS LYS OH |
| 237 | PAL M3L ALA ALA ALA GLY DHIS DLYS OH |
| 242 | BIO P06 LYS HIS GLY NH2 |
| 255 | PAL P06 GLY HIS LHY OH |

(continued)

| SEQ ID NO: | The structural formula of the linear peptide compound (N-terminus→C-terminus) |
|---|---|
| 256 | PAL P21 GLY HIS LHY OH |
| 258 | PAL ALA ALA ALA GLY HIS LHY OH |
| 259 | PAL GLY GLY GLY GLY HIS LHY OH |
| 261 | BIO P06 GLY HIS LHY NH2 |
| 265 | BIO M3L P06 GLY HIS LYO NH2 |
| 266 | ACE P05 GLY HIS LYS NME |
| 267 | ACE P07 GLY HIS LYS NME |
| 268 | ACE P08 GLY HIS LYS NME |
| 269 | ACE GLY GLY GLY HIS LYS NME |
| 270 | ACE ALA ALA GLY HIS LYS NME |
| 271 | ACE PRO PRO GLY HIS LYS NME |
| 272 | ACE PRO PRO PRO GLY HIS LYS NME |
| 273 | ACE P29 GLY HIS LYS NME |
| 274 | ACE M3L P05 GLY HIS LYS NME |
| 275 | ACE M3L P07 GLY HIS LYS NME |
| 276 | ACE M3L P08 GLY HIS LYS NME |
| 277 | ACE M3L GLY GLY GLY HIS LYS NME |
| 278 | ACE M3L ALA ALA GLY HIS LYS NME |
| 279 | ACE M3L PRO PRO GLY HIS LYS NME |
| 280 | ACE M3L PRO PRO PRO GLY HIS LYS NME |
| 281 | ACE M3L P29 GLY HIS LYS NME |
| 282 | ACE 4PH PRO PRO PRO GLY HIS LYS NME |
| 283 | ACE 4PH P29 GLY HIS LYS NME |
| 284 | ACE PRO PRO PRO M3L GLY HIS LYS NME |
| 285 | ACE P29 M3L GLY HIS LYS NME |
| 286 | ACE PRO PRO PRO LYS HIS GLY NME |
| 287 | ACE P29 LYS HIS GLY NME |
| 288 | ACE M3L PRO PRO PRO LYS HIS GLY NME |
| 289 | ACE M3L P29 LYS HIS GLY NME |
| 290 | ACE PRO PRO PRO GLY DHIS DLYS NME |
| 291 | ACE P29 GLY DHIS DLYS NME |
| 292 | ACE M3L PRO PRO PRO GLY DHIS DLYS NME |
| 293 | ACE M3L P29 GLY DHIS DLYS NME |
| 294 | PAL PRO PRO PRO GLY HIS LYS OH |
| 295 | PAL P29 GLY HIS LYS OH |
| 296 | BIO PRO PRO PRO GLY HIS LYS NH2 |
| 297 | BIO P29 GLY HIS LYS NH2 |

Table B-2: The preferred structural formula of the linear peptide compound

| SEQ ID NO: | The structural formula of the linear peptide compound (N-terminus→C-terminus) |
|---|---|
| 5 | ACE GLY HIS LYS P05 GLY HIS LYS NME |
| 6 | ACE GLY HIS LYS P06 GLY HIS LYS NME |
| 7 | ACE GLY HIS LYS P07 GLY HIS LYS NME |
| 8 | ACE GLY HIS LYS P08 GLY HIS LYS NME |
| 19 | ACE GLY HIS LYS GLY GLY GLY HIS LYS NME |
| 20 | ACE GLY HIS LYS GLY GLY GLY GLY HIS LYS NME |
| 21 | ACE GLY HIS LYS P21 GLY HIS LYS NME |
| 22 | ACE GLY HIS LYS ALA ALA GLY HIS LYS NME |
| 23 | ACE GLY HIS LYS ALA ALA ALA GLY HIS LYS NME |
| 24 | ACE GLY HIS LYS PRO PRO GLY HIS LYS NME |
| 25 | ACE GLY HIS LYS PRO PRO PRO GLY HIS LYS NME |
| 29 | ACE GLY HIS LYS P29 GLY HIS LYS NME |
| 36 | ACE P21 GLY HIS LYS NME |
| 37 | ACE P06 GLY HIS LYS NME |
| 44 | ACE M3L ALA ALA ALA GLY HIS LYS NME |
| 46 | ACE M3L P21 GLY HIS LYS NME |
| 47 | ACE M3L P06 GLY HIS LYS NME |
| 50 | ACE M3L GLY GLY GLY GLY HIS LYS NME |
| 74 | ACE ALA ALA ALA LYS HIS GLY NME |
| 76 | ACE P21 LYS HIS GLY NME |
| 77 | ACE P06 LYS HIS GLY NME |
| 80 | ACE GLY GLY GLY LYS HIS GLY NME |
| 194 | ACE M3L ALA ALA ALA GLY HIS LYO NME |
| 196 | ACE M3L P21 GLY HIS LYO NME |
| 197 | ACE M3L P06 GLY HIS LYO NME |
| 200 | ACE M3L GLY GLY GLY GLY HIS LYO NME |
| 214 | ACE M3L ALA ALA ALA GLY HIS LHY NME |
| 216 | ACE M3L P21 GLY HIS LHY NME |
| 217 | ACE M3L P06 GLY HIS LHY NME |
| 220 | ACE M3L GLY GLY GLY GLY HIS LHY NME |
| 224 | PAL P06 LYS HIS GLY OH |
| 234 | PAL M3L ALA ALA ALA GLY HIS LYS OH |
| 242 | BIO P06 LYS HIS GLY NH2 |
| 255 | PAL P06 GLY HIS LHY OH |
| 256 | PAL P21 GLY HIS LHY OH |
| 258 | PAL ALA ALA ALA GLY HIS LHY OH |
| 259 | PAL GLY GLY GLY GLY HIS LHY OH |
| 261 | BIO P06 GLY HIS LHY NH2 |
| 265 | BIO M3L P06 GLY HIS LYO NH2 |

(continued)

| SEQ ID NO: | The structural formula of the linear peptide compound (N-terminus→C-terminus) |
|---|---|
| 274 | ACE M3L P05 GLY HIS LYS NME |
| 275 | ACE M3L P07 GLY HIS LYS NME |
| 276 | ACE M3L P08 GLY HIS LYS NME |
| 277 | ACE M3L GLY GLY GLY HIS LYS NME |
| 278 | ACE M3L ALA ALA GLY HIS LYS NME |
| 279 | ACE M3L PRO PRO GLY HIS LYS NME |
| 280 | ACE M3L PRO PRO PRO GLY HIS LYS NME |
| 281 | ACE M3L P29 GLY HIS LYS NME |
| 286 | ACE PRO PRO PRO LYS HIS GLY NME |
| 287 | ACE P29 LYS HIS GLY NME |
| 288 | ACE M3L PRO PRO PRO LYS HIS GLY NME |
| 289 | ACE M3L P29 LYS HIS GLY NME |
| 294 | PAL PRO PRO PRO GLY HIS LYS OH |
| 295 | PAL P29 GLY HIS LYS OH |
| 296 | BIO PRO PRO PRO GLY HIS LYS NH2 |
| 297 | BIO P29 GLY HIS LYS NH2 |

Table B-3: The preferred structural formula of the linear peptide compound

| SEQ ID NO: | The structural formula of the linear peptide compound (N-terminus→C-terminus) |
|---|---|
| 328 | ACE M3L GLY HIS LYS 4PH NME |
| 329 | ACE M3L GLY HIS LYS ZCL NME |
| 330 | ACE M3L GLY HIS LYS A30 NME |
| 334 | ACE M3L GLY HIS LYO 4PH NME |
| 335 | ACE M3L GLY HIS LYO ZCL NME |
| 336 | ACE M3L GLY HIS LYO A30 NME |
| 337 | ACE M3L GLY HIS LYS ALA 4PH NME |
| 338 | ACE M3L GLY HIS LYS ALA ZCL NME |
| 339 | ACE M3L GLY HIS LYS ALA A30 NME |
| 346 | ACE 4PH GLY HIS LYS 4PH NME |
| 347 | ACE ZCL GLY HIS LYS ZCL NME |
| 348 | ACE A30 GLY HIS LYS A30 NME |
| 349 | ACE M3L GLY HIS LYS GLY 4PH NME |
| 350 | ACE M3L GLY HIS LYS GLY ZCL NME |
| 351 | ACE M3L GLY HIS LYS GLY A30 NME |
| 352 | ACE M3L GLY HIS LYS PRO 4PH NME |
| 353 | ACE M3L GLY HIS LYS PRO ZCL NME |
| 354 | ACE M3L GLY HIS LYS PRO A30 NME |
| 355 | ACE M3L LYS HIS GLY 4PH NME |
| 356 | ACE M3L LYS HIS GLY ZCL NME |

(continued)

| SEQ ID NO: | The structural formula of the linear peptide compound (N-terminus→C-terminus) |
|---|---|
| 357 | ACE M3L LYS HIS GLY A30 NME |
| 358 | ACE 4PH LYS HIS GLY M3L NME |
| 359 | ACE ZCL LYS HIS GLY M3L NME |
| 360 | ACE A30 LYS HIS GLY M3L NME |
| 373 | ACE M3L GLY HIS LHY 4PH NME |
| 374 | ACE M3L GLY HIS LHY ZCL NME |
| 375 | ACE M3L GLY HIS LHY A30 NME |
| 379 | PAL M3L GLY HIS LYS 4PH NME |
| 380 | PAL M3L GLY HIS LYS ZCL NME |
| 381 | PAL M3L GLY HIS LYS A30 NME |
| 382 | BIO M3L GLY HIS LYS 4PH NME |
| 383 | BIO M3L GLY HIS LYS ZCL NME |
| 384 | BIO M3L GLY HIS LYS A30 NME |
| 391 | ACE M3L GLY HIS LYS 4PH OH |
| 392 | ACE M3L GLY HIS LYS ZCL OH |
| 393 | ACE M3L GLY HIS LYS A30 OH |
| 394 | ACE M3L GLY HIS LYS 4PH NH2 |
| 395 | ACE M3L GLY HIS LYS ZCL NH2 |
| 396 | ACE M3L GLY HIS LYS A30 NH2 |
| 397 | PAL M3L GLY HIS LYO 4PH OH |
| 398 | PAL M3L GLY HIS LYO ZCL OH |
| 399 | PAL M3L GLY HIS LYO A30 OH |
| 400 | BIO M3L GLY HIS LYO 4PH NH2 |
| 401 | BIO M3L GLY HIS LYO ZCL NH2 |
| 402 | BIO M3L GLY HIS LYO A30 NH2 |
| 403 | PAL M3L GLY HIS LHY 4PH OH |
| 404 | PAL M3L GLY HIS LHY ZCL OH |
| 405 | PAL M3L GLY HIS LHY A30 OH |
| 406 | BIO M3L GLY HIS LHY 4PH NH2 |
| 407 | BIO M3L GLY HIS LHY ZCL NH2 |
| 408 | BIO M3L GLY HIS LHY A30 NH2 |
| 409 | ACE 4PH ALA LYS HIS GLY M3L NME |
| 410 | ACE ZCL ALA LYS HIS GLY M3L NME |
| 411 | ACE A30 ALA LYS HIS GLY M3L NME |
| 412 | ACE 4PH GLY LYS HIS GLY M3L NME |
| 413 | ACE ZCL GLY LYS HIS GLY M3L NME |
| 414 | ACE A30 GLY LYS HIS GLY M3L NME |
| 415 | PAL 4PH ALA LYS HIS GLY M3L OH |
| 416 | PAL ZCL ALA LYS HIS GLY M3L OH |

(continued)

| SEQ ID NO: | The structural formula of the linear peptide compound (N-terminus→C-terminus) |
|---|---|
| 417 | PAL A30 ALA LYS HIS GLY M3L OH |
| 418 | BIO 4PH ALA LYS HIS GLY M3L NH2 |
| 419 | BIO ZCL ALA LYS HIS GLY M3L NH2 |
| 420 | BIO A30 ALA LYS HIS GLY M3L NH2 |
| 421 | PAL M3L GLY HIS LYS 4PH OH |
| 422 | PAL M3L GLY HIS LYS ZCL OH |
| 423 | PAL M3L GLY HIS LYS A30 OH |
| 424 | PAL M3L GLY HIS LYS ALA 4PH OH |
| 425 | PAL M3L GLY HIS LYS ALA ZCL OH |
| 426 | PAL M3L GLY HIS LYS ALA A30 OH |

In some embodiments, the structure of the linear peptide compound is as shown in Table C-1 and Table C-2.

In some embodiments, the structure of the linear peptide compound is as shown in Table C-1.

In some embodiments, the structure of the linear peptide compound is as shown in Table C-2.

Table C-1: The more preferred structural formula of the linear peptide compound

| SEQ ID NO: | The structural formula of the linear peptide compound (N-terminus→C-terminus) |
|---|---|
| 5 | ACE GLY HIS LYS P05 GLY HIS LYS NME |
| 6 | ACE GLY HIS LYS P06 GLY HIS LYS NME |
| 7 | ACE GLY HIS LYS P07 GLY HIS LYS NME |
| 8 | ACE GLY HIS LYS P08 GLY HIS LYS NME |
| 19 | ACE GLY HIS LYS GLY GLY GLY HIS LYS NME |
| 20 | ACE GLY HIS LYS GLY GLY GLY GLY HIS LYS NME |
| 21 | ACE GLY HIS LYS P21 GLY HIS LYS NME |
| 22 | ACE GLY HIS LYS ALA ALA GLY HIS LYS NME |
| 23 | ACE GLY HIS LYS ALA ALA ALA GLY HIS LYS NME |
| 24 | ACE GLY HIS LYS PRO PRO GLY HIS LYS NME |
| 25 | ACE GLY HIS LYS PRO PRO PRO GLY HIS LYS NME |
| 196 | ACE M3L P21 GLY HIS LYO NME |
| 197 | ACE M3L P06 GLY HIS LYO NME |
| 255 | PAL P06 GLY HIS LHY OH |
| 256 | PAL P21 GLY HIS LHY OH |
| 261 | BIO P06 GLY HIS LHY NH2 |
| 265 | BIO M3L P06 GLY HIS LYO NH2 |
| 36 | ACE P21 GLY HIS LYS NME |
| 37 | ACE P06 GLY HIS LYS NME |
| 46 | ACE M3L P21 GLY HIS LYS NME |
| 47 | ACE M3L P06 GLY HIS LYS NME |
| 234 | PAL M3L ALA ALA ALA GLY HIS LYS OH |
| 294 | PAL PRO PRO PRO GLY HIS LYS OH |
| 296 | BIO PRO PRO PRO GLY HIS LYS NH2 |

(continued)

| SEQ ID NO: | The structural formula of the linear peptide compound (N-terminus→C-terminus) |
|---|---|
| 74 | ACE ALA ALA ALA LYS HIS GLY NME |
| 76 | ACE P21 LYS HIS GLY NME |
| 77 | ACE P06 LYS HIS GLY NME |
| 80 | ACE GLY GLY GLY LYS HIS GLY NME |
| 224 | PAL P06 LYS HIS GLY OH |
| 242 | BIO P06 LYS HIS GLY NH2 |
| 286 | ACE PRO PRO PRO LYS HIS GLY NME |

Table C-2: The more preferred structural formula of the linear peptide compound

| SEQ ID NO: | The structural formula of the linear peptide compound (N-terminus→C-terminus) |
|---|---|
| 328 | ACE M3L GLY HIS LYS 4PH NME |
| 329 | ACE M3L GLY HIS LYS ZCL NME |
| 330 | ACE M3L GLY HIS LYS A30 NME |
| 337 | ACE M3L GLY HIS LYS ALA 4PH NME |
| 338 | ACE M3L GLY HIS LYS ALA ZCL NME |
| 339 | ACE M3L GLY HIS LYS ALA A30 NME |
| 349 | ACE M3L GLY HIS LYS GLY 4PH NME |
| 350 | ACE M3L GLY HIS LYS GLY ZCL NME |
| 351 | ACE M3L GLY HIS LYS GLY A30 NME |
| 424 | PAL M3L GLY HIS LYS ALA 4PH OH |
| 425 | PAL M3L GLY HIS LYS ALA ZCL OH |
| 426 | PAL M3L GLY HIS LYS ALA A30 OH |
| 358 | ACE 4PH LYS HIS GLY M3L NME |
| 359 | ACE ZCL LYS HIS GLY M3L NME |
| 360 | ACE A30 LYS HIS GLY M3L NME |
| 409 | ACE 4PH ALA LYS HIS GLY M3L NME |
| 410 | ACE ZCL ALA LYS HIS GLY M3L NME |
| 411 | ACE A30 ALA LYS HIS GLY M3L NME |
| 412 | ACE 4PH GLY LYS HIS GLY M3L NME |
| 413 | ACE ZCL GLY LYS HIS GLY M3L NME |
| 414 | ACE A30 GLY LYS HIS GLY M3L NME |
| 415 | PAL 4PH ALA LYS HIS GLY M3L OH |
| 416 | PAL ZCL ALA LYS HIS GLY M3L OH |
| 417 | PAL A30 ALA LYS HIS GLY M3L OH |
| 418 | BIO 4PH ALA LYS HIS GLY M3L NH2 |
| 419 | BIO ZCL ALA LYS HIS GLY M3L NH2 |
| 420 | BIO A30 ALA LYS HIS GLY M3L NH2 |
| 334 | ACE M3L GLY HIS LYO 4PH NME |
| 335 | ACE M3L GLY HIS LYO ZCL NME |

(continued)

| SEQ ID NO: | The structural formula of the linear peptide compound (N-terminus→C-terminus) |
|---|---|
| 336 | ACE M3L GLY HIS LYO A30 NME |
| 373 | ACE M3L GLY HIS LHY 4PH NME |
| 374 | ACE M3L GLY HIS LHY ZCL NME |
| 375 | ACE M3L GLY HIS LHY A30 NME |
| 397 | PAL M3L GLY HIS LYO 4PH OH |
| 398 | PAL M3L GLY HIS LYO ZCL OH |
| 399 | PAL M3L GLY HIS LYO A30 OH |
| 400 | BIO M3L GLY HIS LYO 4PH NH2 |
| 401 | BIO M3L GLY HIS LYO ZCL NH2 |
| 402 | BIO M3L GLY HIS LYO A30 NH2 |
| 403 | PAL M3L GLY HIS LHY 4PH OH |
| 404 | PAL M3L GLY HIS LHY ZCL OH |
| 405 | PAL M3L GLY HIS LHY A30 OH |
| 406 | BIO M3L GLY HIS LHY 4PH NH2 |
| 407 | BIO M3L GLY HIS LHY ZCL NH2 |
| 408 | BIO M3L GLY HIS LHY A30 NH2 |

In some embodiments, the structure of the linear peptide compound is as shown in Table D.

Table D: The most preferred structural formula of the linear peptide compound

| SEQ ID NO: | The structural formula of the linear peptide compound (N-terminus→C-terminus) |
|---|---|
| 21 | ACE GLY HIS LYS P21 GLY HIS LYS NME |
| 77 | ACE P06 LYS HIS GLY NME |
| 76 | ACE P21 LYS HIS GLY NME |
| 286 | ACE PRO PRO PRO LYS HIS GLY NME |
| 294 | PAL PRO PRO PRO GLY HIS LYS OH |
| 336 | ACE M3L GLY HIS LYO A30 NME |
| 234 | PAL M3L ALA ALA ALA GLY HIS LYS OH |
| 6 | ACE GLY HIS LYS P06 GLY HIS LYS NME |
| 23 | ACE GLY HIS LYS ALA ALA ALA GLY HIS LYS NME |
| 224 | PAL P06 LYS HIS GLY OH |
| 242 | BIO P06 LYS HIS GLY NH2 |
| 256 | PAL P21 GLY HIS LHY OH |
| 265 | BIO M3L P06 GLY HIS LYO NH2 |
| 296 | BIO PRO PRO PRO GLY HIS LYS NH2 |
| 337 | ACE M3L GLY HIS LYS ALA 4PH NME |
| 338 | ACE M3L GLY HIS LYS ALA ZCL NME |
| 349 | ACE M3L GLY HIS LYS GLY 4PH NME |
| 358 | ACE 4PH LYS HIS GLY M3L NME |

(continued)

| SEQ ID NO: | The structural formula of the linear peptide compound (N-terminus→C-terminus) |
|---|---|
| 373 | ACE M3L GLY HIS LHY 4PH NME |
| 397 | PAL M3L GLY HIS LYO 4PH OH |
| 403 | PAL M3L GLY HIS LHY 4PH OH |
| 406 | BIO M3L GLY HIS LHY 4PH NH2 |
| 415 | PAL 4PH ALA LYS HIS GLY M3L OH |
| 417 | PAL A30 ALA LYS HIS GLY M3L OH |
| 419 | BIO ZCL ALA LYS HIS GLY M3L NH2 |
| 424 | PAL M3L GLY HIS LYS ALA 4PH OH |

In some embodiments, the structure of the linear peptide compound is as shown in Table D-1.

Table D-1: The most preferred structural formula of the linear peptide compound

| SEQ ID NO: | The structural formula of the linear peptide compound (N-terminus→C-terminus) |
|---|---|
| 21 | ACE GLY HIS LYS P21 GLY HIS LYS NME |
| 77 | ACE P06 LYS HIS GLY NME |
| 76 | ACE P21 LYS HIS GLY NME |
| 286 | ACE PRO PRO PRO LYS HIS GLY NME |
| 294 | PAL PRO PRO PRO GLY HIS LYS OH |
| 234 | PAL M3L ALA ALA ALA GLY HIS LYS OH |
| 6 | ACE GLY HIS LYS P06 GLY HIS LYS NME |
| 23 | ACE GLY HIS LYS ALA ALA ALA GLY HIS LYS NME |
| 224 | PAL P06 LYS HIS GLY OH |
| 242 | BIO P06 LYS HIS GLY NH2 |
| 256 | PAL P21 GLY HIS LHY OH |
| 265 | BIO M3L P06 GLY HIS LYO NH2 |
| 296 | BIO PRO PRO PRO GLY HIS LYS NH2 |

In some embodiments, the structure of the linear peptide compound is as shown in Table D-2.

Table D-2: The most preferred structural formula of the linear peptide compound

| SEQ ID NO: | The structural formula of the linear peptide compound (N-terminus→C-terminus) |
|---|---|
| 336 | ACE M3L GLY HIS LYO A30 NME |
| 337 | ACE M3L GLY HIS LYS ALA 4PH NME |
| 338 | ACE M3L GLY HIS LYS ALA ZCL NME |
| 349 | ACE M3L GLY HIS LYS GLY 4PH NME |
| 358 | ACE 4PH LYS HIS GLY M3L NME |
| 373 | ACE M3L GLY HIS LHY 4PH NME |
| 397 | PAL M3L GLY HIS LYO 4PH OH |
| 403 | PAL M3L GLY HIS LHY 4PH OH |
| 406 | BIO M3L GLY HIS LHY 4PH NH2 |

(continued)

| SEQ ID NO: | The structural formula of the linear peptide compound (N-terminus→C-terminus) |
|---|---|
| 415 | PAL 4PH ALA LYS HIS GLY M3L OH |
| 417 | PAL A30 ALA LYS HIS GLY M3L OH |
| 419 | BIO ZCL ALA LYS HIS GLY M3L NH2 |
| 424 | PAL M3L GLY HIS LYS ALA 4PH OH |

In a second aspect of the present invention, the present invention provides a pharmaceutical composition comprising the aforementioned linear peptide compound or the stereoisomer thereof.

In some embodiments, the pharmaceutical composition further comprises at least one carrier and/or excipient.

In a third aspect of the present invention, the present invention provides a cosmetic composition (such as a skin care product, a cosmetic, a hair growth serum, etc.) comprising the aforementioned linear peptide compound or the stereoisomer thereof.

In some embodiments, the cosmetic composition further comprises a functional substance.

In some embodiments, the functional substance is selected from one or more of skin conditioners, antioxidants, surfactants, moisturizers, preservatives, chelating agents, and fragrances.

In a fourth aspect of the present invention, the present invention provides use of the aforementioned linear peptide compound or the stereoisomer thereof or the aforementioned pharmaceutical composition in the manufacture of a medicament which is used for one or more selected from the following (1) - (7):

(1) delaying skin aging;
(2) reducing skin damage;
(3) accelerating skin wound healing;
(4) reducing hyperpigmentation;
(5) stimulating hair growth;
(6) improving the success rate of hair transplantation; and
(7) preventing hair loss.

In a fifth aspect of the present invention, the present invention provides the aforementioned linear peptide compound or the stereoisomer thereof or the aforementioned pharmaceutical composition for use in one or more selected from the following (1) - (7):

(1) delaying skin aging;
(2) reducing skin damage;
(3) accelerating skin wound healing;
(4) reducing hyperpigmentation;
(5) stimulating hair growth;
(6) improving the success rate of hair transplantation; and
(7) preventing hair loss.

In a sixth aspect of the present invention, the present invention provides a method selected from one or more of the following (1) - (7), the method comprising: administering to a subject in need thereof an effective amount of the aforementioned linear peptide compound or the stereoisomer thereof or the aforementioned pharmaceutical composition,

(1) a method for delaying skin aging;
(2) a method for reducing skin damage;
(3) a method for accelerating skin wound healing;
(4) a method for reducing hyperpigmentation;
(5) a method for stimulating hair growth;
(6) a method for improving the success rate of hair transplantation; and
(7) a method for preventing hair loss.

In a seventh aspect of the present invention, the present invention provides use of the aforementioned linear peptide compound or the stereoisomer thereof or the aforementioned cosmetic composition in one or more selected from the following (1) - (17), wherein the use is a non-therapeutic use,

(1) anti-aging of the skin;
(2) anti-wrinkling and reducing fine lines of the skin;
(3) improving mechanical properties of the skin: firmness, skin tone, elasticity, and/or flexibility;
(4) increasing skin density (reconstructive effect);
(5) increasing skin volume (plumping effect);
(6) combating the appearance of stretch marks;
(7) reducing skin damage;
(8) accelerating skin wound healing;
(9) combating spots;
(10) improving skin homogeneity and/or radiance;
(11) reducing skin pigmentation;
(12) moisturizing;
(13) stimulating hair growth;
(14) improving the success rate of hair transplantation;
(15) preventing hair loss;
(16) providing localized cosmetic care for eyelashes; and
(17) increasing hair width, density, and/or length.

In an eighth aspect of the present invention, the present invention provides a method selected from one or more of the following (1) - (17), the method comprising: administering to a subject in need thereof an effective amount of the aforementioned linear peptide compound or the stereoisomer thereof or the aforementioned cosmetic composition,

(1) a method for anti-aging of the skin;
(2) a method for anti-wrinkling and reducing fine lines of the skin;
(3) a method for improving mechanical properties of the skin: firmness/skin tone/elasticity/flexibility;
(4) a method for increasing skin density (reconstructive effect);
(5) a method for increasing skin volume (plumping effect);
(6) a method for combating the appearance of stretch marks;
(7) a method for reducing skin damage;
(8) a method for accelerating skin wound healing;
(9) a method for combating spots;
(10) a method for improving skin homogeneity and/or radiance;
(11) a method for reducing skin pigmentation;
(12) a method for moisturizing;
(13) a method for stimulating hair growth;
(14) a method for improving the success rate of hair transplantation;
(15) a method for preventing hair loss;
(16) a method for providing localized cosmetic care for eyelashes; and
(17) a method for increasing hair width, density, and/or length.

In a ninth aspect of the present invention, the present invention provides use of the aforementioned linear peptide compound or the stereoisomer thereof or the aforementioned cosmetic composition in one or more selected from the following (1) - (17):

(1) anti-aging of the skin;
(2) anti-wrinkling and reducing fine lines of the skin;
(3) improving mechanical properties of the skin: firmness, skin tone, elasticity, and/or flexibility;
(4) increasing skin density (reconstructive effect);
(5) increasing skin volume (plumping effect);
(6) combating the appearance of stretch marks;
(7) reducing skin damage;
(8) accelerating skin wound healing;
(9) combating spots;
(10) improving skin homogeneity and/or radiance;
(11) reducing skin pigmentation;
(12) moisturizing;
(13) stimulating hair growth;
(14) improving the success rate of hair transplantation;

(15) preventing hair loss;
(16) providing localized cosmetic care for eyelashes; and
(17) increasing hair width, density, and/or length.

**Beneficial Effects**

1. The linear peptide compound of the present invention (e.g., the linear peptide compound in Table A, or Table A-1, Table A-2, or Table B-1, Table B-2, Table B-3, or any combination thereof, or Table C-1, Table C-2, or Table D, or Table D-1, Table D-2) or the stereoisomer thereof can achieve anti-wrinkling efficacy by significantly increasing the content of Collagen I.
2. The linear peptide compound of the present invention (e.g., the linear peptide compound in Table A, or Table A-1, Table A-2, or Table B-1, Table B-2, Table B-3, or any combination thereof, or Table C-1, Table C-2, or Table D, or Table D-1, Table D-2) or the stereoisomer thereof can significantly increase the content of hyaluronic acid (HA), thereby achieving firming efficacy.
3. The linear peptide compound of the present invention (e.g., the linear peptide compound in Table A, or Table A-1, Table A-2, or Table B-1, Table B-2, Table B-3, or any combination thereof, or Table C-1, Table C-2, or Table D, or Table D-1, Table D-2) or the stereoisomer thereof can significantly increase the content of Elastin, thereby achieving firming efficacy.
4. The linear peptide compound of the present invention (e.g., the linear peptide compound in Table A, or Table A-1, Table A-2, or Table B-1, Table B-2, Table B-3, or any combination thereof, or Table C-1, Table C-2, or Table D, or Table D-1, Table D-2) or the stereoisomer thereof can significantly increase the contents of Collagen I, hyaluronic acid (HA), and Elastin, thereby achieving anti-wrinkling and firming efficacies with excellent medical and cosmetic effects.
5. The linear peptide compound of the present invention (e.g., the linear peptide compound in Table A, or Table A-1, Table A-2, or Table B-1, Table B-2, Table B-3, or any combination thereof, or Table C-1, Table C-2, or Table D, or Table D-1, Table D-2) does not exhibit significant cytotoxicity (e.g., within the concentration range of 5 $\mu$M) and has good prospects for pharmaceutical and cosmetic applications.
6. The linear peptide compound of the present invention (e.g., the linear peptide compound in Table A, or Table A-1, Table A-2, or Table B-1, Table B-2, Table B-3, or any combination thereof, or Table C-1, Table C-2, or Table D, or Table D-1, Table D-2) or the stereoisomer thereof, when used as a medicament, has one or more beneficial effects selected from the following:

(1) delaying skin aging;
(2) reducing skin damage;
(3) accelerating skin wound healing;
(4) reducing hyperpigmentation;
(5) stimulating hair growth;
(6) improving the success rate of hair transplantation; and
(7) preventing hair loss.

7. The linear peptide compound of the present invention (e.g., the linear peptide compound in Table A, or Table A-1, Table A-2, or Table B-1, Table B-2, Table B-3, or any combination thereof, or Table C-1, Table C-2, or Table D, or Table D-1, Table D-2) or the stereoisomer thereof, when used as a cosmetic or a cosmetic for non-therapeutic use, has one or more beneficial effects selected from the following:

(1) anti-aging of the skin;
(2) anti-wrinkling and reducing fine lines of the skin;
(3) improving mechanical properties of the skin: firmness, skin tone, elasticity, and/or flexibility;
(4) increasing skin density (reconstructive effect);
(5) increasing skin volume (plumping effect);
(6) combating the appearance of stretch marks;
(7) reducing skin damage;
(8) accelerating skin wound healing;
(9) combating spots;
(10) improving skin homogeneity and/or radiance;
(11) reducing skin pigmentation;
(12) moisturizing;

(13) stimulating hair growth;
(14) improving the success rate of hair transplantation;
(15) preventing hair loss;
(16) providing localized cosmetic care for eyelashes; and
(17) increasing hair width, density, and/or length.

8. The efficacies of the linear peptide compound of the present invention (e.g., the linear peptide compound in Table A, or Table A-1, Table A-2, or Table B-1, Table B-2, Table B-3, or any combination thereof, or Table C-1, Table C-2, or Table D, or Table D-1, Table D-2) or the stereoisomer thereof in the above seven aspects are comparable to or even better than that of GHK tripeptide.

## DETAILED DESCRIPTION OF EMBODIMENTS

Embodiments of the present invention will be described in detail below with reference to examples; however, those skilled in the art will understand that the following examples are only used to illustrate the present invention and should not be regarded as limiting the scope of the present invention. If no specific conditions are specified in the examples, experiments are carried out according to conventional conditions or conditions recommended by the manufacturer. If manufacturers of reagents or instruments used are not specified, the reagents or instruments are conventional products commercially available.

In the present invention, scientific and technical terms used herein have the meanings commonly understood by those skilled in the art unless otherwise specified. Moreover, the terms and laboratory procedures as used herein related to protein and nucleic acid chemistry, molecular biology, cell and tissue culture, microbiology, and immunology are commonly used terms and routine procedures in their respective fields. Furthermore, for a better understanding of the present invention, definitions and explanations of relevant terms are provided below.

The term "stereoisomer" means that in compound molecules of the same molecular formula, atoms or substituents are linked to each other in the same order, but are in different spatial arrangements, belonging to a type of isomerism in the field of organic chemistry.

The term "carrier" refers to a system that can alter the way a drug enters the human body and its distribution in the body, control the release rate of the drug, and deliver the drug to the targeted organ. Drug carrier release and targeting systems can reduce drug degradation and loss, decrease side effects, and improve bioavailability. For example, the macromolecular surfactants that can be used as carriers, due to their unique amphiphilic structure, can undergo self-assembly, forming various forms of aggregates, such as micelles, microemulsions, gels, liquid crystals, vesicles, etc., as preferred examples. These aggregates have the ability to encapsulate and load drug molecules and also have good permeability to membranes, which can serve as excellent drug carriers.

The term "excipient" refers to an additive in a pharmaceutical formulation other than the active ingredient, which may also be referred to as an adjuvant. The adjuvant includes, but is not limited to: ion exchangers, alumina, aluminum stearate, lecithin, serum proteins such as human serum albumin, buffer substances such as phosphates, glycerol, sorbic acid, potassium sorbate, partial glyceride mixtures of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinylpyrrolidone, cellulose substances, polyethylene glycols, sodium carboxymethyl cellulose, polyacrylates, beeswax, lanolin, etc.

The term "skin conditioner" includes, but is not limited to, hydroquinone, kojic acid, arbutin, ellagic acid, azelaic acid, azelaoyl diglycine, L-ascorbic acid and derivatives thereof, licorice extract, *Malpighia emarginata* fruit extract, *Hydrangea macrophylla* extract, alkoxysalicylic acid, *Polygonum cuspidatum* extract, *Sophora japonica* flower extract, turmeric extract, mulberry bark extract, *Phyllanthus emblica* extract, pea extract, aloin, etc.

The term "antioxidant" includes, but is not limited to, butylated hydroxytoluene, butylated hydroxyanisole, propyl gallate, octyl gallate, dodecanol gallate, tert-butyl hydroquinone, tocopherol, ascorbyl palmitate, isopropyl citrate (mixture), monoglyceride citrate, anoxomer, sodium sulfite, sodium bisulfite, sodium pyrosulfite, cysteine, erythorbic acid, thiourea, dilauryl thiodipropionate, nordihydroguaiaretic acid, thioctic acid, alpha-tocopheryl acetate, carotenoids, *Phyllanthus emblica* tannin, ascorbic acid, glutathione, coenzyme Q10, etc.

The term "surfactant" may be an anionic surfactant, a cationic surfactant, a nonionic surfactant, an ampholytic surfactant, a natural surfactant, a fluorine-containing surfactant, etc.

The term "moisturizer" includes, but is not limited to, glycerol, propylene glycol, butylene glycol, sorbitol, Sorbeth-20, amide moisturizers, glycereth moisturizers, lactic acid and sodium lactate, sodium pyrrolidone carboxylate, hydroxyethyl urea, erythritol, Glycereth-5 lactate, sodium hyaluronate, saccharide isomers, D-panthenol, polyethylene glycol, diglycerol, methacryloyloxyethyl phosphorylcholine, etc.

The term "preservative" includes, but is not limited to, parabens and salts thereof, formaldehyde and formaldehyde donors, isothiazolinones, phenol preservatives, acid preservatives, halide preservatives, quaternary ammonium

compounds, alcohols, commonly used compound preservatives, natural preservatives, etc.

The term "chelating agent" can be selected from ethylenediaminetetraacetic acid and its sodium salts, glycine, citric acid, succinic acid, etc.

The term "fragrance" may in particular be sandalwood fragrance, jasmine fragrance, rose fragrance, sweet orange fragrance, vanilla fragrance, etc. The fragrance may also include essential oils, in particular it may be sour orange peel oil, bitter orange leaf/shoot oil, *Pelargonium graveolens* oil, *Rosa damascena* flower oil, etc.

The term "treatment" generally refers to obtaining the desired pharmacological and/or physiological effects. The effects may be prophylactic on the basis of the complete or partial prevention of a disease or symptoms thereof; and/or the effects may be therapeutic on the basis of the partial or complete stabilization or cure of the disease and/or side effects due to the disease. As used herein, "treatment" covers any treatment of a disease in a patient, including: (a) prevention of a disease or symptom in a patient who is susceptible to the disease or symptom but has not been diagnosed with the disease; (b) suppressing a symptom of a disease, i.e., preventing the development thereof; or (c) relieving a symptom of a disease, i.e., causing the disease or symptom to resolve.

The term "subject" includes humans or non-human animals. In the present invention, the term "non-human animals" include all vertebrates, such as non-mammals (e.g., birds, amphibians, and reptiles) and mammals such as non-human primates, livestock, and/or domesticated animals (e.g., sheep, dogs, cats, cows, pigs, etc.).

The term "effective amount" refers to an amount effective, at dosages and for periods of time necessary, to achieve the desired effects. The "effective amount" in the present invention may vary depending on factors such as the individual's skin/hair state, age, sex, and body weight.

In the present invention, the twenty conventional amino acids and their abbreviations comply with the conventional usage. *See* Immunology-A Synthesis (2nd ed., E.S. Golub and D.R. Gren, Eds., Sinauer Associates, Sunderland, Mass. (1991)), which is incorporated herein by reference.

As is well known to those skilled in the art, the term "amino acid residue" refers to the incomplete amino acid structure with an amino terminus (N-terminus) and a carboxy terminus (C-terminus) remained after an amino acid loses one hydrogen on its amino group and one hydroxyl group on its carboxyl group.

In the linear peptide compound of the present invention, the sequence is by default from the N-terminus to the C-terminus from left to right unless otherwise specified, and those skilled in the art will understand that each linking group is linked to each amino acid residue via a peptide bond. For example, taking the linear peptide compound BIO P06 LYS HIS GLY NH2 as set forth in SEQ ID NO: 242 as an example, its left side is by default the N-terminus and its right side is by default the C-terminus, with linking groups linked to amino acid residues via peptide bonds. Therefore, the chemical structural formula of P06 LYS HIS GLY is

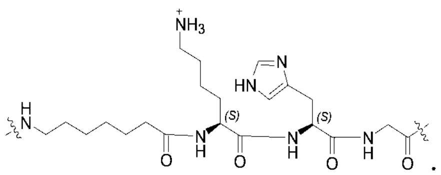

Obviously, in this structure, P06 is linked to LYS via a peptide bond. Further, the left side (N-terminus) of this structure is linked to the capping group BIO, and the right side (C-terminus) of this structure is linked to the capping group NH2, thus forming the linear peptide compound BIO P06 LYS HIS GLY NH2, the structural formula of which is

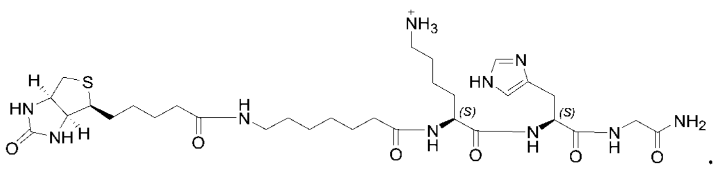

In the linear peptide compound of the present invention, if an amino acid residue is preceded by D or D-, it indicates that the amino acid residue is in the D-configuration, and correspondingly, if an amino acid residue is not preceded by D or D-, the amino acid residue is by default in the L-configuration (with the exception of GLY, which has no chiral center).

In the present invention, the meaning represented by each abbreviated letter is as shown in Table E below.

Table E: The meaning of each abbreviated letter in the present invention

| | |
|---|---|
| Acetyl (ACE) | 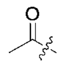 |
| Palmitoyl (PAL) | 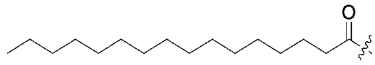 |
| Biotin (BIO) | 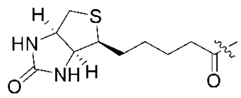 |
| Ascorbic acid (VIC) | 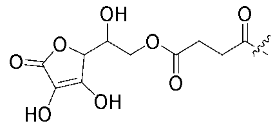 |
| Polyethylene glycol (PEG) | 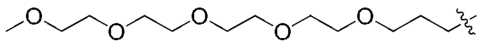 |
| Methylamino (NME) | 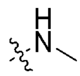 |
| P01 | 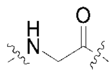 |
| P02 | 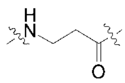 |
| P03 | 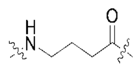 |
| P04 | 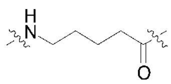 |
| P05 | 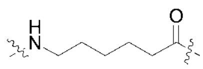 |
| P06 | 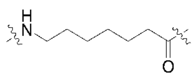 |
| P07 | 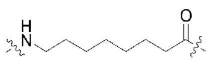 |
| P08 | 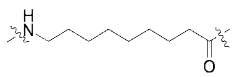 |
| P09 | 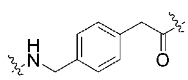 |
| P10 | 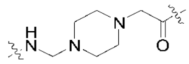 |
| P11 | 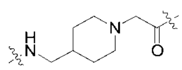 |
| P12 | 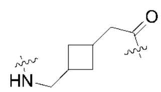 |

(continued)

| | |
|---|---|
| P13 | 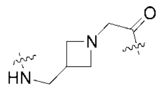 |
| P14 | 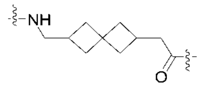 |
| P15 | 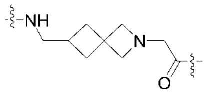 |
| P16 | 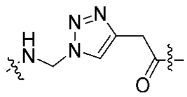 |
| P17 | 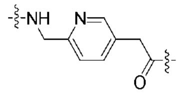 |
| P18 | 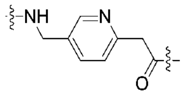 |
| P19 (GLY GLY) | 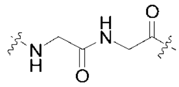 |
| P20 (GLY GLY GLY) | 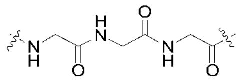 |
| P21 | 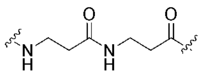 |
| P22 (ALA ALA) | 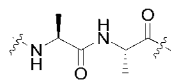 |
| P23 (ALA ALA ALA) | 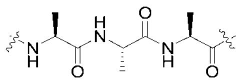 |
| P24 (PRO PRO) | 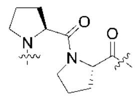 |
| P25 (PRO PRO PRO) | 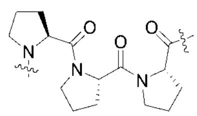 |
| P26 | 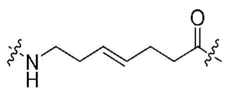 |
| P27 | 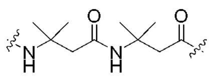 |
| P28 | 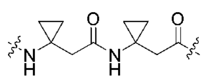 |

(continued)

| | |
|---|---|
| P29 | 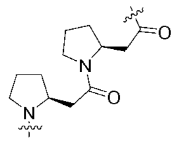 |
| P30 | 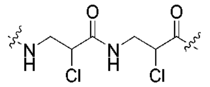 |
| 4PH | 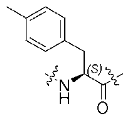 |
| D-4PH | 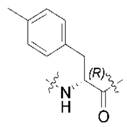 |
| ZCL | 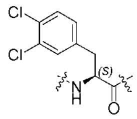 |
| D-ZCL | 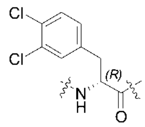 |
| A30 | 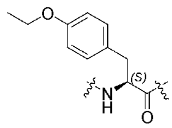 |
| D-A30 | 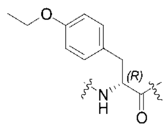 |
| LYO | 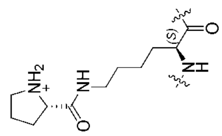 |
| LHY | 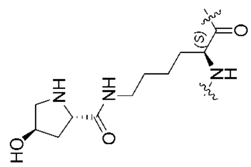 |
| LHI | 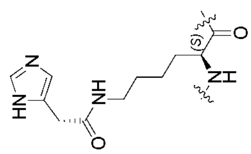 |

(continued)

| | |
|---|---|
| LPY | 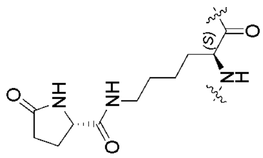 |
| AGM | 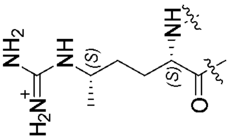 |
| NMM | 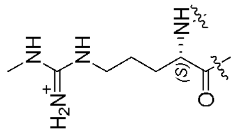 |
| 2MR | 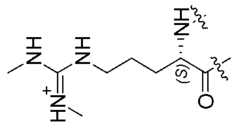 |
| MLZ | 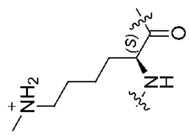 |
| MLY | 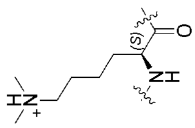 |
| M3L | 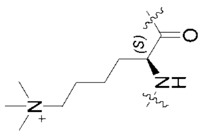 |
| D-M3L | 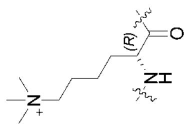 |
| D0Q | 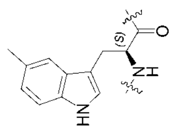 |
| EXL | 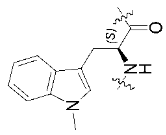 |
| 0UO | 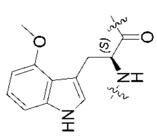 |

(continued)

| | |
|---|---|
| GLY | 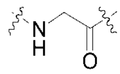 |
| HIS | 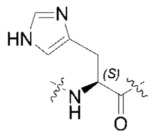 |
| DHIS | 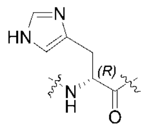 |
| LYS | 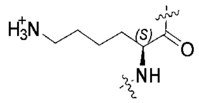 |
| DLYS | 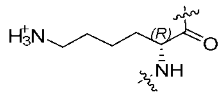 |
| ALA | 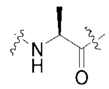 |
| PRO | 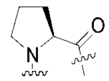 |

In addition, the abbreviations in the examples have conventional meanings well known in the art, and are listed below by the way of illustration:

AcOH, Acetic Acid
ACN, Acetonitrile
DCM, Dichloromethane (Methylene chloride)
DMF, N,N-Dimethylformamide
DIEA, N,N-Diisopropylethylamine
Fmoc, 9-fluorenylmethyloxycarbonyl
HOBT, 1-Hydroxybenzotriazole
Piperidine
PyBOP, Benzotriazol-1-yl-oxytripyrrolidinophosphonium hexafluorophosphate
Resin
TBTU, O-(Benzotriazol-1-yl)-N,N,N',N'-tetramethyluronium tetrafluoroborate
TFA, Trifluoroacetic acid
TFE, 2,2,2-Trifluoroethanol
TIS, Triisopropylsilane
Fmoc-7-Ahp-OH, FMOC-7-AMINO-HEPTANOIC ACID

The present invention will be further explained below in connection with specific examples, which are in no way to be construed as limiting the present invention. In addition, raw materials or reagents are commercially available unless otherwise specified.

**<u>Example 1: Synthesis and Identification of Linear Peptide Compounds</u>**

1. Synthesis of the Linear Peptide Compounds

Taking the linear peptide compound (BIO P06 LYS HIS GLY NH2) as set forth in SEQ ID NO: 242 as an example,

amino acids were condensed sequentially from the C-terminus to the N-terminus (from right to left), with the amino group as the starting point.

Step I. Solid-phase synthesis

Fmoc-Linker MBHA Resin (S = 0.32 mmol/g) was weighed, placed in a glass reaction column, and swollen with DCM for 30 min. The DCM was removed under reduced pressure. A 20% piperidine/DMF solution was added to react for 20 min so as to remove the protecting group Fmoc. The solution was removed under reduced pressure. The resin was washed with DMF five times. Samples were taken for color development detection. A solid-phase resin with amino attached was obtained.
Fmoc-Gly-OH was weighed and added into the resin, and dissolved with DMF. DIEA and TBTU were added, and the mixture was stirred for 60 min. The solution was removed under reduced pressure. The resin was washed with DMF five times. Samples were taken for color development detection. A solid-phase resin with GLY NH2 attached was obtained.
This procedure was repeated, and His, Lys, Ahp (from the raw material Fmoc-7-Ahp-OH), and Biotin were coupled sequentially. Finally, a solid-phase resin with BIO P06 LYS HIS GLY NH2 attached was obtained, i.e., the peptide resin.
Then, the resin was sequentially washed with DMF, DCM, and methanol three times, and was dried to constant weight.

Step II. Cleavage of the peptide resin

Preparation of cleavage reagent: Based on the ratio of 1 g of the peptide resin to 10 mL $\pm$ 2 mL of cleavage reagents (TFA : $H_2O$ : TIS = 95 : 2.5 : 2.5, volume ratio), the usage amounts of the cleavage reagents were calculated. The desired cleavage reagents $H_2O$, TFA, and TIS were added sequentially to a cleavage reaction flask, and the temperature of the cleavage reagents were controlled between 0°C-10°C. The cleavage reagents were added to the peptide resin with stirring, and the temperature of the system was allowed to be stabilized. Subsequently, the reaction was stirred for 2.5 hours while controlling the temperature between 25°C-30°C.
The cleavage solution was filtered off, and the product was precipitated with 5 volumes of ice-cold diethyl ether. The precipitate was filtered off and washed three times with 3 volumes of ice-cold diethyl ether, followed by drying under reduced pressure at room temperature, and a crude solid product was obtained.

Step III. Lyophilization and purification

The crude product was ground into a fine powder. Purified water was prepared, and the ground crude product was added slowly with stirring. An acetonitrile aqueous solution was added dropwise simultaneously. After the addition and dissolution of the crude product were completed, the solution was filtered through a 0.45 $\mu$m microporous filter membrane. The crude product was purified using a C-18 packing preparative column. Mobile phase A was 0.1% TFA in water, and mobile phase B was 0.1% TFA in acetonitrile. Separation and purification were performed at ambient temperature with an appropriate gradient. The target products were collected, analyzed and detected, and classified. The purity requirement was $\geq$ 95%. Unqualified target product was collected and further separated and purified with an appropriate gradient. The qualified main peak was freeze-dried under reduced pressure to give a refined polypeptide as a powder.
Other linear peptide compounds of the present application can be prepared by those skilled in the art by reference to the synthesis procedures described above.

2. Identification of the Linear Peptide Compounds

The refined polypeptide prepared above was weighed, dissolved in a mixed solution of water and acetonitrile until the solution became clear, filtered and then loaded for detection. Detection conditions of high performance liquid chromatography: C18 column, 4.6 $\mu$m $\times$ 250 mm; mobile phase A: 0.1% TFA in acetonitrile, mobile phase B: 0.1% TFA in water; gradient conditions: 25% A and 75% B at 0.01 min $\rightarrow$ 50% A and 50% B at 25 min $\rightarrow$ 100% A at 25.01 min $\rightarrow$ stop at 30 min; detection wavelength: 220 nm; flow rate: 1.0 ml/min; column temperature: 25°C; injection volume: 10 $\mu$L.
The mass spectrometry detector was Agilent-6125B, using an electrospray ionization (ESI) source, with positive ion scanning; voltage of mass spectrometer: +4.5 kv; voltage of detector: 1.5 kv; flow rate of sprayer: 1.5 L/min; voltage of CDL: -20.0 v; temperature of CDL: 250°C; temperature of heating module: 200°C; mobile phase: 50% water/50% acetonitrile; flow rate of mobile phase: 0.2 mL/min.
Mass spectral data of some exemplary linear peptide compounds are as shown in Table 1 below.

Table 1: The mass spectral data of some exemplary linear peptide compounds

| SEQ ID NO: | Theoretical MS value $[M+H]^+$ | Found MS value $[M+H]^+$ | SEQ ID NO: | Theoretical MS value $[M+H]^+$ | Found MS value $[M+H]^+$ |
|---|---|---|---|---|---|
| 21 | 861.0 | 860.6 | 336 | 855.1 | 854.7 |
| 77 | 523.6 | 523.6 | 337 | 799.0 | 798.5 |
| 234 | 963.3 | 963.0 | 338 | 853.9 | 854.0 |
| 6 | 846.0 | 845.6 | 349 | 785.0 | 784.5 |
| 23 | 932.0 | 931.6 | 358 | 727.9 | 727.5 |
| 76 | 538.6 | 538.4 | 373 | 841.0 | 840.6 |
| 224 | 707.0 | 706.6 | 397 | 1007.4 | 1008.2 |
| 242 | 693.9 | 693.5 | 403 | 1024.3 | 1024.4 |
| 256 | 835.0 | 834.6 | 406 | 1011.3 | 1010.6 |
| 265 | 961.2 | 960.6 | 415 | 982.3 | 982.4 |
| 286 | 687.8 | 687.5 | 417 | 1011.4 | 1012.2 |
| 294 | 871.1 | 870.6 | 419 | 1025.1 | 1025.8 |
| 296 | 858.0 | 857.5 | 424 | 982.3 | 982.0 |

As shown in Table 1, the desired linear peptide compounds can be successfully obtained by the preparation method of the present invention.

**Example 2: Biological Activity Test of the Linear Peptide Compounds**

1. Test Methods

A. Fibroblast cytotoxicity test

Test materials: Human-derived fibroblasts (Guangdong BioCell Biotechnology Co., Ltd., Lot No. Fb20081902), DMEM culture medium (Gibco, 23042301, expiration date 230522), PBS (Solarbio, P1010), MTT (Sigma, M5655), DMSO (Sigma, D4540-1L), CO2 incubator (Thermo, 150I), super-clean bench (AIRTECH, SW-CJ-1F), and inverted microscope (Olympus, CKX53).

Test methods:

1) Cell inoculation: After cell resuscitation, the cells were inoculated into 96-well plates when the plating rate reached around 60%, and were incubated overnight in an incubator (37°C, 5% $CO_2$).
2) Experiment grouping: The blank group, solvent control group, positive control group, and sample groups were set for the experiment. In the sample groups, seven concentration gradients were set for each sample, and three duplicate wells were set for each concentration gradient.
3) Formulation of solutions: Sample working solutions at different concentrations were formulated based on the test concentrations of 0.0781 μM, 0.1563 μM, 0.3125 μM, 0.625 μM, 1.25 μM, 2.5 μM, and 5 μM.
4) Administration: Administration was performed when the cell plating rate in the 96-well plates reached 50% to 60%. 200 μL of culture medium per well was added for the solvent control group; 200 μL of culture medium containing 10% DMSO per well was added for the positive control group; 200 μL of culture medium containing the sample at the respective concentration per well was added for the sample groups; no cells were inoculated in the blank group, and only 200 μL of cell culture medium was added. After the administration was complete, the 96-well plates were incubated in an incubator (37°C, 5% $CO_2$) for 24 h.
5) Detection: After 24 h of cell incubation and culture, the supernatant was discarded, and MTT working solution (0.5 mg/mL) was added. Incubation was performed at 37°C for 4 h in the dark. After the incubation was completed, the supernatant was discarded, and 150 μL DMSO was added to each well. The OD value was read at 490 nm.
6) Calculation of relative cell viability: Calculation was performed according to the formula below:

$$\text{Relative cell viability} = \frac{\text{OD of sample well} - \text{OD of blank well}}{\text{OD of solvent control well} - \text{OD of blank well}} \times 100\%.$$

The detection result data of some exemplary linear peptide compounds are as shown in Table 2.

Table 2: The detection result data of the exemplary linear peptide compounds

| SEQ ID NO: | Concentration (μM) | | | | | | | Positive control |
|---|---|---|---|---|---|---|---|---|
| | 0.0781 | 0.1563 | 0.3125 | 0.625 | 1.25 | 2.5 | 5 | 10% DMSO |
| 21 | 102.96% | 100.88% | 100.83% | 99.48% | 99.01% | 98.75% | 98.54% | 2.35% |
| 77 | 110.15% | 108.42% | 108.27% | 105.20% | 105.45% | 105.89% | 103.76% | 3.52% |
| 234 | 107.08% | 107.18% | 106.59% | 106.19% | 107.33% | 106.49% | 106.24% | 3.52% |
| 6 | 98.06% | 97.40% | 97.14% | 94.67% | 94.27% | 94.36% | 94.01% | 6.33% |
| 23 | 102.90% | 99.37% | 99.47% | 99.05% | 99.21% | 95.16% | 95.79% | 4.24% |
| 76 | 106.85% | 106.69% | 104.84% | 104.05% | 105.21% | 103.74% | 103.95% | 4.24% |
| 224 | 100.40% | 98.02% | 98.77% | 97.93% | 97.27% | 92.51% | 93.52% | 1.57% |
| 242 | 98.14% | 96.20% | 94.22% | 93.64% | 92.73% | 92.57% | 91.86% | 1.95% |
| 256 | 97.26% | 96.47% | 96.43% | 96.38% | 93.92% | 92.67% | 91.46% | 1.95% |
| 265 | 99.72% | 98.84% | 97.59% | 96.80% | 96.71% | 94.62% | 94.29% | 1.95% |
| 286 | 104.27% | 103.67% | 103.44% | 103.39% | 102.79% | 102.69% | 101.72% | 1.95% |
| 294 | 106.73% | 104.69% | 104.46% | 104.41% | 103.71% | 103.20% | 103.02% | 1.95% |
| 296 | 98.97% | 95.66% | 95.04% | 95.00% | 94.13% | 93.47% | 93.31% | 1.57% |
| 336 | 106.77% | 103.49% | 102.96% | 102.27% | 100.42% | 100.00% | 99.68% | 4.07% |
| 337 | 96.48% | 96.93% | 93.41% | 93.91% | 93.56% | 92.92% | 91.63% | 3.95% |
| 338 | 98.91% | 95.39% | 96.58% | 93.26% | 92.47% | 92.32% | 91.08% | 3.95% |
| 349 | 94.54% | 92.74% | 92.69% | 93.29% | 92.52% | 93.01% | 92.09% | 1.07% |
| 358 | 103.60% | 97.76% | 97.43% | 97.16% | 97.27% | 96.18% | 95.31% | 1.07% |
| 373 | 98.48% | 96.80% | 94.64% | 94.59% | 94.10% | 93.02% | 93.17% | 2.26% |
| 397 | 95.85% | 95.45% | 95.18% | 95.00% | 94.82% | 93.66% | 92.37% | 3.75% |
| 403 | 100.85% | 96.30% | 96.21% | 96.17% | 95.74% | 94.13% | 94.00% | 3.89% |
| 406 | 103.11% | 99.74% | 99.23% | 98.93% | 97.87% | 95.10% | 94.50% | 4.03% |
| 415 | 103.13% | 96.61% | 96.09% | 94.61% | 94.00% | 92.91% | 92.52% | 1.96% |
| 417 | 96.12% | 95.67% | 94.55% | 94.51% | 91.29% | 90.98% | 90.85% | 3.75% |
| 419 | 94.17% | 93.82% | 93.16% | 93.20% | 91.80% | 91.05% | 91.71% | 1.50% |
| 424 | 95.70% | 94.30% | 94.56% | 94.69% | 92.94% | 93.11% | 92.72% | 1.50% |

As shown in Table 2, in the fibroblast toxicity test experiment, none of the linear peptide compounds exhibited significant cytotoxicity within the concentration range of 5 μM, with good prospects for pharmaceutical and cosmetic applications.

B. Effects of increasing the contents of Collagen I (type I collagen), hyaluronic acid (HA), and Elastin (ELN) in fibroblasts

Test materials: Human-derived fibroblasts (Guangdong BioCell Biotechnology Co., Ltd., Lot No. Fb20081902), DMEM culture medium (Gibco, 23042301, expiration date 230522), PBS (Solarbio, P1010), TGF-β1 (Peprotech, 230504), Collagen I ELISA Kit (Cusabio, B21025824), Elastin ELISA Kit (Abcam, GR3446070-2), HA ELISA Kit (RD, P325066),

CO2 incubator (Thermo, 150I), super-clean bench (AIRTECH, SW-CJ-1F), and microplate reader (BioTek, Epoch).

Test methods:

1) Inoculation: After cell resuscitation, the cells were inoculated into 6-well plates when the plating rate reached around 60%, and were incubated overnight in an incubator (37°C, 5% $CO_2$).

2) Formulation of solutions: The linear peptide compounds in the sample groups, and PC2 (Biotinoyl-GHK,

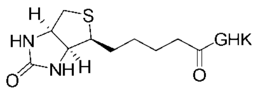

) and PC3 (

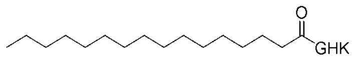

) in the positive control group were all at the concentration of 1 μM, and the test substance working solutions were formulated.

3) Administration: When the cell plating rate in the 6-well plates reached 40% to 60%, administration was performed at 2 mL per well. Three duplicate wells were set for each group. Incubation was performed in an incubator (37°C, 5% $CO_2$) for 24 h.

4) ELISA assay: The ELISA assays were performed according to the instructions of the respective ELISA kits, with the detection indicators being effect of increasing the content of Collagen I in the fibroblasts, effect of increasing the content of HA in the fibroblasts, and effect of increasing the content of Elastin in the fibroblasts, respectively.

The activity test data of some exemplary linear peptide compounds are as shown in Table 3A, Table 3B, and Table 3C below.

Table 3A: Data of effect of increasing the content of Collagen I in the fibroblasts for some exemplary linear peptide compounds

| Blank control/SEQ ID NO: | Effect of increasing the content of Collagen I in the fibroblasts | Blank control/SEQ ID NO: | Effect of increasing the content of Collagen I in the fibroblasts |
|---|---|---|---|
| BC | 35.6 | 337 | 95.66 |
| 77 | 89.94 | 338 | 89.07 |
| 76 | 47.05 | 349 | 90.16 |
| 286 | 53.24 | 358 | 81.27 |
| 294 | 91.47 | 373 | 57.84 |
| 234 | 49.53 | 397 | 54.45 |
| 6 | 44.54 | 403 | 97.51 |
| 23 | 46.06 | 406 | 88.9 |
| 224 | 56.19 | 415 | 85.52 |
| 242 | 70.24 | 417 | 92.88 |
| 256 | 49.42 | 419 | 109.96 |
| 265 | 56.34 | 424 | 97.9 |
| 296 | 88.1 | | |

As shown in Table 3A, compared to the BC, the exemplary linear peptide compounds of the present invention, such as the linear peptide compounds as set forth in SEQ ID NOs: 77, 224, 242, 265, 286, 294, 296, 337, 338, 349, 358, 373, 397, 403, 406, 415, 417, 419, 424, 76, 234, 6, 23, and 256, result in a significant increase in the content of type I collagen (Collagen I), demonstrating that the linear peptide compounds of the present invention can achieve **anti-wrinkling efficacy** by increasing the content of Collagen I.

Table 3B: Data of effect of increasing the content of HA in the fibroblasts for some exemplary linear peptide compounds

| Blank control/SEQ ID NO: | Effect of increasing the content of HA in the fibroblasts | Blank control/SEQ ID NO: | Effect of increasing the content of HA in the fibroblasts |
|---|---|---|---|
| BC | 1402.44 | 337 | 2421.21 |
| 21 | 2423.11 | 338 | 1913.07 |
| 77 | 3493 | 349 | 1794.21 |
| 76 | 2607.31 | 358 | 1913.78 |
| 286 | 2791.21 | 373 | 2472.81 |
| 294 | 1761.46 | 397 | 1832.87 |
| 336 | 2658.08 | 403 | 2688.75 |
| 234 | 2356.91 | 406 | 1473.32 |
| 23 | 2533.64 | 415 | 1570.27 |
| 256 | 1453.81 | 417 | 1938.51 |
| 296 | 1686.42 | | |

As shown in Table 3B, compared to the BC, the exemplary linear peptide compounds of the present invention, such as the linear peptide compounds as set forth in SEQ ID NOs: 21, 77, 23, 76, 286, 294, 336, 337, 338, 358, 397, 415, 234, 256, 296, 349, 373, 403, 406, and 417, result in a significant increase in the content of hyaluronic acid (HA), demonstrating that the linear peptide compounds of the present invention can achieve **firming efficacy** by increasing the content of HA.

Table 3C: Data of effect of increasing the content of Elastin in the fibroblasts for some exemplary linear peptide compounds

| Blank control/SEQ ID NO: | Effect of increasing the content of Elastin in the fibroblasts | Blank control/SEQ ID NO: | Effect of increasing the content of Elastin in the fibroblasts |
|---|---|---|---|
| BC | 756.52 | 296 | 930.98 |
| 21 | 1160.14 | 337 | 1320.95 |
| 77 | 1093.49 | 338 | 1060.72 |
| 76 | 1061.55 | 349 | 836 |
| 286 | 1096.59 | 358 | 1133.42 |
| 294 | 1037.26 | 373 | 843.14 |
| 336 | 978.04 | 397 | 1115.1 |
| 234 | 900.83 | 403 | 928.61 |
| 6 | 1070.92 | 406 | 947.33 |
| 23 | 1097.64 | 415 | 1010.76 |
| 224 | 932.65 | 417 | 861.32 |
| 242 | 1159.72 | 419 | 1015.58 |
| 256 | 1358.18 | 424 | 897.36 |
| 265 | 1008.26 | | |

As shown in Table 3C, compared to the BC, the exemplary linear peptide compounds of the present invention, such as the linear peptide compounds as set forth in SEQ ID NOs: 21, 77, 23, 76, 286, 294, 336, 337, 338, 358, 397, 415, 234, 6, 224, 242, 256, 265, 296, 349, 373, 403, 406, 417, 419, and 424, result in a significant increase in the content of Elastin, demonstrating that the linear peptide compounds of the present invention can achieve **firming efficacy** by increasing the content of Elastin.

In addition, as shown in Table 3A, Table 3B, and Table 3C, compared to the BC, the exemplary linear peptide compounds of the present invention, such as the linear peptide compounds as set forth in SEQ ID NOs: 77, 76, 286, 294, 234, 23, 256, 296, 337, 338, 349, 358, 373, 397, 403, 406, 415, and 417, result in a significant increase in the contents of type I collagen (Collagen I), hyaluronic acid (HA), and Elastin, demonstrating that the linear peptide compounds of the present invention can achieve **firming and anti-wrinkling efficacies** by increasing the contents of Collagen I, HA, and Elastin, with excellent medical and cosmetic effects.

## Claims

1. A linear peptide compound represented by formula O, or a stereoisomer thereof,

$$R^1\text{-}(AA^1)_{n1}\text{-}(AA^2)_{n2}\text{-}(L^1)_{n3}\text{-}(AA^3)_{n4}\text{-}AA^a\text{-}AA^b\text{-}AA^c\text{-}(L^2)_{n5}\text{-}(AA^4)_{n6}\text{-}(AA^5)_{n7}\text{-}(AA^a\text{-}AA^b\text{-}AA^c)_m\text{-}R^2$$

Formula O

wherein:

n1, n2, n3, n4, n5, n6, and n7 are each independently selected from 0 and 1; m is selected from 0 and 1; and the following two conditions are met:

1) when n1 and n7 are both 0, n3 and n5 are not both 0;
2) when n3 and n5 are both 0, n1 and n7 are not both 0;

$AA^a$ is selected from the following amino acid residues: GLY, LYS, DLYS, LYO, LHY, LHI, and LPY;
preferably, $AA^a$ is selected from the following amino acid residues: GLY, LYS, DLYS, LYO, and LHY;
more preferably, $AA^a$ is the following amino acid residue: GLY or LYS;
$AA^b$ is selected from the following amino acid residues: HIS and DHIS;
preferably, $AA^b$ is the following amino acid residue: HIS;
$AA^c$ is selected from the following amino acid residues: LYS, DLYS, LYO, LHY, LHI, LPY, and GLY;
preferably, $AA^c$ is selected from the following amino acid residues: LYS, DLYS, LYO, LHY, and GLY;
more preferably, $AA^c$ is selected from the following amino acid residues: LYS, LYO, LHY, and GLY;
most preferably, $AA^c$ is the following amino acid residue: LYS or GLY;
wherein when $AA^a$ is the following amino acid residue: GLY, $AA^c$ is selected from the following amino acid residues: LYS, DLYS, LYO, LHY, LHI, and LPY, and when $AA^a$ is selected from the following amino acid residues: LYS, DLYS, LYO, LHY, LHI, and LPY, $AA^c$ is the following amino acid residue: GLY;
preferably, $AA^a$-$AA^b$-$AA^c$, as a whole, is selected from the following peptide residues: GLY HIS LYS, LYS HIS GLY, GLY DHIS DLYS, GLY HIS LYO, GLY HIS LHY, GLY HIS LHI, and GLY HIS LPY;
$AA^1$ is selected from the following amino acid residues: AGM, NMM, 2MR, MLZ, MLY, M3L, 4PH, ZCL, A30, and D-M3L;
preferably, $AA^1$ is selected from the following amino acid residues: M3L, 4PH, ZCL, and A30;
$AA^2$ is selected from the following amino acid residues: M3L, 2MR, ALA, and GLY;
$AA^3$ is the following amino acid residue: M3L;
$AA^4$ is selected from the following amino acid residues: ALA, GLY, and PRO; preferably, $AA^4$ is selected from the following amino acid residues: ALA and GLY;
$AA^5$ is selected from the following amino acid residues: M3L, 4PH, ZCL, A30, D0Q, EXL, 0UO, D-4PH, D-ZCL, and D-A30;
preferably, $AA^5$ is selected from the following amino acid residues: M3L, 4PH, ZCL, and A30;
$R^1$ is selected from ACE, PAL, BIO, PEG, and VIC;
preferably, $R^1$ is selected from ACE, PAL, and BIO;
more preferably, $R^1$ is ACE;
$R^2$ is selected from NME, -OH, and -NH2;
preferably, $R^2$ is NME;
$L^1$ is selected from P01, P02, P03, P04, P05, P06, P07, P08, P09, P10, P11, P12, P13, P14, P15, P16, P17, P18, P19, P20, P21, P22, P23, P24, P25, P26, P27, P28, P29, and P30;
preferably, $L^1$ is selected from P15, P10, P13, P23, P11, P21, P06, P12, P14, P20, P05, P07, P08, P19, P22, P24, P25, and P29;
more preferably, $L^1$ is selected from P23, P21, P06, P20, P05, P07, P08, P19, P22, P24, P25, and P29;
most preferably, $L^1$ is selected from P23, P21, P06, P20, and P25;

$L^2$ is selected from P01, P02, P03, P04, P05, P06, P07, P08, P09, P10, P11, P12, P13, P14, P15, P16, P17, P18, P19, P20, P21, P22, P23, P24, P25, P26, P27, P28, P29, and P30;
preferably, $L^2$ is selected from P05, P06, P07, P08, P19, P20, P21, P22, P23, P24, P25, and P29;
more preferably, $L^2$ is selected from P05, P06, P07, P08, P19, P20, P21, P22, P23, P24, and P25;
wherein in the linear peptide compound represented by formula O, the sequence from left to right is from the N-terminus to the C-terminus;
wherein the meanings of P01, P02, P03, P04, P05, P06, P07, P08, P09, P10, P11, P12, P13, P14, P15, P16, P17, P18, P19, P20, P21, P22, P23, P24, P25, P26, P27, P28, P29, P30, ACE, PAL, BIO, PEG, VIC, NME, GLY, HIS, DHIS, LYS, DLYS, LYO, LHY, LHI, LPY, AGM, NMM, 2MR, MLZ, MLY, M3L, 4PH, ZCL, A30, D0Q, EXL, 0UO, D-4PH, D-ZCL, D-A30, D-M3L, ALA, and PRO are as shown in Table E.

2. The linear peptide compound or the stereoisomer thereof of claim 1, **characterized in that** the linear peptide compound has a structure represented by formula I,

$$R^1\text{-}AA^a\text{-}AA^b\text{-}AA^c\text{-}L^2\text{-}AA^a\text{-}AA^b\text{-}AA^c\text{-}R^2 \qquad \text{Formula I}$$

wherein:

$AA^a$ is the following amino acid residue: GLY;
$AA^b$ is selected from the following amino acid residues: HIS and DHIS;
preferably, $AA^b$ is the following amino acid residue: HIS;
$AA^c$ is selected from the following amino acid residues: LYS, DLYS, LYO, and LHY;
preferably, $AA^c$ is the following amino acid residue: LYS;
preferably, $AA^a$-$AA^b$-$AA^c$, as a whole, is the following peptide residue: GLY HIS LYS;
$R^1$ is selected from ACE, PAL, and BIO;
preferably, $R^1$ is ACE;
$R^2$ is selected from NME, -OH, and -NH2;
preferably, $R^2$ is NME;
$L^2$ is selected from P01, P02, P03, P04, P05, P06, P07, P08, P09, P10, P11, P12, P13, P14, P15, P16, P17, P18, P19, P20, P21, P22, P23, P24, P25, P26, P27, P28, P29, and P30;
preferably, $L^2$ is selected from P05, P06, P07, P08, P19, P20, P21, P22, P23, P24, P25, and P29;
more preferably, $L^2$ is selected from P05, P06, P07, P08, P19, P20, P21, P22, P23, P24, and P25.

3. The linear peptide compound or the stereoisomer thereof of claim 1, **characterized in that** the linear peptide compound has a structure represented by formula II,

$$R^1\text{-}(AA^1)_{n1}\text{-}(AA^2)_{n2}\text{-}L^1\text{-}(AA^3)_{n4}\text{-}AA^a\text{-}AA^b\text{-}AA^c\text{-}(AA^4)_{n6}\text{-}(AA^5)_{n7}\text{-}R^2 \qquad \text{Formula II}$$

wherein:

n1, n2, n4, n6, and n7 are each independently selected from 0 and 1;
preferably, n1 is selected from 0 and 1, and n2, n4, n6, and n7 are all 0;
or preferably, n1, n2, n4, n6, and n7 are all 0;
$AA^a$ is selected from the following amino acid residues: GLY, LYS, DLYS, LYO, and LHY;
preferably, $AA^a$ is selected from the following amino acid residues: GLY and LYS;
$AA^b$ is selected from the following amino acid residues: HIS and DHIS;
preferably, $AA^b$ is the following amino acid residue: HIS;
$AA^c$ is selected from the following amino acid residues: LYS, DLYS, LYO, LHY, and GLY;
preferably, $AA^c$ is selected from the following amino acid residues: LYS, LYO, LHY, and GLY;
preferably, $AA^c$ is selected from the following amino acid residues: LYS and GLY;
wherein when $AA^a$ is the following amino acid residue: GLY, $AA^c$ is selected from the following amino acid residues: LYS, DLYS, LYO, and LHY, and when $AA^a$ is selected from the following amino acid residues: LYS, DLYS, LYO, and LHY, $AA^c$ is the following amino acid residue: GLY;
preferably, $AA^a$-$AA^b$-$AA^c$, as a whole, is selected from the following peptide residues: GLY HIS LYS, LYS HIS GLY, GLY DHIS DLYS, GLY HIS LYO, and GLY HIS LHY;
$AA^1$ is selected from the following amino acid residues: M3L, 4PH, ZCL, and A30;
preferably, $AA^1$ is the following amino acid residue: M3L;
$AA^2$ is the following amino acid residue: M3L;

$AA^3$ is the following amino acid residue: M3L;
$AA^4$ is selected from the following amino acid residues: GLY and ALA;
$AA^5$ is the following amino acid residue: 4PH;
$R^1$ is selected from ACE, PAL, and BIO;
preferably, $R^1$ is ACE;
$R^2$ is selected from NME, -OH, and -NH2;
preferably, $R^2$ is NME;
$L^1$ is selected from P15, P10, P13, P23, P11, P21, P06, P12, P14, P20, P05, P07, P08, P19, P22, P24, P25, and P29;
preferably, $L^1$ is selected from P23, P21, P06, P20, P05, P07, P08, P19, P22, P24, P25, and P29;
more preferably, $L^1$ is selected from P23, P21, P06, P20, and P25.

**4.** The linear peptide compound or the stereoisomer thereof of claim 3, **characterized in that** the linear peptide compound has a structure represented by formula II-1,

$$R^1\text{-}(AA^1)_{n1}\text{-}(AA^2)_{n2}\text{-}L^1\text{-}(AA^3)_{n4}\text{-}AA^{a1}\text{-}AA^{b1}\text{-}AA^{c1}\text{-}(AA^4)_{n6}\text{-}(AA^5)_{n7}\text{-}R^2 \quad \text{Formula II-1}$$

wherein:

n1, n2, n4, n6, and n7 are each independently selected from 0 and 1;
preferably, n1 is selected from 0 and 1, and n2, n4, n6, and n7 are all 0;
$AA^{a1}$ is the following amino acid residue: GLY;
$AA^{b1}$ is selected from the following amino acid residues: HIS and DHIS;
preferably, $AA^{b1}$ is the following amino acid residue: HIS;
$AA^{c1}$ is selected from the following amino acid residues: LYS, DLYS, LYO, and LHY;
preferably, $AA^{c1}$ is the following amino acid residue: LYS, LYO, or LHY;
preferably, $AA^{c1}$ is the following amino acid residue: LYS;
preferably, $AA^{a1}$-$AA^{b1}$-$AA^{c1}$, as a whole, is selected from the following peptide residues: GLY HIS LYS, GLY DHIS DLYS, GLY HIS LYO, and GLY HIS LHY;
$AA^1$ is selected from the following amino acid residues: M3L, 4PH, ZCL, and A30;
preferably, $AA^1$ is the following amino acid residue: M3L;
$AA^2$ is the following amino acid residue: M3L;
$AA^3$ is the following amino acid residue: M3L;
$AA^4$ is selected from the following amino acid residues: GLY and ALA;
$AA^5$ is the following amino acid residue: 4PH;
$R^1$ is selected from ACE, PAL, and BIO;
preferably, $R^1$ is ACE;
$R^2$ is selected from NME, -OH, and -NH2;
preferably, $R^2$ is NME;
$L^1$ is selected from P15, P10, P13, P23, P11, P21, P06, P12, P14, P20, P05, P07, P08, P19, P22, P24, P25, and P29;
preferably, $L^1$ is selected from P23, P21, P06, P20, P05, P07, P08, P19, P22, P24, P25, and P29;
more preferably, $L^1$ is selected from P23, P21, P06, P20, and P25.

**5.** The linear peptide compound or the stereoisomer thereof of claim 3, **characterized in that** the linear peptide compound has a structure represented by formula II-2,

$$R^1\text{-}(AA^1)_{n1}\text{-}L^1\text{-}AA^{a2}\text{-}AA^{b2}\text{-}AA^{c2}\text{-}R^2 \quad \text{Formula II-2}$$

wherein:

n1 is selected from 0 and 1;
preferably, n1 is 0;
$AA^{a2}$ is selected from the following amino acid residues: LYS, DLYS, LYO, and LHY;
preferably, $AA^{a2}$ is the following amino acid residue: LYS;
$AA^{b2}$ is selected from the following amino acid residues: HIS and DHIS;
preferably, $AA^{b2}$ is the following amino acid residue: HIS;
$AA^{c2}$ is the following amino acid residue: GLY;

preferably, $AA^{a2}$-$AA^{b2}$-$AA^{c2}$, as a whole, is the following peptide residue: LYS HIS GLY;
$AA^1$ is the following amino acid residue: M3L;
$R^1$ is selected from ACE, PAL, and BIO;
$R^2$ is selected from NME, -OH, and -NH2;
$L^1$ is selected from P15, P10, P13, P23, P11, P21, P06, P12, P14, P20, P25, and P29;
preferably, $L^1$ is selected from P23, P21, P06, P20, P25, and P29;
more preferably, $L^1$ is selected from P23, P21, P06, P20, and P25.

6. The linear peptide compound or the stereoisomer thereof of claim 1, **characterized in that** the linear peptide compound has a structure represented by formula III,

$$R^1\text{-}AA^1\text{-}(AA^2)_{n2}\text{-}AA^a\text{-}AA^b\text{-}AA^c\text{-}(AA^4)_{n6}\text{-}AA^5\text{-}R^2 \qquad \text{Formula III}$$

wherein:

n2 and n6 are each independently selected from 0 and 1;
preferably, n2 is 0, and n6 is selected from 0 and 1;
or preferably, n2 is selected from 0 and 1, and n6 is 0;
$AA^a$ is selected from the following amino acid residues: GLY, LYS, DLYS, LYO, and LHY;
preferably, $AA^a$ is selected from the following amino acid residues: GLY and LYS;
$AA^b$ is selected from the following amino acid residues: HIS and DHIS;
preferably, $AA^b$ is the following amino acid residue: HIS;
$AA^c$ is selected from the following amino acid residues: LYS, DLYS, LYO, LHY, LHI, LPY, and GLY;
preferably, $AA^c$ is selected from the following amino acid residues: LYS, LYO, LHY, and GLY;
more preferably, $AA^c$ is selected from the following amino acid residues: LYS and GLY;
wherein when $AA^a$ is the following amino acid residue: GLY, $AA^c$ is selected from the following amino acid residues: LYS, DLYS, LYO, LHY, LHI, and LPY, and when $AA^a$ is selected from the following amino acid residues: LYS, DLYS, LYO, and LHY, $AA^c$ is the following amino acid residue: GLY;
preferably, $AA^a$-$AA^b$-$AA^c$, as a whole, is selected from the following peptide residues: GLY HIS LYS, LYS HIS GLY, GLY DHIS DLYS, GLY HIS LYO, GLY HIS LHI, GLY HIS LHY, and GLY HIS LPY;
$R^1$ is selected from ACE, PAL, BIO, PEG, and VIC;
preferably, $R^1$ is selected from ACE, PAL, and BIO;
$R^2$ is selected from NME, -OH, and -NH2;
$AA^1$ is selected from the following amino acid residues: AGM, NMM, 2MR, MLZ, MLY, M3L, 4PH, ZCL, A30, and D-M3L;
preferably, $AA^1$ is selected from the following amino acid residues: M3L, 4PH, ZCL, and A30;
more preferably, $AA^1$ is selected from the following amino acid residues: 4PH, ZCL, and A30;
more preferably, $AA^1$ is the following amino acid residue: M3L;
$AA^2$ is selected from the following amino acid residues: M3L, 2MR, ALA, and GLY;
preferably, $AA^2$ is selected from the following amino acid residues: M3L and 2MR;
preferably, $AA^2$ is selected from the following amino acid residues: ALA and GLY;
$AA^4$ is selected from the following amino acid residues: ALA, GLY, and PRO;
preferably, $AA^4$ is selected from the following amino acid residues: ALA and GLY;
$AA^5$ is selected from the following amino acid residues: M3L, 4PH, ZCL, A30, D0Q, EXL, 0UO, D-4PH, D-ZCL, and D-A30;
preferably, $AA^5$ is selected from the following amino acid residues: M3L, 4PH, ZCL, and A30;
more preferably, $AA^5$ is selected from the following amino acid residues: 4PH, ZCL, and A30;
more preferably, $AA^5$ is the following amino acid residue: M3L.

7. The linear peptide compound or the stereoisomer thereof of claim 6, **characterized in that** the linear peptide compound has a structure represented by formula III-1,

$$R^1\text{-}AA^1\text{-}(AA^2)_{n2}\text{-}AA^{a1}\text{-}AA^{b1}\text{-}AA^{c1}\text{-}(AA^4)_{n6}\text{-}AA^5\text{-}R^2 \qquad \text{Formula III-1}$$

wherein:

n2 and n6 are each independently selected from 0 and 1;
preferably, n2 is 0, and n6 is selected from 0 and 1;

$AA^{a1}$ is the following amino acid residue: GLY;
$AA^{b1}$ is selected from the following amino acid residues: HIS and DHIS;
preferably, $AA^{b1}$ is the following amino acid residue: HIS;
$AA^{c1}$ is selected from the following amino acid residues: LYS, DLYS, LYO, LHY, LHI, and LPY;
preferably, $AA^{c1}$ is selected from the following amino acid residues: LYS, LYO, and LHY;
more preferably, $AA^{c1}$ is the following amino acid residue: LYS;
preferably, $AA^{a1}$-$AA^{b1}$-$AA^{c1}$, as a whole, is selected from the following peptide residues: GLY HIS LYS, GLY DHIS DLYS, GLY HIS LYO, GLY HIS LHI, GLY HIS LHY, and GLY HIS LPY;
$R^1$ is selected from ACE, PAL, BIO, PEG, and VIC;
preferably, $R^1$ is selected from ACE, PAL, and BIO;
$R^2$ is selected from NME, -OH, and -NH2;
$AA^1$ is selected from the following amino acid residues: AGM, NMM, 2MR, MLZ, MLY, M3L, 4PH, ZCL, A30, and D-M3L;
preferably, $AA^1$ is selected from the following amino acid residues: M3L, 4PH, ZCL, and A30;
more preferably, $AA^1$ is the following amino acid residue: M3L;
$AA^2$ is selected from the following amino acid residues: M3L and 2MR;
$AA^4$ is selected from the following amino acid residues: ALA, GLY, and PRO;
preferably, $AA^4$ is selected from the following amino acid residues: ALA and GLY;
$AA^5$ is selected from the following amino acid residues: 4PH, ZCL, A30, D0Q, EXL, 0UO, D-4PH, D-ZCL, and D-A30;
preferably, $AA^5$ is selected from the following amino acid residues: 4PH, ZCL, and A30.

**8.** The linear peptide compound or the stereoisomer thereof of claim 6, **characterized in that** the linear peptide compound has a structure represented by formula III-2,

$$R^1\text{-}AA^1\text{-}(AA^2)_{n2}\text{-}AA^{a2}\text{-}AA^{b2}\text{-}AA^{c2}\text{-}AA^5\text{-}R^2 \qquad \text{Formula III-2}$$

wherein:

n2 is selected from 0 and 1;
$AA^{a2}$ is selected from the following amino acid residues: LYS, DLYS, LYO, and LHY;
preferably, $AA^{a2}$ is the following amino acid residue: LYS;
$AA^{b2}$ is selected from the following amino acid residues: HIS and DHIS;
preferably, $AA^{b2}$ is the following amino acid residue: HIS;
$AA^{c2}$ is the following amino acid residue: GLY;
preferably, $AA^{a2}$-$AA^{b2}$-$AA^{c2}$, as a whole, is the following peptide residue: LYS HIS GLY;
$R^1$ is selected from ACE, PAL, BIO, PEG, and VIC;
preferably, $R^1$ is selected from ACE, PAL, and BIO;
preferably, $R^1$ is ACE;
$R^2$ is selected from NME, -OH, and -NH2;
preferably, $R^2$ is NME;
$AA^1$ is selected from the following amino acid residues: M3L, 4PH, ZCL, and A30;
preferably, $AA^1$ is selected from the following amino acid residues: 4PH, ZCL, and A30;
$AA^2$ is selected from the following amino acid residues: ALA and GLY;
$AA^5$ is selected from the following amino acid residues: M3L, 4PH, ZCL, and A30;
preferably, $AA^5$ is the following amino acid residue: M3L.

**9.** The linear peptide compound or the stereoisomer thereof of any one of claims 1-8, **characterized in that** the linear peptide compound has a structure represented by formula IV,

$$R^1\text{-}(AA^1)_{n1}\text{-}(L^1)_{n3}\text{-}AA^a\text{-}AA^b\text{-}AA^c\text{-}(AA^5)_{n7}\text{-}R^2 \qquad \text{Formula IV}$$

wherein:

n1, n3, and n7 are each independently selected from 0 and 1, and are not all 0;
preferably, n1 and n7 are 0, and n3 is 1; or n1 and n7 are 1; n3 is 0; or n1 and n3 are 1, and n7 is 0;
$AA^a$-$AA^b$-$AA^c$, as a whole, is selected from the following peptide residues: LYS HIS GLY, GLY HIS LYS, GLY HIS LYO, and GLY HIS LHY;

preferably, $AA^a$-$AA^b$-$AA^c$, as a whole, is selected from the following peptide residues: LYS HIS GLY, GLY HIS LYS, and GLY HIS LYO;
$AA^1$ is selected from the following amino acid residues: M3L, AGM, NMM, 2MR, MLZ, MLY, 4PH, ZCL, A30, and D-M3L;
preferably, $AA^1$ is selected from the following amino acid residues: M3L, 4PH, ZCL, and A30;
preferably, $AA^1$ is selected from the following amino acid residues: M3L and 4PH;
preferably, $AA^1$ is the following amino acid residue: M3L;
$AA^5$ is selected from the following amino acid residues: 4PH, ZCL, A30, D0Q, EXL, 0UO, M3L, D-4PH, D-ZCL, and D-A30;
preferably, $AA^5$ is selected from the following amino acid residues: 4PH, ZCL, A30, and M3L;
preferably, $AA^5$ is selected from the following amino acid residues: 4PH, A30, and M3L;
preferably, $AA^5$ is the following amino acid residue: 4PH;
preferably, $AA^5$ is the following amino acid residue: A30;
$L^1$ is selected from P15, P10, P13, P23, P11, P21, P06, P12, P14, P20, P05, P07, P08, P19, P22, P24, P25, and P29;
preferably, $L^1$ is selected from P23, P21, P06, P20, P05, P07, P08, P19, P22, P24, P25, and P29;
preferably, $L^1$ is selected from P21, P06, P23, P25, and P20;
preferably, $L^1$ is selected from P21, P06, P25, and P23;
$R^1$ is selected from ACE, PAL, BIO, PEG, and VIC;
preferably, $R^1$ is selected from ACE, PAL, and BIO;
preferably, $R^1$ is selected from ACE and PAL;
more preferably, $R^1$ is ACE;
$R^2$ is selected from NME, -OH, and -NH2;
preferably, $R^2$ is selected from NME and -OH;
more preferably, $R^2$ is NME.

**10.** The linear peptide compound or the stereoisomer thereof of any one of claims 1-9, **characterized in that** the structure of the linear peptide compound is as shown in Table A, Table A-1, Table A-2, Table B-1, Table B-2, Table B-3, Table C-1, Table C-2, Table D, Table D-1, and/or Table D-2.

**11.** A pharmaceutical composition comprising the linear peptide compound or the stereoisomer thereof of any one of claims 1-10;
optionally, further comprising at least one carrier and/or excipient.

**12.** A cosmetic composition comprising the linear peptide compound or the stereoisomer thereof of any one of claims 1-10;

optionally, further comprising a functional substance;
wherein preferably, the functional substance is selected from one or more of skin conditioners, antioxidants, surfactants, moisturizers, preservatives, chelating agents, and fragrances.

**13.** Use of the linear peptide compound or the stereoisomer thereof of any one of claims 1-10 or the pharmaceutical composition of claim 11 in the manufacture of a medicament which is used for one or more selected from the following (1) - (7):

(1) delaying skin aging;
(2) reducing skin damage;
(3) accelerating skin wound healing;
(4) reducing hyperpigmentation;
(5) stimulating hair growth;
(6) improving the success rate of hair transplantation; and
(7) preventing hair loss.

**14.** Use of the linear peptide compound or the stereoisomer thereof of any one of claims 1-10 or the cosmetic composition of claim 12 in one or more selected from the following (1) - (17):

(1) anti-aging of the skin;
(2) anti-wrinkling and reducing fine lines of the skin;

(3) improving mechanical properties of the skin: firmness, skin tone, elasticity, and/or flexibility;
(4) increasing skin density;
(5) increasing skin volume;
(6) combating the appearance of stretch marks;
(7) reducing skin damage;
(8) accelerating skin wound healing;
(9) combating spots;
(10) improving skin homogeneity and/or radiance;
(11) reducing skin pigmentation;
(12) moisturizing;
(13) stimulating hair growth;
(14) improving the success rate of hair transplantation;
(15) preventing hair loss;
(16) providing localized cosmetic care for eyelashes; and
(17) increasing hair width, density, and/or length.

## INTERNATIONAL SEARCH REPORT

International application No. **PCT/CN2024/121723**

**A. CLASSIFICATION OF SUBJECT MATTER**

C07K7/06(2006.01)i; A61K38/08(2019.01)i; A61Q19/08(2006.01)i; A61P17/00(2006.01)i

According to International Patent Classification (IPC) or to both national classification and IPC

**B. FIELDS SEARCHED**

Minimum documentation searched (classification system followed by classification symbols)

IPC:C07K; A61K; A61Q; A61P

Documentation searched other than minimum documentation to the extent that such documents are included in the fields searched

Electronic data base consulted during the international search (name of data base and, where practicable, search terms used)

CJFD, CNKI, CNABS, CNTXT, ENTXT, ENTXTC, VEN, BING, WEB OF SCIENCE, PUBMED, NCBI, EMBL, STN, 中国专利生物序列检索数据库, China Patents Biological Sequence Database: 深圳众格生物科技有限公司, 三肽, 六肽, 肽, KHG, GHK, Gly-His-Lys-Gly-His-Lys, LYS HIS GLY, GLY HIS LYS, 组氨酸, 甘氨酸, 赖氨酸, 修饰, biotin, bio+, 生物素, 乙酰基, ace, 棕榈酰, pal, 聚乙二醇, peg, 抗坏血酸, vic, 维生素, 维他命, vitamin, 聚氧乙烯, 聚环氧乙烷, PEO, POE, polyethylene glycol, Hexapeptide, tripeptide, 相关序列

**C. DOCUMENTS CONSIDERED TO BE RELEVANT**

| Category* | Citation of document, with indication, where appropriate, of the relevant passages | Relevant to claim No. |
|---|---|---|
| A | CN 114702549 A (SUN YAT-SEN UNIVERSITY) 05 July 2022 (2022-07-05)<br>claims 1-10 | 1-14 |
| A | WO 2016097966 A1 (SEDERMA) 23 June 2016 (2016-06-23)<br>claims 1-15, and description, page 2, line 13, page 4, line 8, and page 6, line 28-page 7, line 15 | 1-14 |
| A | WO 2012164488 A2 (SEDERMA) 06 December 2012 (2012-12-06)<br>claims 1-9 | 1-14 |
| A | CN 101790536 A (INNOVACTIV INC.) 28 July 2010 (2010-07-28)<br>entire document | 1-14 |
| A | CN 106999401 A (SEDERMA SA) 01 August 2017 (2017-08-01)<br>entire document | 1-14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 December 2024** | **07 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2024/121723**

**C. DOCUMENTS CONSIDERED TO BE RELEVANT**

| Category* | Citation of document, with indication, where appropriate, of the relevant passages | Relevant to claim No. |
|---|---|---|
| A | US 2015140139 A1 (GWANGJU INSTITUTE OF SCIENCE AND TECHNOLOGY) 21 May 2015 (2015-05-21) entire document | 1-14 |
| A | WO 2013117573 A1 (INSTITUT EUROPEEN DE BIOLOGIE CELLULAIRE et al.) 15 August 2013 (2013-08-15) entire document | 1-14 |

Form PCT/ISA/210 (second sheet) (July 2022)

**INTERNATIONAL SEARCH REPORT**

International application No.
**PCT/CN2024/121723**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/121723**

| Patent document cited in search report | Publication date (day/month/year) | Patent family member(s) | Publication date (day/month/year) |
|---|---|---|---|
| CN 114702549 A | 05 July 2022 | None | |
| WO 2016097966 A1 | 23 June 2016 | FR 3029915 A1 | 17 June 2016 |
| | | FR 3029915 B1 | 09 December 2016 |
| | | EP 3233210 A1 | 25 October 2017 |
| | | EP 3233210 B1 | 05 February 2020 |
| WO 2012164488 A2 | 06 December 2012 | FR 2975904 A1 | 07 December 2012 |
| | | FR 2975904 B1 | 23 August 2013 |
| | | WO 2012164488 A3 | 23 January 2014 |
| | | EP 2714001 A2 | 09 April 2014 |
| CN 101790536 A | 28 July 2010 | BRPI 0814709 A2 | 06 June 2017 |
| | | BRPI 0814709 B1 | 29 September 2020 |
| | | US 2010311667 A1 | 09 December 2010 |
| | | US 9115176 B2 | 25 August 2015 |
| | | WO 2009003283 A1 | 08 January 2009 |
| | | WO 2009003283 A8 | 04 February 2010 |
| | | ES 2605162 T3 | 13 March 2017 |
| | | KR 20100042637 A | 26 April 2010 |
| | | EP 2164858 A1 | 24 March 2010 |
| | | EP 2164858 A4 | 30 April 2014 |
| | | EP 2164858 B1 | 31 August 2016 |
| | | AU 2008271875 A1 | 08 January 2009 |
| | | AU 2008271875 B2 | 07 March 2013 |
| | | CA 2692190 A1 | 08 January 2009 |
| | | CA 2692190 C | 02 June 2015 |
| | | MX 2009014233 A | 10 March 2010 |
| | | JP 2010531815 A | 30 September 2010 |
| | | JP 5570978 B2 | 13 August 2014 |
| CN 106999401 A | 01 August 2017 | FR 3029782 A1 | 17 June 2016 |
| | | FR 3029782 B1 | 07 June 2019 |
| | | BR 112017012474 A2 | 27 February 2018 |
| | | BR 112017012474 B1 | 11 May 2021 |
| | | EP 3256102 A1 | 20 December 2017 |
| | | EP 3256102 B1 | 09 August 2023 |
| | | JP 2022019764 A | 27 January 2022 |
| | | KR 20170086680 A | 26 July 2017 |
| | | KR 102655355 B1 | 05 April 2024 |
| | | JP 2018500330 A | 11 January 2018 |
| | | JP 7040940 B2 | 23 March 2022 |
| | | WO 2016097965 A1 | 23 June 2016 |
| | | US 2018000717 A1 | 04 January 2018 |
| | | US 11612556 B2 | 28 March 2023 |
| | | MX 2017007819 A | 02 October 2017 |
| US 2015140139 A1 | 21 May 2015 | KR 20130128584 A | 27 November 2013 |
| | | KR 101385196 B1 | 14 April 2014 |
| | | WO 2013172586 A1 | 21 November 2013 |
| | | JP 2015520160 A | 16 July 2015 |
| | | JP 6145505 B2 | 14 June 2017 |
| | | US 9724416 B2 | 08 August 2017 |
| WO 2013117573 A1 | 15 August 2013 | FR 2986528 A1 | 09 August 2013 |

Form PCT/ISA/210 (patent family annex) (July 2022)

**INTERNATIONAL SEARCH REPORT**
**Information on patent family members**

International application No.
**PCT/CN2024/121723**

| Patent document cited in search report | Publication date (day/month/year) | Patent family member(s) | Publication date (day/month/year) |
|---|---|---|---|
| | | FR 2986528 B1 | 04 December 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311272706
- CN 202411324581


### Non-patent literature cited in the description


- Immunology-A Synthesis. Sinauer Associates, 1991